# EUROPÄISCHE PATENTSCHRIFT

(11) **EP 4 360 669 B1**
(45) Veröffentlichungstag und Bekanntmachung des Hinweises auf die Patenterteilung: **09.12.2026**
(21) Anmeldenummer: 24162858.5
(22) Anmeldetag: 21.02.2022
(51) Int. Cl.: A61K 9/08, A61K 31/216, A61L 2/07, A61M 5/00, A61M 5/31, A61M 5/315, A61P 13/10

(54) **VERFAHREN ZUR SPRITZENSTERILISATION**
METHOD FOR SYRINGE STERILIZATION
PROCÉDÉ DE STÉRILISATION PAR SERINGUE

(30) Priorität: 24.04.2021 DE 102021002145; 07.05.2021 DE 102021112033
(43) Veröffentlichungstag der Anmeldung: 01.05.2024
(62) Dokumentnummer(n) der früheren Anmeldung(en) nach Art. 76 EPÜ: 22708881.2 / 4 297 725
(73) Patentinhaber: Farco-Pharma GmbH, 50670 Köln (DE)
(72) Erfinder: KÜHBACHER, Andreas, Dr., 50670 Köln (DE); ZEPPEK, Cathrin, Dr., 50670 Köln (DE); THIELICKE, Wolfram, 50670 Köln (DE)
(74) Vertreter: Strehlke, Ingo Kurt

(56) Entgegenhaltungen:
- EP-A1- 1 088 565
- EP-A1- 3 888 630
- DE-A1- 102017 104 931

## Beschreibung

Die vorliegende Erfindung betrifft das medizinisch-technische Gebiet der Sterilisation bzw. der Keimreduzierung einer speziellen Zusammensetzung, welche Oxybutynin bzw. Oxybutyninhydrochlorid als Wirkstoff aufweist und welche in eine insbesondere anwendungsfertige Kolbenspritze eingebracht ist bzw. in dieser vorliegt.

Die vorliegende Erfindung betrifft eine mit einer sterilen oxybutyninhaltigen Zusammensetzung, wie sie insbesondere zur Verwendung bei der prophylaktischen bzw. therapeutischen Behandlung von neurogenen Blasenfunktionsstörungen, insbesondere mittels Instillation in die Harnblase, eingesetzt wird, befüllte Kolbenspritze als solche, wobei die in der Kolbenspritze enthaltene oxybutyninhaltige Zusammensetzung einschließlich der Kolbenspritze nach einem vorliegend beschriebenen Verfahren dampfsterilisiert worden ist.

Weiterhin betrifft die vorliegende Erfindung auch eine Verpackungseinheit, welche mindestens eine erfindungsgemäße Kolbenspritze aufweist, wobei die Kolbenspritze in eine Verpackung eingebracht ist bzw. in einer Verpackung vorliegt.

Weiterhin betrifft die vorliegende Erfindung auch entsprechende Kits auf Basis der erfindungsgemäßen Kolbenspritze bzw. der oxybutyninhaltigen Zusammensetzung nach der Erfindung.

Der Wirkstoff Oxybutynin (synonym auch als 4-Diethylaminobut-2-inyl-2-cyclohexyl-2-hydroxy-2-phenylethanoat bezeichnet) bzw. Oxybutyninhydrochlorid ist ein Anticholinergikum, welches zur Behandlung von Blasenfunktionsstörungen bzw. Dysfunktionen der Harnblase eingesetzt wird, und zwar insbesondere von solchen Blasenfunktionsstörungen, welche mit einer Detrusorhyperaktivität und/oder eine Detrusor-Sphinkter-Dyssynergie einhergehen bzw. hierdurch bedingt sind.

Neurogene Blasenfunktionsstörungen (nBFS) bzw. neurogene Blasenentleerungsstörungen stellen eine oftmals therapiebedürfte pathologische Situation der Harnblase und der an der Harnspeicherung und Harnausscheidung beteiligten anatomischen Strukturen dar. Bei neurogenen Blasenfunktionsstörungen handelt es sich insbesondere um solche Dysfunktionen der Harnblase bzw. der relevanten anatomischen Strukturen, die infolge einer Fehlfunktion oder Verletzung des Nervensystems auftreten, beispielsweise infolge von Rückenmarksverletzungen, Spina bifida ("offener Rücken"), Diabetes, Multiple Sklerose, Schlaganfall oder Morbus Parkinson. Der mit der Dysfunktion der Harnblase einhergehende Leidensdruck von Patienten ist dabei mitunter sehr groß, zumal die oftmals bereits schon eingeschränkte Lebensqualität durch die mit der Dysfunktion einhergehenden Beschwerden zusätzlich verschlechtert wird.

Im Allgemeinen wird als neurogene Blasenfunktionsstörung (nBFS) somit eine Störung der Harnspeicherung bzw. der Harnleerung bezeichnet, welche insbesondere durch neurologische Veränderungen bzw. Verletzungen im Bereich des Rückenmarks im Bereich der für die Blasenfunktion relevanten Abschnitte des Gehirns oder im Bereich der Peripherie verursacht werden, wobei eine normale nervale Signalübertragung zur Steuerung der Harnblase oftmals beeinträchtigt oder behindert ist. Infolge der zugrundeliegenden neurologischen Schäden ist die natürliche Funktion der Harnblase im Hinblick auf deren Funktionszustände, nämlich einer (Harn-)Füllphase zum einen und einer (Harn-)Entleerungs- bzw. Miktionsphase zum anderen, gestört. Dabei kann der physiologische Ablauf von Blasenfüllung und Blasenentleerung sogar auf mehreren Ebenen beeinträchtigt bzw. unterbunden sein.

Infolge der neurologischen Fehlfunktion liegt bei einer neurogenen Blasenfunktionsstörung (nBFS) oftmals eine Detrusorhyperaktivität und/oder eine Detrusor-Sphinkter-Dyssynergie vor. Damit geht häufig eine Störung des Zusammenspiels bzw. der Koordination der für die natürliche Blasenfunktion relevanten anatomischen Strukturen einher.

Der Detrusor (*Musculus detrusor vesicae* bzw. "Austreiber der Harnblase") stellt ein kräftiges Muskelsystem dar, welches die Harnblase umgibt bzw. einen Teil der Harnblase bildet, wobei es sich bei dem Detrusor um einen glatten Muskel handelt, welcher in einer physiologischer Situation bei der Miktion bzw. Blasenentleerung und somit der Harnaustreibung eine große Rolle spielt. Dabei wird durch Signale des Parasympathikus eine Anspannung des Muskelsystems hervorgerufen, wodurch der Blaseninhalt unter Druck gesetzt wird. Zudem wird der Detrusor durch einen intrinsischen Nervenplexus beeinflusst, welcher in der Blasenwand liegt und den Tonus an den Füllungszustand der Harnblase anpasst.

In der pathologischen Situation liegt bei einer neurogenen Blasenfunktionsstörung oftmals eine Detrusorhyperaktivität vor, welche in diesem Zusammenhang auch als neurogene Detrusorhyperaktivität (*Neurogenic Detrusor Overactifity, NDO*) bezeichnet wird. Eine Detrusorhyperaktivität, welche mit einer relevanten neurologischen Grunderkrankung in Verbindung steht, geht dabei insbesondere mit Pollakisurie, Nykturie sowie einer übermäßigen Drangsymptomatik einher.

Bei einer Detrusor-Sphinkter-Dyssynergie (DSD) ist zudem das Zusammenwirken der bei der Blasenentleerung beteiligten anatomischen Strukturen gestört, wobei eine Detrusorüberaktivität einer spastischen Funktionsstörung der Beckenbodenmuskulatur bzw. des äußeren Blasenschließmuskels gegenübersteht, was zu einer Obstruktion des Blasenausgangs bei gleichzeitiger Miktionsanstrengung führt. Typisch für eine Detrusor-Sphinkter-Dyssynergie ist dabei ein häufig unterbrochener Harnstrahl und Schwierigkeiten bei der Initiierung der Miktion. Darüber hinaus liegen oftmals auch Pollakisurie sowie eine Restharnbildung vor.

Neben den vorgenannten Symptomen ist eine neurogene Blasenfunktionsstörung oftmals auch mit hohen intravesikalen Drücken verbunden, d. h. aufgrund der Hyperaktivität des Detrusor bzw. der Detrusor-Sphinkter-Dyssynergie wird ein übermäßiger Druck in der Harnblase aufgebaut. Dies stellt nicht zuletzt auch eine Gefahr für den oberen Harntrakt dar, so gehen übermäßig hohe intravesikale Drücke oftmals mit einer Nierenschädigung einher bzw. rufen diese hervor.

Im Lichte der obigen Ausführungen besteht somit im Stand der Technik ein großer Bedarf an der Bereitstellung therapeutischer Konzepte, welche bei Vorliegen einer neurogenen Blasenfunktionsstörung zu einer Normalisierung bzw. Verbesserung der Blasenfunktion bzw. zu einer Verringerung der Symptome führen, und zwar sowohl was die Probleme beim Harnlassen als auch die vorliegenden hohen Drücke in der Harnblase anbelangt. Dabei besteht ein wesentliches Ziel neben der Verbesserung der Miktion auch in der Verringerung des intrinsischen Drucks, um hierdurch etwaige Nierenschäden oder dergleichen zu vermeiden.

In diesem Zusammenhang verfolgt die Therapie der neurogenen Blasenfunktionsstörung somit mehrere Ziele, und zwar insbesondere den Schutz des oberen Harntraktes und der Nierenfunktion, die Verbesserung der Kontinenzsituation, die Wiederherstellung bzw. Verbesserung der Funktion des unteren Harntraktes sowie nicht zuletzt die Steigerung der Lebensqualität.

Neben konservativen Maßnahmen, wie regelmäßige Bewegung, gezieltes Blasentraining oder dergleichen, kommen auch pharmakotherapeutische Maßnahmen zur Behandlung neurogener Blasenfunktionsstörungen zum Einsatz. Beispielsweise kann auf Basis minimalinvasiver Verfahren eine Injektion mit Botulinumtoxin in die Harnblasenwand durchgeführt werden, um hierdurch die Aktivität des Detrusors zu vermindern. Die Verabreichung ist jedoch relativ aufwendig und nur durch einen Arzt durchzuführen, da die Wirksubstanz relativ aufwendig in die Blasenwandung injiziert werden muss. Weiterhin kommen auch minimalinvasive Verfahren in Form von sakraler Neuromodulation zum Einsatz, bei welcher im oberen Gesäßbereich ein Schrittmacher implantiert wird, welcher über eine Elektrode schwache elektrische Impulse an die Sakralnerven abgibt. Der diesbezügliche Einsatzbereich ist jedoch im Hinblick auf die zugrundeliegenden Grunderkrankungen beschränkt. Zudem handelt es sich hierbei um einen nicht unwesentlichen operativen Eingriff, welcher mit weiteren Risiken einhergeht.

Zudem sind im Stand der Technik pharmakologische Therapien bekannt, bei denen Anticholinergika zum Einsatz kommen. In diesem Zusammenhang wird insbesondere auf eine Verabreichung von Oxybutynin bzw. Oxybutyninhydrochlorid, Trospiumchlorid, Propiverin, Tolterodin oder dergleichen abgestellt. Dabei besteht im Stand der Technik ein Fokus auf der systemischen bzw. oralen Applikation bzw. Verabreichung dieser Wirksubstanzen. Die systemische Verabreichung kann jedoch im verstärkten Maße mit systemischen Nebenwirkungen einhergehen, wie Restharnbildung, Obstipation, Akkomodationsstörungen, Mundtrockenheit, Tachykardie, Herzrhythmusstörungen und dergleichen. Darüber hinaus ist die Konzentration der Wirksubstanz am Wirkort, nämlich der Harnblase, mitunter nicht optimal einzustellen.

Darüber hinaus ist eine systemische Verabreichung der Wirksubstanz in Form von Anticholinergika insofern nachteilig, als ein Teil der verabreichten Wirksubstanz bei deren erster Passage im Gastrointestinalbereich bzw. in der Leber verstoffwechselt wird (sogenannter *First* pass-Metabolismus), wobei mitunter nichtwirksame Metaboliten entstehen, so dass die Bioverfügbarkeit am Wirkort geringer ausfällt bzw. die Wirkmenge am Wirkort nur schwer einzustellen bzw. vorzugeben ist. Folglich können Patienten mit systemischen Darreichungsformen mitunter nicht hinreichend eingestellt werden.

Die WO 2012/154779 A1 bzw. die zu derselben Patentfamilie gehörende US 2012/0289564 A1 betrifft die Verwendung einer Kombination von Oxybutynin und einer die Bildung von speichelanregenden Substanz zur Behandlung der überaktiven Blase, wobei diesbezüglich auf eine orale Applikation fokussiert wird.

Die EP 1 088 565 A1 betrifft eine verschweißte Verpackung mit einem Set für die intravesikale Instillation, umfassend eine sterilisierte Einmalspritze mit einem opaken Zylinder und einem latexfreien Dichtungsring am vorderen Ende des Kolbens, gefüllt mit einer isotonischen Natriumchloridlösung eines Arzneimittelwirkstoffs, einem sterilisierten Adapter und einer sterilisierten Verschluss.

Die DE 10 2017 104 931 A1 betrifft ein Verfahren zur insbesondere thermischen Sterilisation einer Spritze, wobei die Spritze zur Aufnahme bzw. Abgabe mindestens eines medizinischen Befüllungsgutes geeignet ist. Der Spritzenkörper weist einen Aufnahmeraum zur Aufnahme des Befüllungsgutes auf, wobei der Aufnahmeraum über eine Öffnung mit mindestens einer lösbaren bzw. verschiebbaren Dichteinrichtung abdichtend verschließbar ist. Die Dichteinrichtung weist eine mit der Wandung der Spritze in Kontakt stehende, dem Aufnahmeraum zugewandte bzw. der Öffnung abgewandte erste Dichtlamelle und mindestens eine mit der Wandung in Kontakt stehende, dem Aufnahmeraum abgewandte bzw. der Öffnung zugewandte zweite Dichtlamelle auf, wobei die erste Dichtlamelle und die zweite Dichtlamelle einen Zwischenraum begrenzen. Die zweite Dichtlamelle soll dabei mit mindestens einer Ausnehmung versehen sein, insbesondere derart, dass eine Sterilisationsatmosphäre durch die Ausnehmung in den durch die beiden Dichtlamellen begrenzten Zwischenraum einzudringen imstande ist.

Die EP 3 888 630 A1 betrifft ein Verfahren zur Herstellung einer sterilen oxybutyninhaltigen Zusammensetzung in einer vorzugsweise anwendungsfertigen Kolbenspritze. Der Spritzenkörper soll dabei aus einem Cycloolefin-(Co-)Polymer (COP bzw. COC) gebildet sein.

Darüber hinaus können Anticholinergika topisch appliziert werden, beispielsweise mittels eines transdermalen Pflasters oder dergleichen. Auch bei dieser systemischen Applikation können im erhöhten Maße systemische Nebenwirkungen auftreten. Zudem besteht auch hier das Problem einer vorzeitigen Verstoffwechselung.

Darüber hinaus können Anticholinergika, wie Oxybutynin bzw. Oxybutyninhydrochlorid, topisch in den Urogenitalbereich, insbesondere in die Harnblase, appliziert bzw. instilliert werden, beispielsweise über einen Katheter oder dergleichen. Durch die diesbezüglich lokale Applikation bzw. die diesbezüglich vorgesehene Instillation erfolgt eine Darreichung der Wirksubstanz unmittelbar an seinem Wirkort, wodurch die gezielte Wirkung bzw. die Bioverfügbarkeit erhöht und systemische Nebenwirkungen reduziert werden. Zudem wird der Wirkstoff bei dieser Verabreichungsart nicht vorzeitig über andere Organe verstoffwechselt. Zudem kann eine diesbezügliche Verabreichung gegebenenfalls auch von dem Patienten selbst vorgenommen werden.

Oxybutynin bzw. Oxybutyninhydrochlorid wirkt dabei insbesondere als kompetitiver Antagonist von Acetylcholin auf postganglionäre Muskarinrezeptoren, was zu einer Entspannung bzw. krampflösenden Wirkung im Hinblick auf die glatte Blasenmuskulatur und somit auch zu einer Abnahme der Detrusorhyperaktivität führt. Insbesondere wird die Blasenkontraktion gehemmt, wobei auch Spasmen gelockert werden. Zudem führt die Verabreichung von Oxybutynin bzw. Oxybutyninhydrochlorid zu einer Vergrößerung des Blasenvolumens und zu einer Verringerung von Kontraktionen, wobei auch der Drang zum Harnlassen bei neurogenen Blasenfunktionsstörungen verringert bzw. verzögert wird. Weiterhin kann durch den Einsatz von Oxybutynin bzw. Oxybutyninhydrochlorid auch der intravesikale (Blasen-)Druck verringert werden, was auch zu einer Entlastung der oberen Harnwege führt. Darüber hinaus weist Oxybutynin bzw. Oxybutyninhydrochlorid Eigenschaften eines Lokalanästhetikums auf. Oxybutynin bzw. Oxybutyninhydrochlorid gehört dabei insbesondere zur Gruppe der Parasymphatolytika.

Im Rahmen der im Stand der Technik vorgesehenen Instillation bzw. topischen Applikation in die Harnblase wird Oxybutynin bzw. Oxybutyninhydrochlorid im Allgemeinen in Form einer wässrigen Zusammensetzung (wässrigen Formulierungen) bzw. wässrigen Lösung eingesetzt, wobei die wirkstoffhaltige Zusammensetzung bzw. Lösung unter Einsatz entsprechender Applikationsvorrichtungen in die Harnblase appliziert bzw. verabreicht wird.

Dabei kommt der Sterilität einer für die Instillation bzw. topischen Verabreichung in die Harnblase eingesetzten Zusammensetzung eine hohe Bedeutung zu, und zwar insbesondere vor dem Hintergrund, etwaige Infektionen der hierfür besonders anfälligen und mitunter durch das Krankheitsbild bereits vorgeschädigten Harnblase zu vermeiden.

Um eine Verkeimung zu vermeiden, können die wirkstoffhaltigen Zusammensetzungen beispielsweise unmittelbar vor deren Anwendung bzw. Verabreichung hergestellt werden, was aber aufwendig ist und zudem noch mit einer gewissen Gefahr der Kontamination der Zusammensetzung bei deren Herstellung einhergeht. Weiterhin können die wirkstoffhaltigen Zusammensetzungen als solche sterilisiert werden, beispielsweise mittels Mikrofiltrationsverfahren oder dergleichen, und anschließend in eine Aufbewahrungs- bzw. Applikationsvorrichtung abgefüllt werden. Auch hierbei kann es jedoch zu einer unerwünschten Kontaminationen kommen, insbesondere im Hinblick auf mögliche an der Aufbewahrungs- bzw. Verabreichungsvorrichtung anhaftende Keime oder dergleichen. In diesem Zusammenhang kann auch eine separate Sterilisation der Aufbewahrungs- bzw. Applikationsvorrichtung vorgesehen sein, was aber gleichermaßen zu mitunter nicht zufriedenstellenden Sterilisationsergebnissen führt, insbesondere bedingt durch das nachträgliche Abfüllen der Zusammensetzung in die Aufnahme- bzw. Applikationsvorrichtung. In diesem Zusammenhang kann zudem unter sterilen Bedingungen, beispielsweise unter Reinraumbedingungen, gearbeitet werden, was aber mit einem gewissen Aufwand einhergeht und zudem kostenintensiv ist.

Problematisch bei der Sterilisation ist darüber hinaus auch, dass der Wirkstoffgehalt in der zugrundeliegenden wässrigen Zusammensetzung bzw. wässrigen Formulierung mitunter nicht aufrechterhalten werden kann, wobei auch davon ausgegangen worden ist, dass ein Abbau der Wirksubstanz in Form von Oxybutynin bzw. Oxybutyninhydrochlorid, einhergehend mit der Bildung unerwünschter Abbauprodukte, vorliegt, beispielsweise infolge einer unerwünschten Wechselwirkung der Wirksubstanz mit Materialien, welche in direktem Kontakt mit der zugrundeliegenden wässrigen Zusammensetzung stehen, wie Materialien entsprechender Aufbewahrungs- bzw. Applikationsvorrichtungen oder dergleichen. Die Verringerung der Wirkstoffmenge in einer zugrundeliegenden Zusammensetzung bzw. die Bildung unerwünschter Abbauprodukte kann jedoch die Wirksamkeit als auch die Anwendungssicherheit einer entsprechenden Zusammensetzung negativ beeinflussen, so dass diese nach erfolgter Sterilisation mitunter nicht mehr eingesetzt werden kann, was auch mit einem entsprechenden wirtschaftlichen Verlust einhergeht.

In diesem Zusammenhang ist bislang insbesondere davon ausgegangen worden, für Oxybutynin bzw. Oxybutyninhydrochlorid in wässrigen Zusammensetzungen insbesondere bei thermischer Sterilisation eine Inkompatibilität bzw. Instabilität vorliegt, und zwar insbesondere für den Fall, dass die wässrigen Zusammensetzungen mit dem Oxybutynin bzw. Oxybutyninhydrochlorid in (Einweg-)Spritzen auf Basis von Polypropylen (PP) als Spritzenmaterial vorliegen, wobei zudem davon ausgegangen worden ist, dass die Inkompatibilität bzw. Instabilität mitunter noch dadurch verstärkt wird, dass für die Spritze im Allgemeinen Kolbenstopfen bzw. Gummistopfen sowie Verschlusskappen aus üblicherweise eingesetzten Gummimaterialien verwendet werden.

Bei derartigen Systemen ist bislang insbesondere auch bei der (Dampf-) Sterilisation eine mitunter deutliche Verringerung der Wirkstoffmenge in Form des Oxybutynins bzw. Oxybutyninhydrochlorids bei Durchführung der Sterilisation, insbesondere Dampfsterilisation, gefunden worden, so dass bislang davon ausgegangen worden ist, dass derartige (Einweg-)Spritzen auf Basis von Polypropylen, insbesondere in Kombination mit der Verwendung von Kolbenstopfen bzw. Gummistopfen sowie Verschlusskappen aus üblicherweise eingesetzten Gummimaterialien, nicht für entsprechende wässrige Zusammensetzungen in Betracht kommen, welche Oxybutynin bzw. Oxybutyninhydrochlorid als Wirkstoff enthalten. Im Hinblick auf die Verwendung von (Einweg-)Spritzen mit diesbezüglichem Spritzenzylinder und Kolbenstange aus Polypropylen als Primärpackmittel für entsprechende wässrige Zusammensetzungen ist man somit bislang davon ausgegangen, dass eine hierin eingebrachte wässrige Zusammensetzung unter Verwendung von Oxybutynin bzw. Oxybutyninhydrochlorid als Wirkstoff insbesondere bei der Sterilisation nicht stabil ist, wobei maßgeblich von einem Abbau des aktiven Wirkstoffs in Form von Oxybutynin bzw. Oxybutyninhydrochlorid insbesondere in sein Hydrolyseprodukt ausgegangen worden ist, einhergehend mit einer entsprechenden Verringerung der Wirkstoffmenge bzw. -konzentration in der Zusammensetzung.

Vor diesem Hintergrund wird im Stand der Technik beispielsweise ein Konzept verfolgt, wonach (Einweg-)Spritzen auf Basis andersartiger Materialien als Primärpackmittel bzw. zur Aufbewahrung und Sterilisation von Oxybutynin bzw. Oxybutyninhydrochlorid enthaltenden wässrigen Zusammensetzungen verwendet werden, wie beispielsweise Spritzen aus Cycloolefin-(Co-)Polymer (COP bzw. COC). Durch die Verwendung entsprechender (Einweg-)Spritzen auf Basis von COP bzw. COC, welche zudem insbesondere als Kolbenspritzen ausgebildet sind, soll die Zusammensetzung im Hinblick auf den Wirkstoff in Form von Oxybutynin bzw. Oxybutyninhydrochlorid insbesondere auch bei der Sterilisation dahingehend stabilisiert werden, dass ein Wirkstoffverlust in der Zusammensetzung verringert wird, um auf dieser Basis sterile und anwendungsfertige Zusammensetzungen mit konstantem bzw. möglichst unverändertem Wirkstoffgehalt und mit verbesserten Produkt- bzw. Lagereigenschaften bereitzustellen. Entsprechende (Einweg-) Spritzen aus COP bzw. COC weisen mitunter jedoch eine relativ hohe Kratzempfindlichkeit auf und sind zudem insbesondere aufgrund der relativ hohen Starrheit des Materials in gewisser Weise zerbrechlich und empfindlicher gegenüber mechanischen Einwirkungen sowie gegenüber starken Temperaturschwankungen, was deren Handhabbarkeit auch bei einer durchzuführenden (Wärme-)Sterilisation mitunter beeinflusst. Zudem sind (Einweg-)Spritzen aus COP bzw. COC relativ kostenintensiv und somit weniger wirtschaftlich.

Insgesamt handelt es sich somit bei (Einweg-)Spritzen aus COP bzw. COC um ein solches Primärpackmittel für eine wässrige Zusammensetzung mit Oxybutynin bzw. Oxybutyninhydrochlorid als Wirkstoff, welches im Allgemeinen zwar eine hohe (Lager-)Stabilität darin aufgenommener und sterilisierter Zusammensetzungen gewährleistet, jedoch insgesamt kostenintensiv ist und mitunter auch mit erhöhten Anforderungen hinsichtlich der Durchführung einer (Wärme-)Sterilisation einhergeht.

Insgesamt besteht somit im Stand der Technik ein großer Bedarf an entsprechenden Konzepten, die eine effektive Sterilisierung von in entsprechenden Aufnahme- bzw. Applikationsvorrichtungen vorliegenden wässrigen Zusammensetzungen mit Oxybutynin bzw. Oxybutyninhydrochlorid als Wirksubstanz ermöglichen, und dies unter Beibehaltung der Stabilität der Zusammensetzungen, um auf dieser Basis zu anwendungsfertigen Produkten zu gelangen, welche im Hinblick auf die Instillation in die Harnblase bei hoher Wirksamkeit entsprechend anwendungssicher sind (d. h. bei hohem Wirkstoffgehalt bzw. geringer Menge an Abbauprodukten nicht zu Infektionen oder Fehlanwendungen bei der Anwendung führen). Dabei soll auch eine hohe (Lager-)Stabilität der Zusammensetzungen gegeben sein und zudem auch der kostenspezifische Aspekt berücksichtigt sein.

Vor diesem Hintergrund besteht eine Aufgabe der vorliegenden Erfindung darin, ein effizientes Verfahren zur Herstellung einer sterilen Zusammensetzung, welche Oxybutynin bzw. Oxybutyninhydrochlorid als Wirkstoff aufweist und welche in einer vorzugsweise anwendungsfertigen Aufbewahrungs- bzw. Applikationsvorrichtung, nämlich einer Kolbenspritze, vorliegt, bereitzustellen, wobei die zuvor geschilderten Nachteile des Standes der Technik zumindest weitgehend vermieden oder aber wenigstens abgeschwächt werden sollen.

**In** diesem Zusammenhang ist eine Aufgabe der vorliegenden Erfindung insbesondere darin zu sehen, ein diesbezügliches Verfahren bereitzustellen, welches zu einer anwendungsfertigen Aufbewahrungs- bzw. Applikationsvorrichtung führt, welche eine wässrige Zusammensetzung mit Oxybutynin bzw. Oxybutyninhydrochlorid als Wirksubstanz in steriler Form enthält, wobei die Zusammensetzung auch nach durchgeführter Sterilisation neben einer hohen Keimfreiheit gleichermaßen einen hohen bzw. unveränderten Wirkstoffgehalt bzw. allenfalls einen geringen Gehalt an entsprechenden Abbauprodukten aufweist. Insbesondere soll im Rahmen der vorliegenden Erfindung ein möglicher Wirkstoffverlust in der Zusammensetzung bzw. die mögliche Entstehung von Abbauprodukten des Wirkstoffs minimiert bzw. auf ein pharmazeutisch akzeptables Maß reduziert werden.

Diesbezüglich soll im Rahmen der vorliegenden Erfindung insbesondere ein solches Verfahren bereitgestellt werden, welches gleichermaßen eine hohe Kosteneffizienz aufweist, und zwar auch im Hinblick auf die im Rahmen des bereitgestellten Verfahrens eingesetzten Materialien bzw. Komponenten. Weiterführend soll auch eine gute Handhabbarkeit der verfahrensgemäß eingesetzten Materialien bzw. Komponenten insbesondere auch im Hinblick auf durchzuführende Sterilisationsbehandlungen gewährleistet werden. Zudem soll das erfindungsgemäß bereitgestellte Verfahren auch im industriellen bzw. großtechnischen Maßstab anwendbar bzw. skalierbar sein.

Im Rahmen der vorliegenden Erfindung soll zudem insbesondere ein solches Verfahren bereitgestellt werden, anhand dessen eine entsprechend keimfreie Zusammensetzung mit Oxybutynin bzw. Oxybutyninhydrochlorid als Wirksubstanz bereitgestellt wird, welche bei hoher Keimfreiheit bzw. Sterilität auch eine hohe Wirksamkeit und Verträglichkeit aufweist. Dabei soll auch eine verbesserte Anwendungssicherheit und eine verbesserte (Lager-)Stabilität bzw. Haltbarkeit gewährleistet werden. Insbesondere soll auf dieser Basis ein anwendungsfertiges Produkt bereitgestellt werden, bei welchem die entsprechende Zusammensetzung in einer Aufbewahrungs- bzw. Applikationsvorrichtung eingebracht vorliegt, und zwar in einer diesbezüglichen (Einweg-)Spritze bzw. Kolbenspritze.

Weiterführend soll gemäß einer weiteren Aufgabe der vorliegenden Erfindung auch eine wirkstoffhaltige Zusammensetzung als solche bzw. eine mit der wirkstoffhaltigen Zusammensetzung befüllte Aufbewahrungs- bzw. Applikationsvorrichtung, und zwar in Form einer (Einweg-)Spritze bzw. Kolbenspritze, bereitgestellt werden. **In** diesem Zusammenhang soll die (Einweg-)Spritze bzw. Kolbenspritze in verbesserter Weise einem Sterilisationsverfahren zugänglich sein, wobei eine effektive Sterilisation unter Beibehaltung der Stabilität des Befüllungsgutes in Form der wirkstoffhaltigen Zusammensetzung gewährleistet sein soll.

Vorliegend beschrieben ist ein Verfahren zur Herstellung einer sterilen oxybutyninhaltigen Zusammensetzung in einer vorzugsweise anwendungsfertigen Kolbenspritze.

Vorliegend beschrieben ist zudem eine sterile oxybutyninhaltige Zusammensetzung, wie sie insbesondere zur Verwendung bei der prophylaktischen bzw. therapeutischen Behandlung von neurogenen Blasenfunktionsstörungen eingesetzt werden soll.

Gegenstand der vorliegenden Erfindung - gemäß einem **ersten** Aspekt der vorliegenden Erfindung - ist die erfindungsgemäße Kolbenspritze, welche die sterile oxybutyninhaltige Zusammensetzung aufweist, gemäß dem diesbezüglichen, die erfindungsgemäße Kolbenspritze betreffenden unabhängigen Patentanspruch; jeweils vorteilhafte Weiterbildungen und Ausgestaltungen dieses Erfindungsaspekts sind Gegenstand der die erfindungsgemäße Kolbenspritze betreffenden Unteransprüche.

Gegenstand der vorliegenden Erfindung - gemäß einem **zweiten** Aspekt der vorliegenden Erfindung - ist zudem auch die erfindungsgemäße Verpackungseinheit, welche die erfindungsgemäße Kolbenspritze mit der Zusammensetzung enthält, gemäß dem diesbezüglichen, die Verpackungseinheit nach der Erfindung betreffenden unabhängigen Patentanspruch; vorteilhafte Weiterbildungen und Ausgestaltungen dieses Erfindungsaspekts sind Gegenstand des die erfindungsgemäße Verpackungseinheit betreffenden Unteranspruchs.

Weiterführend sind auch Gegenstand der vorliegenden Erfindung - gemäß einem **dritten** Aspekt der vorliegenden Erfindung - die erfindungsgemäßen Kits (*Kit-of-parts*) gemäß den diesbezüglichen, die erfindungsgemäßen Kits betreffenden unabhängigen Patentansprüchen.

Vorliegend beschrieben ist auch die Verwendung einer speziellen Kolbenspritze in einem Verfahren zur Herstellung einer sterilen oxybutyninhaltigen Zusammensetzung.

Schließlich ist vorliegend beschrieben die Verwendung einer Hitzesterilisation, insbesondere Wasserdampfsterilisation, zur Bereitstellung einer sterilen oxybutyninhaltigen Zusammensetzung.

Es versteht sich von selbst, dass Ausgestaltungen, Ausführungsformen, Vorteile und dergleichen, welche nachfolgend zu Zwecken der Vermeidung von Wiederholungen zur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Darüber hinaus verhält es sich im Hinblick auf die nachfolgende Beschreibung der vorliegenden Erfindung auch derart, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Bei allen nachstehend genannten relativen bzw. prozentualen gewichtsbezogenen Angaben, insbesondere Mengen- bzw. Konzentrationsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, zu 100 % bzw. 100 Gew.- % ergänzen bzw. addieren; dies versteht sich aber für den Fachmann von selbst.

Weiterhin gilt, dass der Fachmann - anwendungsbezogen oder einzelfallbedingt -von den nachfolgend angeführten Konzentrations-, Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren und andernfalls mit dem Fachmann auf diesem Gebiet an sich geläufigen Bestimmungs- bzw. Messmethoden ermittelt bzw. bestimmt werden können. Sofern nicht anders angegeben, werden die zugrundeliegenden Werte bzw. Parameter unter Standardbedingungen (d. h. insbesondere bei einer Temperatur von 20 °C und/oder bei einem Druck von 1.013,25 hPa bzw. 1,01325 bar) bzw. Atmosphärendruck ermittelt.

Dies vorausgeschickt wird im Folgenden die vorliegende Erfindung im Detail erläutert:
Im Rahmen der vorliegenden Erfindung und wie nachfolgend noch im Detail angeführt, hat die Anmelderin gefunden, dass unter Verwendung spezieller (Einweg-)Spritzen bzw. Kolbenspritzen auf Basis von Polypropylen (PP) als Spritzenmaterial insbesondere im Rahmen einer Hitzesterilisation, insbesondere Dampfsterilisation, zum Erhalt steriler oxybutyninhaltiger Zusammensetzungen auch eine Migration bzw. Einlagerung von Oxybutynin in den Spritzenkörper vorliegt, wobei erfindungsgemäß eine derartige Migration bzw. Einlagerung gesteuert bzw. kompensiert wird, wie es auch für einen möglichen Abbau von Oxybutynin in der Zusammensetzung zu entsprechenden Abbauprodukten der Fall ist. Zudem stellt die vorliegende Erfindung auch auf eine Kontrolle des pH-Werts bzw. diesbezüglicher Veränderungen in der Zusammensetzung ab. Im Rahmen der vorliegenden Erfindung können dabei unter Verwendung polypropylenbasierter Kolbenspritzen anwendungsfertige Produkte auf Basis steriler oxybutyninhaltiger Zusammensetzungen bereitgestellt werden.

Gegenstand der vorliegenden Erfindung - gemäß einem **ersten** Aspekt der vorliegenden Erfindung - ist die erfindungsgemäße Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper, insbesondere Spritzenzylinder, aus einem Polypropylen (PP) gebildet ist,
wobei die Kolbenspritze mit einer sterilen oxybutyninhaltigen Zusammensetzung (Endzusammensetzung), insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere wie nachfolgend definiert, befüllt ist,
wobei die Kolbenspritze erhältlich ist bzw. erhalten ist nach einem wie nachfolgend definierten Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze,
wobei die nach Abschluss des Verfahrens, insbesondere nach Verfahrensschritt b), erhaltene oxybutyninhaltige Endzusammensetzung als sterile wässrige Zusammensetzung, insbesondere terminal sterilisierte Zusammensetzung, ausgebildet ist und die nachfolgend angeführten Spezifikationsvorgaben (i), (ii) und (iii) aufweist:
   (i) vorgegebene Spezifikations-Konzentration von Oxybutynin, bevorzugt in Form des Hydrochlorids, mit vorgegebener Abweichung von höchstens ± 5 %, bezogen auf die Konzentration von Oxybutynin,
   (ii) vorgegebener Spezifikations-pH-Wert mit vorgegebener Abweichung von höchstens ± 0,5 pH-Werteinheiten,
   (iii) vorgegebene Spezifikations-Maximalmenge an Oxybutynin-Abbauprodukt(en), insbesondere reaktiv hervorgerufenen Oxybutynin-Abbauprodukt(en), vorzugsweise hydrolytisch und/oder oxidativ hervorgerufenen Oxybutynin-Abbauprodukt(en), vorzugsweise Phenylcyclohexylhydroxyessigsäure;
wobei das Verfahren die nachfolgenden Schritte umfasst:
   a) Bereitstellen einer oxybutyninhaltigen Ausgangszusammensetzung in der Kolbenspritze und/oder Befüllen der Kolbenspritze mit einer oxybutyninhaltigen Ausgangszusammensetzung
      und nachfolgend
   b) Hitzesterilisation, insbesondere Dampfsterilisation, vorzugsweise Wasserdampfsterilisation, bevorzugt terminale Sterilisation, der oxybutyninhaltigen Ausgangszusammensetzung in der Kolbenspritze und/oder der mit der oxybutyninhaltigen Ausgangszusammensetzung befüllten Kolbenspritze zum Erhalt der sterilen oxybutyninhaltigen Endzusammensetzung in der vorzugsweise anwendungsfertigen Kolbenspritze und/oder zum Erhalt der vorzugsweise anwendungsfertigen, sterilen, mit der oxybutyninhaltigen Zusammensetzung befüllten Kolbenspritze,
wobei die im Verfahren, insbesondere in Verfahrensschritt b), auftretende Migration und/oder Einlagerung des Oxybutynins in den Spritzenkörper und/oder in den Kolbenstopfen, und der im Verfahren, insbesondere in Verfahrensschritt b), auftretende Abbau des Oxybutynins, insbesondere der reaktive Abbau des Oxybutynins, vorzugsweise der hydrolytisch und/oder oxidativ hervorgerufene Abbau, bevorzugt der Abbau zu Phenylcyclohexylhydroxyessigsäure, gesteuert und/oder kompensiert wird derart und mit der Maßgabe,
dass die nach Durchführung von Verfahrensschritt b) erhaltene sterile oxybutyninhaltige Endzusammensetzung die zuvor angeführten Spezifikationsvorgaben (i), (ii) und (iii) erfüllt und die sterile oxybutyninhaltige Endzusammensetzung in der vorzugsweise anwendungsfertigen Kolbenspritze bzw. die vorzugsweise anwendungsfertige, sterile, mit der oxybutyninhaltigen Endzusammensetzung befüllte Kolbenspritze erhalten wird,
wobei die im Verfahren, insbesondere in Verfahrensschritt b), auftretende Migration und/oder Einlagerung des Oxybutynins in den Spritzenkörper und/oder in den Kolbenstopfen, und der im Verfahren, insbesondere in Verfahrensschritt b), auftretende Abbau, insbesondere der reaktive Abbau des Oxybutynins, vorzugsweise der hydrolytisch und/oder oxidativ hervorgerufene Abbau des Oxybutynins, bevorzugt der Abbau zu Phenylcyclohexylhydroxyessigsäure, durch die nachfolgenden Maßnahmen und/oder Schritte (1), (2), (3) und (4) gesteuert und/oder kontrolliert wird, insbesondere verhindert und/oder minimiert und/oder kompensiert wird:
   (1) Einstellung und/oder Verwendung einer im Vergleich zur Spezifikations-Konzentration und/oder Spezifikations-Menge des Oxybutynins, bevorzugt in Form des Hydrochlorids, erhöhte Konzentration und/oder Menge an Oxybutynin, bevorzugt in Form des Hydrochlorids, in der Ausgangszusammensetzung;
   (2) Einstellung eines im Vergleich zu dem Spezifikations-pH-Wert abweichenden, insbesondere erhöhten pH-Werts in der Ausgangszusammensetzung;
   (3) Einstellung und/oder Auswahl der Sterilisationsbedingungen, insbesondere ausgewählt aus der Gruppe von Sterilisationsart, Sterilisationsdauer, Sterilisationstemperatur, Sterilisationsdruck, Sterilisationsatmosphäre sowie deren Kombinationen, vorzugsweise Einstellung und/oder Auswahl des FO-Werts der Sterilisation;
   (4) Ausrüstung und/oder Behandlung, insbesondere Beschichtung, der Innenwandung des Spritzenkörpers mit mindestens einem migrations- und/oder abbauverringernden Mittel, wobei das migrations- und/oder abbauverringernde Mittel ein siliciumhaltiges, insbesondere silikon- oder silikatbasiertes Mittel ist.

Insbesondere betrifft die vorliegende Erfindung gemäß diesem Aspekt auch die zuvor definierte erfindungsgemäße Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper, insbesondere Spritzenzylinder, aus einem Polypropylen (PP) gebildet ist,
wobei die Kolbenspritze mit einer sterilen oxybutyninhaltigen Zusammensetzung (Endzusammensetzung), insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung als sterile wässrige Zusammensetzung, insbesondere terminal sterilisierte Zusammensetzung, ausgebildet ist und die nachfolgend angeführten Spezifikationsvorgaben (i), (ii) und (iii) aufweist:
   (i) vorgegebene Spezifikations-Konzentration von Oxybutynin, bevorzugt in Form des Hydrochlorids (Oxybutyninhydrochlorid, Oxybutynin-HCl):
      0,5 mg/ml bis 2 mg/ml mit maximaler Abweichung von ± 5 %, bezogen auf die Konzentration von Oxybutynin, vorzugsweise 1 mg/ml mit maximaler Abweichung von ± 0,05 mg/ml,
   (ii) vorgegebener Spezifikations-pH-Wert:
      2,8 bis 5,2, insbesondere 3 bis 5, vorzugsweise 3,2 bis 4,8, bevorzugt 3,5 bis 4,5, besonders bevorzugt 3,5 bis 4,2, ganz besonders bevorzugt 3,8 bis 4,2,
   (iii) vorgegebene Spezifikations-Menge an Oxybutynin-Abbauprodukt(en), insbesondere reaktiv hervorgerufenen Oxybutynin-Abbauprodukt(en), vorzugsweise hydrolytisch und/oder oxidativ hervorgerufenen Oxybutynin-Abbauprodukt(en), vorzugsweise Phenylcyclohexylhydroxyessigsäure (Verunreinigung D):
      höchstens 0,5 Gew.-%, insbesondere höchstens 0,2 Gew.-%, bezogen auf die Gesamtmenge an eingesetztem Oxybutynin, bevorzugt Oxybutyninhydrochlorid;
wobei die in der Kolbenspritze enthaltene oxybutyninhaltige Zusammensetzung einschließlich der Kolbenspritze hitzesterilisiert, insbesondere dampfsterilisiert, vorzugsweise wasserdampfsterilisiert, bevorzugt terminal (hitze-, dampf-, wasserdampf-)sterilisiert, ist bzw. worden ist.

Die oxybutyninhaltige Zusammensetzung kann dabei insbesondere auch die folgenden Spezifikationsvorgaben aufweisen bzw. erfüllen:
- So kann die oxybutyninhaltige Zusammensetzung als weitere Spezifikationsvorgabe (iv) mindestens einen Elektrolyten, insbesondere Alkalimetallsalz, vorzugsweise Alkalimetallchlorid, bevorzugt Natriumchlorid (NaCl), aufweisen, insbesondere in einer vorgegebenen Spezifikations-Menge im Bereich von 0,1 Gew.-% bis 2 Gew.-%, insbesondere im Bereich von 0,5 Gew.-% bis 1,5 Gew.-%, vorzugsweise im Bereich von 0,7 Gew.-% bis 1,2 Gew.-%, bevorzugt von etwa 0,9 Gew.-%, bezogen auf die Endzusammensetzung, insbesondere in einer vorgegebenen Spezifikationsmenge.
- Zudem kann die oxybutyninhaltige Zusammensetzung als weitere Spezifikationsvorgabe (iv) Natriumchlorid, berechnet als Natrium, in einer vorgegebenen Spezifikations-Konzentration von (3,5 ± 0,5) mg/ml, insbesondere (3,5 ± 0,3) mg/ml, vorzugsweise (3,5 ± 0,1) mg/ml, bevorzugt etwa 3,5 mg/ml, enthalten.
- Darüber hinaus kann die oxybutyninhaltige Zusammensetzung als weitere Spezifikationsvorgabe (v) eine vorgegebene Spezifikations-Menge an Gesamt-Abbauprodukten von höchstens 1 Gew.-%, insbesondere höchstens 0,5 Gew.-%, bezogen auf die Gesamtmenge an eingesetztem Oxybutynin, bevorzugt Oxybutyninhydrochlorid, aufweisen.

Erfindungsgemäß kann die Kolbenspritze, bevorzugt der Spritzenkörper, insbesondere Spritzenzylinder, ein Volumen, insbesondere ein Aufnahmevolumen für die oxybutyninhaltige Zusammensetzung, im Bereich von 1 ml bis 100 ml, insbesondere im Bereich von 2 ml bis 50 ml, vorzugsweise im Bereich von 3 ml bis 25 ml, bevorzugt im Bereich von 4 ml bis 25 ml, besonders bevorzugt im Bereich von 6 ml bis 20 ml, weiter bevorzugt im Bereich von 8 ml bis 15 ml, nochmals weiter bevorzugt im Bereich von 9 ml bis 12 ml, ganz besonders bevorzugt von etwa 10 ml, aufweisen. Gleichermaßen kann die Kolbenspritze, bevorzugt der Spritzenkörper, insbesondere Spritzenzylinder, die oxybutyninhaltige Zusammensetzung in einer Menge im Bereich von 1 ml bis 100 ml, insbesondere im Bereich von 2 ml bis 50 ml, vorzugsweise im Bereich von 3 ml bis 25 ml, bevorzugt im Bereich von 4 ml bis 25 ml, besonders bevorzugt im Bereich von 6 ml bis 20 ml, weiter bevorzugt im Bereich von 8 ml bis 15 ml, nochmals weiter bevorzugt im Bereich von 9 ml bis 12 ml, ganz besonders bevorzugt von etwa 10 ml, aufweisen.

Was die erfindungsgemäße Kolbenspritze weiterhin anbelangt, so kann der diesbezügliche Spritzenkolben insbesondere aus Polypropylen gebildet sein bzw. hieraus bestehen.

Weiterhin kann der Kolbenstopfen aus einem elastomeren Material, insbesondere aus einem Kautschukmaterial und/oder Gummimaterial, vorzugsweise Kautschukmaterial, gebildet sein bzw. hieraus bestehen. Gemäß einer erfindungsgemäß bevorzugten Ausführungsform kann der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet sein bzw. hieraus bestehen.

Erfindungsgemäß kann es sich insbesondere derart verhalten, dass die Kolbenspritze, insbesondere der Spritzenkörper, vorzugsweise der Spritzenzylinder, eine Aufnahme- und/oder Befüllöffnung aufweist, insbesondere zum Befüllen des Spritzenkörpers, insbesondere des Spritzenzylinders, mit der oxybutyninhaltigen Zusammensetzung und/oder insbesondere zur vorzugsweise abdichtenden Aufnahme des Spritzenkolbens mit dem Kolbenstopfen und/oder insbesondere wobei die Aufnahme- und/oder Befüllöffnung mit dem Spritzenkolben, insbesondere mit dem Kolbenstopfen, verschließbar ist und/oder verschließbar ausgebildet ist und/oder verschlossen ist.

Weiterhin kann es erfindungsgemäß vorgesehen sein, dass der Spritzenkörper einen Adapter, insbesondere zum Anschluss einer Instillationsvorrichtung und/oder zum Austragen und/oder Applizieren der oxybutyninhaltigen Zusammensetzung, aufweist.

In diesem Zusammenhang kann es insbesondere vorgesehen sein, dass der Spritzenkörper einstückig (einteilig) ausgebildet ist. In diesem Zusammenhang kann es gleichermaßen vorgesehen sein, dass der Adapter mit dem Spritzenkörper, insbesondere Spritzenzylinder, eine verbundene, insbesondere fest und/oder unlösbar verbundene, vorzugsweise stoffschlüssig verbundene, Einheit ausbildet. **In** diesem Zusammenhang kann es auch vorgesehen sein, dass der Adapter mit dem Spritzenkörper, insbesondere Spritzenzylinder, verbunden, insbesondere fest und/oder unlösbar verbunden, vorzugsweise stoffschlüssig verbunden, ist.

Erfindungsgemäß kann es vorgesehen sein, dass der Adapter eine Auslassöffnung, insbesondere am freien Ende des Adapters und/oder insbesondere zum Austragen und/oder Applizieren der oxybutyninhaltigen Zusammensetzung, aufweist. Zudem kann der Adapter eine Einlassöffnung, insbesondere im Übergangsbereich zwischen Adapter und Spritzenzylinder und/oder insbesondere zur Aufnahme der oxybutyninhaltigen Zusammensetzung aus dem Spritzenzylinder, aufweisen.

Insbesondere ist es erfindungsgemäß vorgesehen, dass der Adapter hohl ausgebildet ist.

Insbesondere kann der Adapter einen zwischen der Auslassöffnung des Adapters und der Einlassöffnung des Adapters befindlichen Hohlraum, insbesondere Durchlasskanal, insbesondere für den Auslass der oxybutyninhaltigen Zusammensetzung, aufweisen. In diesem Zusammenhang kann sich der Hohlraum, insbesondere Durchlasskanal, von der Einlassöffnung des Adapters zu der Auslassöffnung des Adapters erstrecken bzw. die Einlassöffnung mit der Auslassöffnung verbinden.

Erfindungsgemäß kann es auch vorgesehen sein, dass der Adapter in Auslassrichtung und/oder in Richtung der Auslassöffnung des Adapters verjüngt bzw. zulaufend ausgebildet ist.

Zudem kann der Adapter in Auslassrichtung und/oder in Richtung der Auslassöffnung des Adapters einen abnehmenden und/oder sich verringernden Umfang, insbesondere Außenumfang, und/oder einen abnehmenden und/oder sich verringernden Durchmesser, insbesondere Außendurchmesser, aufweisen.

Erfindungsgemäß kann der Adapter insbesondere auch wie folgt ausgebildet sein:
- So kann der Adapter düsenförmig bzw. als Düse, insbesondere als konus- oder zylinderförmige Düse, vorzugsweise ohne Gewinde, bevorzugt als Luer-Slip, oder aber mit Gewinde, insbesondere Schraubgewinde, bevorzugt als Luer-Lock, ausgebildet sein.
- Weiterhin kann der Adapter konisch oder stufenkegelförmig, vorzugsweise stufenkegelförmig, ausgebildet sein.
- Gemäß einer erfindungsgemäß bevorzugten Ausführungsform kann der Adapter als Stufenkegeladapter ausgebildet sein. In diesem Zusammenhang kann der Adapter eine Anzahl an Stufen im Bereich von 2 Stufen bis 40 Stufen, insbesondere im Bereich von 3 Stufen bis 30 Stufen, vorzugsweise im Bereich von 5 Stufen bis 25 Stufen, bevorzugt im Bereich von 8 Stufen bis 20 Stufen, besonders bevorzugt im Bereich von 10 Stufen bis 15 Stufen, aufweisen. In diesem Zusammenhang kann es zudem vorgesehen sein, dass der Umfang, insbesondere Außenumfang, und/oder der Durchmesser, insbesondere Außendurchmesser, der Stufen des Adapters in Auslassrichtung und/oder in Richtung der Auslassöffnung des Adapters abnimmt. Auch kann es in diesem Zusammenhang vorgesehen sein, dass das Verhältnis des Durchmessers, insbesondere Außendurchmessers, des Adapters an der Einlassöffnung des Adapters zu dem Durchmesser, insbesondere Außendurchmesser, des Adapters an der Auslassöffnung des Adapters in einem Bereich von 1,1 bis 15, insbesondere in einem Bereich von 1,25 bis 10, vorzugsweise in einem Bereich von 1,5 bis 5, bevorzugt in einem Bereich von 2 bis 4, besonders bevorzugt in einem Bereich von 2,25 bis 3, liegt.

Erfindungsgemäß kann es sich insbesondere derart verhalten, dass die Kolbenspritze einen stufenkegelförmig ausgebildeten Adapter bzw. einen Stufenkegeladapter aufweist, wobei der stufenkegelförmige Adapter bzw. Stufenkegeladapter mit dem Spritzenkörper, insbesondere Spritzenzylinder, eine verbundene, insbesondere fest bzw. unlösbar verbundene, vorzugsweise stoffschlüssig verbundene, Einheit ausbildet bzw. wobei der Adapter mit dem Spritzenkörper, insbesondere Spritzenzylinder, einstückig ausgebildet ist.

Weiterhin kann die erfindungsgemäße Kolbenspritze wie folgt ausgestaltet sein:
- So kann die Kolbenspritze ein insbesondere abnehmbares und/oder entfernbares und/oder lösbares Verschlusselement, insbesondere eine Verschlusskappe (Verschluss- und/oder Dichtkappe), vorzugsweise zum insbesondere abdichtenden Anliegen an den Adapter und/oder vorzugsweise zum insbesondere abdichtenden Aufsetzen auf den Adapter und/oder vorzugsweise zum abdichtenden Verschließen der Auslassöffnung und/oder des Adapters, aufweisen.
- Insbesondere verhält es sich erfindungsgemäß derart, dass die Auslassöffnung und/oder der Adapter mit dem Verschlusselement verschlossen ist. Insbesondere ist die Kolbenspritze bzw. die Auslassöffnung und/oder der Adapter während der Durchführung des vorliegend beschriebenen Verfahrens, insbesondere in Verfahrensschritt b), mit dem Verschlusselement verschlossen.
- Insbesondere kann das Verschlusselement den Adapter, insbesondere mitsamt der Auslassöffnung, zumindest im Wesentlichen vollständig und/oder abdichtend umschließen bzw. abdecken, insbesondere zumindest im Wesentlichen über die gesamte Außenfläche und/oder (Außen-)Wandung des Adapters und/oder insbesondere zumindest im Wesentlichen über die gesamte Auslassöffnung des Adapters. Insbesondere kann das Verschlusselement dem vorzugsweise als Stufenkegeladapter ausgebildeten Adapter zumindest im Wesentlichen vollständig bzw. abdichtend umschließen bzw. abdecken, insbesondere zumindest im Wesentlichen über die gesamte Außenfläche bzw. (Außen-) Wandung des als Stufenkegeladapter ausgebildeten Adapters.

Erfindungsgemäß ist somit insbesondere vorgesehen, dass das Verschlusselement zum insbesondere abdichtenden Anliegen an den Adapter und/oder zum insbesondere abdichtenden Aufsetzen auf den Adapter und/oder vorzugsweise zum abdichtenden Verschließen der Auslassöffnung des Adapters und/oder zum im Wesentlichen vollständigen und/oder abdichtenden Umschließen des Adapters ausgebildet ist.

Im Allgemeinen kann das Verschlusselement im Rahmen der vorliegenden Erfindung kappen- bzw. hutförmig ausgebildet sein.

Zudem kann das Verschlusselement einen Aufnahmeraum, insbesondere zur zumindest im Wesentlichen vollständigen Aufnahme und/oder zum zumindest im Wesentlichen vollständigen Umschließen des Adapters, aufweisen.

Insbesondere kann es erfindungsgemäß vorgesehen sein, dass das Verschlusselement form- und/oder kraftschlüssig, insbesondere reibschlüssig, an dem Adapter anliegt und/oder anliegbar ausgebildet ist und/oder wobei das Verschlusselement den Adapter form- und/oder kraftschlüssig, insbesondere reibschlüssig, umschließt und/oder umschließbar ausgebildet ist.

Erfindungsgemäß kann das Verschlusselement im Allgemeinen aus einem elastomeren Material, insbesondere aus einem Gummimaterial und/oder Kautschukmaterial, gebildet sein. Gemäß einer erfindungsgemäß bevorzugten Ausführungsform kann das Verschlusselement aus einem Halogenbutylkautschuk, insbesondere aus einem Chlorbutylkautschuk oder aus einem Brombutylkautschuk, vorzugsweise aus einem Brombutylkautschuk, gebildet sein bzw. hieraus bestehen.

Gemäß einer erfindungsgemäß bevorzugten Ausführungsform kann es zudem vorgesehen sein, dass das Verschlusselement eine Abnehm- und/oder Entfernungseinrichtung, insbesondere Abzieheinrichtung, vorzugsweise in Form eines insbesondere nippelförmig ausgebildeten Fortsatzes und/oder vorzugsweise zum insbesondere manuellen Abnehmen, insbesondere Abziehen, des Verschlusselements von dem Adapter, aufweist. In diesem Zusammenhang kann die Abnehm- und/oder Entfernungseinrichtung an dem und/oder im Bereich des geschlossenen Endes und/oder an der Spitze des Verschlusselements und/oder in Verlängerung der Längsachse des Verschlusselements angeordnet sein. Hierdurch kann die Bedienung des Verschlusselements insgesamt erleichtert sein, insbesondere auch was das Greifen des Verschlusselements mit der Hand und insbesondere das Abnehmen des Verschlusselements von dem Adapter anbelangt, insbesondere im Hinblick auf die Anwendung der erfindungsgemäßen Kolbenspritze bei der Verabreichung der sterilen oxybutyninhaltigen Zusammensetzung.

Zudem kann das Verschlusselement an dessen Außenseite mindestens eine Vertiefung, insbesondere Einkerbung, Einbuchtung und/oder Materialverjüngung, aufweisen. In diesem Zusammenhang kann die Vertiefung längsförmig und/oder linienförmig und/oder gradlinig verlaufend ausgebildet sein. Zudem kann die Vertiefung zumindest im Wesentlichen in Längsrichtung und/oder zumindest im Wesentlichen parallel zur Längsachse des Verschlusselements verlaufend angeordnet sein. Insbesondere kann das Verschlusselement in diesem Zusammenhang zwei, drei, vier, fünf oder mehr, insbesondere zwei, drei oder vier, bevorzugt vier, Vertiefungen aufweisen. Dabei können die Vertiefungen zumindest im Wesentlichen äquidistant zueinander verlaufend und/oder zumindest im Wesentlichen parallel zueinander verlaufend auf der Außenseite des Verschlusselements angeordnet sein.

Erfindungsgemäß kann die Kolbenspritze, insbesondere der Adapter bzw. das Verschlusselement, körperlich gemäß der DE 10 2020 130 032 A1 und/oder gemäß der DE 20 2020 106 531 U1 ausgebildet sein, wie zuvor angeführt. Diesbezüglich gelten auch die in den jeweiligen Dokumenten angeführten Vorteile zu den jeweiligen Ausgestaltungen vorliegend in entsprechender Weise.

Was die erfindungsgemäße Kolbenspritze weiterhin anbelangt, so kann diese unter mindestens einer der insbesondere nachfolgend im Hinblick auf das vorliegend beschriebene Verfahren definierten Bedingungen hitzesterilisiert, insbesondere dampfsterilisiert, vorzugsweise wasserdampfsterilisiert, bevorzugt terminal (hitze-, dampf-, wasserdampf-)sterilisiert, sein bzw. worden sein.

Insbesondere kann die Kolbenspritze mit einem FO-Wert im Bereich von 6 min bis 45 min, insbesondere im Bereich von 8 min bis 40 min, vorzugsweise im Bereich von 10 min bis 38 min, bevorzugt im Bereich von 11 min bis 35 min, besonders bevorzugt im Bereich von 15 min bis 32 min, ganz besonders bevorzugt im Bereich von 19 min bis 30 min, hitzesterilisiert, insbesondere dampfsterilisiert, vorzugsweise wasserdampfsterilisiert, bevorzugt terminal (hitze-, dampf-, wasserdampf-) sterilisiert, sein bzw. worden sein.

Zudem kann die Kolbenspritze unter overkill-Bedingungen und/oder bei etwa 121 °C und/oder für einen Zeitraum von mindestens 6 min, vorzugsweise von etwa 6 min, oder von mindestens 20 min, vorzugsweise von etwa 20 min, hitzesterilisiert, insbesondere dampfsterilisiert, vorzugsweise wasserdampfsterilisiert, bevorzugt terminal (hitze-, dampf-, wasserdampf-)sterilisiert, sein bzw. worden sein.

Darüber hinaus kann es insbesondere vorgesehen sein, dass die Kolbenspritze gemäß Pharmacopoea Europaea (Ph. Eur.), Kapitel 5.1.1 (07/2017:50101), insbesondere unter overkill-Bedingungen und/oder bei etwa 121 °C und/oder für einen Zeitraum von mindestens 15 min, insbesondere von mindestens 20 min, vorzugsweise von etwa 20 min, hitzesterilisiert, insbesondere dampfsterilisiert, vorzugsweise wasserdampfsterilisiert, bevorzugt terminal (hitze-, dampf-, wasserdampf-)sterilisiert, ist bzw. worden ist. Die diesbezügliche Hitzesterilisation, insbesondere Dampfsterilisation, bezieht sich dabei auf die Zusammensetzung einschließlich der diesbezüglichen Kolbenspritze, in welcher sich die Zusammensetzung befindet.

Insbesondere kann die Zusammensetzung auch im Hinblick auf die erfindungsgemäße Kolbenspritze bzw. die Kolbenspritze mit der hierin aufgenommenen sterilen Zusammensetzung folgende Eigenschaften aufweisen:
So kann die sterile oxybutyninhaltige Zusammensetzung die vorgegebenen Spezifikationsvorgaben (i) sowie (ii) und/oder (iii), insbesondere (i), (ii) und (iii), auch nach Lagerung der mit der oxybutyninhaltigen Zusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweisen bzw. erfüllen.

Zudem kann die sterile oxybutyninhaltige Zusammensetzung die nachfolgend angeführten Spezifikationsvorgaben (i) sowie (ii) und/oder (iii), insbesondere (i), (ii) und (iii), auch nach Lagerung der mit der oxybutyninhaltigen Zusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweisen und/oder erfüllen:
(i) vorgegebene Spezifikations-Konzentration von Oxybutynin, bevorzugt in Form des Hydrochlorids (Oxybutyninhydrochlorid, Oxybutynin-HCl):
   0,5 mg/ml bis 2 mg/ml mit maximaler Abweichung von ± 5 %, bezogen auf die Konzentration von Oxybutynin, vorzugsweise 1 mg/ml mit maximaler Abweichung von ± 0,05 mg/ml,
(ii) vorgegebener Spezifikations-pH-Wert:
   2,8 bis 5,2, insbesondere 3 bis 5, vorzugsweise 3,2 bis 4,8, bevorzugt 3,5 bis 4,5, besonders bevorzugt 3,5 bis 4,2, ganz besonders bevorzugt 3,8 bis 4,2,
(iii) vorgegebene Spezifikations-Menge an Oxybutynin-Abbauprodukt(en), insbesondere reaktiv hervorgerufenen Oxybutynin-Abbauprodukt(en), vorzugsweise hydrolytisch und/oder oxidativ hervorgerufenen Oxybutynin-Abbauprodukt(en), vorzugsweise Phenylcyclohexylhydroxyessigsäure (Verunreinigung D):
   höchstens 0,5 Gew.-%, insbesondere höchstens 0,2 Gew.-%, bezogen auf die Gesamtmenge an eingesetztem Oxybutynin, bevorzugt Oxybutyninhydrochlorid.

Insbesondere kann die sterile Zusammensetzung die vorgegebenen Spezifikationsvorgabe (iv), insbesondere die vorgegebenen Spezifikations-Menge (iv) an Elektrolyten, insbesondere Alkalimetallsalz, vorzugsweise Alkalimetallchlorid, bevorzugt Natriumchlorid (NaCl), im Bereich von 0,1 Gew.-% bis 2 Gew.-%, insbesondere im Bereich von 0,5 Gew.-% bis 1,5 Gew.-%, vorzugsweise im Bereich von 0,7 Gew.-% bis 1,2 Gew.-%, bevorzugt von etwa 0,9 Gew.-%, bezogen auf die Endzusammensetzung, und/oder die vorgegebenen Spezifikations-Konzentration (iv) an Natriumchlorid, berechnet als Natrium, von (3,5 ± 0,5) mg/ml, insbesondere (3,5 ± 0,3) mg/ml, vorzugsweise (3,5 ± 0,1) mg/ml, bevorzugt etwa 3,5 mg/ml, auch nach Lagerung der mit der oxybutyninhaltigen Zusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweisen bzw. erfüllen.

Zudem kann die sterile oxybutyninhaltige Zusammensetzung die vorgegebenen Spezifikationsvorgabe (v), insbesondere die vorgegebene Spezifikations-Menge an Gesamt-Abbauprodukten von höchstens 1 Gew.-%, insbesondere höchstens 0,5 Gew.-%, bezogen auf die Gesamtmenge an eingesetztem Oxybutynin, bevorzugt Oxybutyninhydrochlorid, auch nach Lagerung der mit der oxybutyninhaltigen Zusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweisen bzw. erfüllen.

Darüber hinaus kann die oxybutyninhaltige Zusammensetzung und/oder die mit der oxybutyninhaltigen Zusammensetzung befüllte sterile Kolbenspritze einen SAL-Wert (*Sterility* Assurance) von mindestens 10⁻⁵, insbesondere mindestens 10⁻⁶, vorzugsweise mindestens 10⁻⁷, aufweisen.

Die erfindungsgemäße Kolbenspritze eignet sich insbesondere zur Instillation bzw. zur vorzugsweise topischen Applikation der oxybutyninhaltigen Zusammensetzung in den Urogenitalbereich, insbesondere in die Harnblase, insbesondere unter Verwendung einer entsprechenden Applikations- bzw. Instillationsvorrichtung, insbesondere in Form eines Instillationsschlauches oder dergleichen bzw. in Form eines (Harnblasen-)-Katheters.

Im Hinblick auf weitere Ausgestaltungen der erfindungsgemäßen Kolbenspritze kann auch auf die Ausführungen zu den weiteren erfindungsgemäße bzw. vorliegend beschriebenen Aspekten verwiesen werden, welche vorliegend entsprechend gelten.

Vorliegend beschrieben - gemäß einem vorliegend beschriebenen Aspekt- ist ein Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze,
wobei die Kolbenspritze einen Spritzenkörper, insbesondere Spritzenzylinder, einerseits und einen Spritzenkolben mit einem Kolbenstopfen andererseits aufweist, wobei der Spritzenkörper, insbesondere Spritzenzylinder, aus einem Polypropylen (PP) gebildet ist, und
wobei die nach Abschluss des Verfahrens, insbesondere nach Verfahrensschritt b), erhaltene oxybutyninhaltige Endzusammensetzung als sterile wässrige Zusammensetzung, insbesondere terminal sterilisierte Zusammensetzung, ausgebildet ist und die nachfolgend angeführten Spezifikationsvorgaben (i) sowie (ii) und/oder (iii), vorzugsweise (i), (ii) und (iii), aufweist:
   (i) vorgegebene Spezifikations-Konzentration von Oxybutynin, bevorzugt in Form des Hydrochlorids (Oxybutyninhydrochlorid, Oxybutynin-HCl), mit vorgegebener Abweichung von höchstens ± 5 %, bezogen auf die Konzentration von Oxybutynin,
   (ii) vorgegebener Spezifikations-pH-Wert mit vorgegebener Abweichung von höchstens ± 0,5 pH-Werteinheiten,
   (iii) vorgegebene Spezifikations-Maximalmenge an Oxybutynin-Abbauprodukt(en), insbesondere reaktiv hervorgerufenen Oxybutynin-Abbauprodukt(en), vorzugsweise hydrolytisch und/oder oxidativ hervorgerufenen Oxybutynin-Abbauprodukt(en), vorzugsweise Phenylcyclohexylhydroxyessigsäure (Verunreinigung D);
wobei das Verfahren die nachfolgenden Schritte umfasst:
   a) Bereitstellen einer oxybutyninhaltigen Ausgangszusammensetzung in der Kolbenspritze und/oder Befüllen der Kolbenspritze mit einer oxybutyninhaltigen Ausgangszusammensetzung
      und nachfolgend
   b) Hitzesterilisation, insbesondere Dampfsterilisation, vorzugsweise Wasserdampfsterilisation, bevorzugt terminale (Hitze-, Dampf-, Wasserdampf-) Sterilisation, der oxybutyninhaltigen Ausgangszusammensetzung in der Kolbenspritze und/oder der mit der oxybutyninhaltigen Ausgangszusammensetzung befüllten Kolbenspritze zum Erhalt der sterilen oxybutyninhaltigen Endzusammensetzung in der vorzugsweise anwendungsfertigen Kolbenspritze und/oder zum Erhalt der vorzugsweise anwendungsfertigen, sterilen, mit der oxybutyninhaltigen Zusammensetzung befüllten Kolbenspritze,
wobei die im Verfahren, insbesondere in Verfahrensschritt b), auftretende Migration und/oder Einlagerung des Oxybutynins in den Spritzenkörper, insbesondere Spritzenzylinder, und/oder in den Kolbenstopfen, und/oder der im Verfahren, insbesondere in Verfahrensschritt b), auftretende Abbau des Oxybutynins, insbesondere der reaktive Abbau des Oxybutynins, vorzugsweise der hydrolytisch und/oder oxidativ hervorgerufene Abbau, bevorzugt der Abbau zu Phenylcyclohexylhydroxyessigsäure (Verunreinigung D), gesteuert und/oder kompensiert wird derart und mit der Maßgabe,
dass die nach Durchführung von Verfahrensschritt b) erhaltene sterile oxybutyninhaltige Endzusammensetzung die zuvor angeführten Spezifikationsvorgaben (i) sowie (ii) und/oder (iii), vorzugsweise (i), (ii) und (iii), erfüllt und die sterile oxybutyninhaltige Endzusammensetzung in der vorzugsweise anwendungsfertigen Kolbenspritze bzw. die vorzugsweise anwendungsfertige, sterile, mit der oxybutyninhaltigen Endzusammensetzung befüllte Kolbenspritze erhalten wird.

Folgendes sei ergänzend zum Verständnis der zuvor genannten Spezifikationsvorgaben (i) und (ii) bzw. zum Verständnis von Patentanspruch 1 anzuführen: Bei den Spezifikationsvorgaben (i) und (ii) handelt es sich jeweils um einen Einzelwert (d. h. um einen punktuellen (Einzel-)Wert), welcher durch die für die jeweilige Spezifikationsvorgabe angeführte Abweichung begrenzt ist. Ist beispielsweise die Spezifikations-Konzentration des Oxybutynins gemäß (i) mit 1 mg/ml vorgegeben, resultiert hieraus ein vorgegebenes Spezifikations-Konzentration-Regime von 1 mg/ml ± 5 % bzw. von (1 ± 0,05) mg/ml bzw. von 0,95 bis 1,05 mg/ml. Entsprechendes gilt für den Spezifikations-pH-Wert gemäß (ii): Ist beispielsweise ein Spezifikations-pH-Wert gemäß (ii) von 4,0 vorgegeben, resultiert hieraus ein vorgegebenes Spezifikations-pH-Wert-Regime von 4,0 ± 0,5 bzw. von 3,5 bis 4,5.

Gemäß dem vorliegenden Aspekt ist auch beschrieben ein Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze, insbesondere ein zuvor definiertes Verfahren,
wobei die Kolbenspritze einen Spritzenkörper, insbesondere Spritzenzylinder, einerseits und einen Spritzenkolben mit einem Kolbenstopfen andererseits aufweist, wobei der Spritzenkörper, insbesondere Spritzenzylinder, aus einem Polypropylen (PP) gebildet ist, und
wobei die nach Abschluss des Verfahrens, insbesondere nach Verfahrensschritt b), erhaltene oxybutyninhaltige Endzusammensetzung als sterile wässrige Zusammensetzung, insbesondere terminal sterilisierte Zusammensetzung, ausgebildet ist und die nachfolgend angeführten Spezifikationsvorgaben (i) sowie (ii) und/oder (iii), vorzugsweise (i), (ii) und (iii), aufweist:
   (i) vorgegebene Spezifikations-Konzentration von Oxybutynin, bevorzugt in Form des Hydrochlorids (Oxybutyninhydrochlorid, Oxybutynin-HCl):
      0,5 mg/ml bis 2 mg/ml mit maximaler Abweichung von ± 5 %, bezogen auf die Konzentration von Oxybutynin, vorzugsweise 1 mg/ml mit maximaler Abweichung von ± 0,05 mg/ml (d. h. wobei die vorgegebene Spezifikations-Konzentration von Oxybutynin als Einzelwert bzw. als punktueller (Einzel-)Wert aus dem vorgenannten Bereich von 0,5 mg/ml bis 2 mg/ml auszuwählen oder vorzugeben ist, wobei sich die maximale Abweichung von ± 5 % auf den auszuwählenden oder vorzugebenden Einzelwert bzw. punktuellen (Einzel-)Wert bezieht),
   (ii) vorgegebener Spezifikations-pH-Wert:
      2,8 bis 5,2, insbesondere 3 bis 5, vorzugsweise 3,2 bis 4,8, bevorzugt 3,5 bis 4,5, besonders bevorzugt 3,5 bis 4,2, ganz besonders bevorzugt 3,8 bis 4,2 (d.h. wobei der vorgegebene Spezifikations-pH-Wert als Einzelwert bzw. als punktueller (Einzel-)Wert aus den vorgenannten Bereichen von 2,8 bis 5,2, insbesondere 3 bis 5, vorzugsweise 3,2 bis 4,8, bevorzugt 3,5 bis 4,5, besonders bevorzugt 3,5 bis 4,2, ganz besonders bevorzugt 3,8 bis 4,2, auszuwählen oder vorzugeben ist, wobei sich die maximale Abweichung von ± 0,5 auf den auszuwählenden oder vorzugebenden Einzelwert bzw. punktuellen (Einzel-)Wert bezieht),
   (iii) vorgegebene Spezifikations-Menge an Oxybutynin-Abbauprodukt(en), insbesondere reaktiv hervorgerufenen Oxybutynin-Abbauprodukt(en), vorzugsweise hydrolytisch und/oder oxidativ hervorgerufenen Oxybutynin-Abbauprodukt(en), vorzugsweise Phenylcyclohexylhydroxyessigsäure (Verunreinigung D):
      höchstens 0,5 Gew.-%, insbesondere höchstens 0,2 Gew.-%, bezogen auf die Gesamtmenge an eingesetztem Oxybutynin, bevorzugt Oxybutyninhydrochlorid;
wobei das Verfahren die nachfolgenden Schritte umfasst:
   a) Bereitstellen einer oxybutyninhaltigen Ausgangszusammensetzung in der Kolbenspritze und/oder Befüllen der Kolbenspritze mit einer oxybutyninhaltigen Ausgangszusammensetzung
      und nachfolgend
   b) Hitzesterilisation, insbesondere Dampfsterilisation, vorzugsweise Wasserdampfsterilisation, bevorzugt terminale (Hitze-, Dampf-, Wasserdampf-) Sterilisation, der oxybutyninhaltigen Ausgangszusammensetzung in der Kolbenspritze und/oder der mit der oxybutyninhaltigen Ausgangszusammensetzung befüllten Kolbenspritze zum Erhalt der sterilen oxybutyninhaltigen Endzusammensetzung in der vorzugsweise anwendungsfertigen Kolbenspritze und/oder zum Erhalt der vorzugsweise anwendungsfertigen, sterilen, mit der oxybutyninhaltigen Zusammensetzung befüllten Kolbenspritze,
wobei die im Verfahren, insbesondere in Verfahrensschritt b), auftretende Migration und/oder Einlagerung des Oxybutynins in den Spritzenkörper, insbesondere Spritzenzylinder, und/oder in den Kolbenstopfen, und/oder der im Verfahren, insbesondere in Verfahrensschritt b), auftretende Abbau des Oxybutynins, insbesondere der reaktive Abbau des Oxybutynins, vorzugsweise der hydrolytisch und/oder oxidativ hervorgerufene Abbau, bevorzugt der Abbau zu Phenylcyclohexylhydroxyessigsäure (Verunreinigung D), gesteuert und/oder kompensiert wird derart und mit der Maßgabe,
dass die nach Durchführung von Verfahrensschritt b) erhaltene sterile oxybutyninhaltige Endzusammensetzung die zuvor angeführten Spezifikationsvorgaben (i) sowie (ii) und/oder (iii), vorzugsweise (i), (ii) und (iii), erfüllt und die sterile oxybutyninhaltige Endzusammensetzung in der vorzugsweise anwendungsfertigen Kolbenspritze bzw. die vorzugsweise anwendungsfertige, sterile, mit der oxybutyninhaltigen Endzusammensetzung befüllte Kolbenspritze erhalten wird.

Folgendes sei ergänzend zum Verständnis der zuvor genannten Spezifikationsvorgaben (i) und (ii) bzw. zum Verständnis von Patentanspruch 2 anzuführen: Wie zuvor bereits zum Verständnis von Patentanspruch 1 ausgeführt, handelt es sich bei den Spezifikationsvorgaben (i) und (ii) jeweils um einen Einzelwert (d. h. um einen punktuellen (Einzel-)Wert), welcher durch die für die jeweilige Spezifikationsvorgabe angeführte Abweichung begrenzt ist. Ist beispielsweise die Spezifikations-Konzentration des Oxybutynins gemäß (i) mit 1 mg/ml vorgegeben, resultiert hieraus ein vorgegebenes Spezifikations-Konzentration-Regime von 1 mg/ml ± 5 % bzw. von (1 ± 0,05) mg/ml bzw. von 0,95 bis 1,05 mg/ml. Entsprechendes gilt für den Spezifikations-pH-Wert gemäß (ii): Ist beispielsweise ein Spezifikations-pH-Wert gemäß (ii) von 4,0 vorgegeben, resultiert hieraus ein vorgegebenes Spezifikations-pH-Wert-Regime von 4,0 ± 0,5 bzw. von 3,5 bis 4,5. Entsprechendes gilt auch für den nachfolgend noch beschriebenen Spezifikationsparameter (iv).

Im Hinblick auf die obigen Angaben zu der Spezifikations-Konzentration von Oxybutynin ist die Angabe der maximalen Abweichung von ± 5 % bzw. ± 0,05 mg/ml also als Maximalabweichung zu verstehen (so dass in Bezug auf die angeführten Abweichungen auch sämtliche Zwischenwerte mitumfasst sind; d. h. die Angabe ± 5 % umfasst den Bereich von + 5 % bis - 5 % bzw. die Angabe ± 0,05 mg/ml umfasst den Bereich von + 0,05 mg/ml bis - 0,05 mg/ml, so dass sich beispielsweise für eine vorgegebene Spezifikations-Konzentration von Oxybutynin bzw. Oxybutyninhydrochlorid von 1 mg/ml ein entsprechender Spezifikations-Konzentrationswertebereich von 0,95 mg/ml bis 1,05 mg/ml ergibt, wobei auch die entsprechenden Zwischenwerte mitumfasst sind). Entsprechendes gilt auch für den Spezifikations-pH-Wert gemäß (ii) und den nachfolgend noch beschriebenen Spezifikationsparameter (iv).

Die spezielle Verfahrensführung unter Einbezug der erfindungsgemäß vorgesehenen Hitzesterilisation, insbesondere Dampfsterilisation, führt zu einer effektiven Sterilisation einer oxybutyninhaltigen (End-)Zusammensetzung in der zugrundeliegenden Kolbenspritze, insbesondere Einweg-Kolbenspritze, wobei die erhaltene sterile oxybutyninhaltige (End-)Zusammensetzung in gezielter Weise auf konkrete Spezifikationsvorgaben eingestellt werden kann, wie insbesondere in Form der Konzentration von Oxybutynin, des pH-Werts sowie der Menge an Oxybutynin-Abbauprodukten, wie zuvor angeführt. Auf Basis des vorliegend beschriebenen Verfahrens können somit sterile oxybutyninhaltige Zusammensetzungen mit definierten Eigenschaften insbesondere im Hinblick auf eine vorgegebene Wirkstoffkonzentration, sowie einem vorgegebenem pH-Wert - und dies bei gleichzeitig niedrigem Gehalt an Abbauprodukten bereitgestellt werden, welche folglich bei hoher Wirksamkeit in Bezug auf die zugrundeliegende Indikation aufgrund der vorliegenden Sterilität und der geringen Menge an Abbauprodukten anwendungssicher sind.

Dabei ist es im Rahmen der vorliegenden Erfindung völlig überraschend, dass eine sterile oxybutyninhaltige Zusammensetzung mit definierten Eigenschaften hinsichtlich der zugrundeliegenden Spezifikationsvorgaben in einer Kolbenspritze mit einem Spritzenkörper, insbesondere Spritzenzylinder, aus bzw. auf Basis eines Polypropylens (PP) erhalten werden kann, da diesbezüglich im Stand der Technik bislang davon ausgegangen worden ist, dass polypropylenbasierte Kolbenspritzen eine hohe Inkompatibilität gegenüber dem Wirkstoff Oxybutynin bzw. Oxybutyninhydrochlorid insbesondere im Hinblick auf einen Abbau bzw. Entstehung von Oxybutynin-Abbauprodukten insbesondere bei der Sterilisation aufweisen. Darüber hinaus hat die Anmelderin - völlig überraschend - im Rahmen der vorliegenden Erfindung gefunden, dass im Hinblick auf den Wirkstoff Oxybutynin bzw. Oxybutyninhydrochlorid eine insbesondere thermisch induzierte bzw. insbesondere bei der Hitzesterilisation, vorzugsweise Dampfsterilisation, auftretende Migration bzw. Einlagerung des Oxybutynins, und zwar insbesondere in Form der freien Aminbase, insbesondere in den Spritzenkörper aus Polypropylen (PP) und auch in dem Kolbenstopfen vorliegt, wobei diese Migration bzw. Einlagerung des Oxybutynins im Rahmen der vorliegenden Erfindung durch die gezielten technischen Maßnahmen, wie sie im Rahmen des vorliegend beschriebenen Verfahrens angeführt sind, gesteuert bzw. kompensiert werden kann, so dass auf dieser Basis die zuvor angeführten Spezifikationsvorgaben in Bezug auf die sterile oxybutyninhaltige (End-)-Zusammensetzung erreicht bzw. eingehalten werden können und insbesondere bei der Sterilisation ein nur geringer Verlust an Wirksubstanz vorliegt.

Die Anmelderin konnte in überraschender Weise auch zeigen, dass im Rahmen der vorliegenden Erfindung auf Basis der sehr speziellen Verfahrensführung mit der gezielten Abfolge und Ausgestaltung der zugrundeliegenden Verfahrensschritte zum einen ein insbesondere thermisch induzierter Abbau von Oxybutynin zu entsprechenden Oxybutynin-Abbauprodukten, insbesondere in Form der Verunreinigung D, vermindert bzw. verhindert werden kann bzw. als solcher allenfalls nur in geringem bzw. pharmazeutisch akzeptablem Maß auftritt.

In diesem Zusammenhang konnte die Anmelderin zudem zeigen, dass zumindest im Wesentlichen kein Abbau von Oxybutynin erfolgt bzw. der diesbezügliche Abbau verhindert bzw. auf ein pharmazeutisch akzeptables Minimum reduziert wird, wenn erfindungsgemäß vorgegangen wird, wie nachfolgend ausgeführt. Zudem konnte die Anmelderin erstmals zeigen, dass im Hinblick auf die Verwendung von Kolbenspritzen auf Basis von Polypropylen eine Migration bzw. Einlagerung von Oxybutynin vorliegt und dass diese in überraschender Weise kontrolliert bzw. kompensiert bzw. vergleichmäßigt werden kann, so dass insgesamt eine lagerstabile sterile oxybutyninhaltige Zusammensetzung bzw. damit befüllte Kolbenspritzen unter Einhaltung der Spezifikationsvorgaben bereitgestellt werden können.

Auf Basis des vorliegend beschriebenen Verfahrens können somit erstmals entsprechende (Einweg-)Spritzen bzw. Kolbenspritzen auf Basis von Polypropylen bzw. mit diesbezüglichen Spritzenkörpern aus Polypropylen als Aufbewahrungs- bzw. Applikationsvorrichtung für oxybutyninhaltige Zusammensetzungen eingesetzt werden, und zwar auch im Hinblick auf die Bereitstellung entsprechend steriler Zusammensetzungen unter Einsatz von Hitzesterilisation und insbesondere von Dampfsterilisationsmethoden.

Somit können im Rahmen des vorliegend beschriebenen Verfahrens die mit auf Polypropylen basierten (Einweg-)Spritzen, insbesondere Kolbenspritzen, einhergehenden Nachteile überwunden werden, wobei erfindungsgemäß sowohl der Abbau der Wirksubstanz zu entsprechenden Abbauprodukten, insbesondere zu der Verunreinigung D, als auch die seitens der Anmelderin aufgefundene Migration bzw. Einlagerung des Oxybutynins in die Kolbenspritze bzw. den Spritzenkörper entsprechend gesteuert bzw. kompensiert werden können. Wie nachfolgend noch im Detail angeführt, kann die Steuerung bzw. Kompensation beispielsweise durch Vorgabe bzw. Einstellung der Konzentration bzw. Menge an Oxybutynin bzw. Oxybutyninhydrochlorid, der Einstellung bzw. Vorgabe des pH-Werts in der zugrundeliegenden Ausgangszusammensetzung bzw. durch Einstellung bzw. Auswahl der Sterilisationsbedingungen sowie durch eine gezielte Ausrüstung bzw. Behandlung der Kolbenspritze bzw. des Spritzenkörpers erfolgen.

Im Rahmen der vorliegenden Erfindung ist es somit insgesamt gelungen, eine sterile oxybutyninhaltige Zusammensetzung (Endzusammensetzung), welche in einer Kolbenspritze auf Basis von Polypropylen eingebracht vorliegt, bereitzustellen, welche auch nach Hitzesterilisation, insbesondere Dampfsterilisation, eine definierte bzw. vorgegebene Konzentration an Oxybutynin bzw. Oxybutyninhydrochlorid bei gleichermaßen eingestelltem bzw. vorgegebenem pH-Wert und definierter Menge an Verunreinigungen aufweist. Erfindungsgemäß wird dabei insbesondere darauf abgestellt, den bei der Hitzesterilisation, insbesondere Dampfsterilisation, auftretenden Verlust an Oxybutynin, welcher insbesondere bedingt ist durch die Migration bzw. Einlagerung in das Spritzenmaterial sowie durch den Abbau bzw. die Bildung des entsprechenden Hydrolyseprodukts, insbesondere in Form der Verunreinigung D, durch gezielte Maßnahmen hinsichtlich der zugrundeliegenden Ausgangszusammensetzung bzw. der eingesetzten Hitzesterilisation bzw. des zugrundeliegenden Spritzenmaterials zu steuern bzw. zu kompensieren, und zwar vor dem Hintergrund der Minimierung des Wirkstoffverlustes in der Zusammensetzung sowie Aufrechterhaltung eines bestimmten pH-Werts.

Wie nachfolgend noch angeführt, hat die Anmelderin in diesem Zusammenhang auch gefunden, dass der bei der Sterilisation auftretende Verluste an Wirksubstanz auch von dem pH-Wert sowie der Menge bzw. Konzentration des Wirkstoffs in Form von Oxybutynin bzw. Oxybutyninhydrochlorid in der Ausgangszusammensetzung abhängt bzw. hierdurch gesteuert werden können.

Insbesondere ermöglicht die vorliegend beschriebene Verfahrensführung auch die Bereitstellung einer spezifikationskonformen oxybutyninhaltigen Zusammensetzung in steriler Form.

Im Rahmen des vorliegend beschriebenen Verfahrens kann zudem die Migration bzw. Einlagerung des Oxybutynins in den für die Kolbenspritze eingesetzten Kolbenstopfen kontrolliert bzw. kompensiert werden, wobei die diesbezügliche Wechselwirkung durch Auswahl spezieller Materialien für den Kolbenstopfen noch weiterführend verringert werden können, wie nachfolgend ausgeführt. Gleichermaßen kann auch ein mit dem Kolbenstopfen im Zusammenhang stehender Abbau des Oxybutynins weiterführend verringert werden.

Entsprechendes gilt auch für die nachfolgend noch beschriebene Verschlusselement. Folglich kann erfindungsgemäß auch eine Migration bzw. Einlagerung des Oxybutynins in das Verschlusselement kontrolliert bzw. kompensiert werden und/oder ein mit dem Verschlusselement im Zusammenhang stehender Abbau des Oxybutynins weiterführend verringert werden, wie auch nachfolgend angeführt.

Ein weiterer Vorteil des vorliegend beschriebenen Verfahrens ist zudem darin zu sehen, dass mit der Verwendung von Kolbenspritzen auf Basis von Polypropylen eine insgesamt kostengünstige Aufbewahrungs- bzw. Applikationsvorrichtung als Primärverpackung eingesetzt werden kann, was auch mit einer Kostenoptimierung des zugrundeliegenden Verfahrens insgesamt und damit auch der resultierenden Produkte einhergeht. Darüber hinaus weisen die erfindungsgemäß eingesetzten Kolbenspritzen auf Basis von Polypropylen auch anwendungs- bzw. einsatzspezifische Vorteile im Hinblick auf eine hohe Kratzunempfindlichkeit und hohe Temperaturbeständigkeit bei gleichzeitiger hoher mechanischer Belastbarkeit und verringerter Starrheit des Materials auf. Nicht zuletzt von daher kann das vorliegend beschriebene Verfahren in effizienter Weise auch im industriellen bzw. großtechnischen Einsatz realisiert werden.

Ohne sich auf diese Theorie beschränken oder berufen zu wollen, steht die im Rahmen der vorliegenden Erfindung gefundene und gezielt kontrollierte bzw. kompensierte Migration bzw. Einlagerung von Oxybutynin insbesondere in den Spritzenkörper bzw. in den Kolbenstopfen bzw. der insbesondere bei der Hitzesterilisation auftretende Abbau des Oxybutynins auch mit den chemischstrukturellen bzw. physikochemischen Eigenschaften von Oxybutynin im Zusammenhang.

So kann Oxybutynin in wässrigen Lösungen auf Basis einer sowohl sauren als auch basischen Hydrolyse zur korrespondierenden freien Säure reagieren bzw. abgebaut werden, nämlich zu Phenylcyclohexylhydroxyessigsäure (vgl. nachfolgende Strukturformel (2)), wobei diese Verunreinigung als sogenannte Verunreinigung D ("*Impurity D"* bzw. "*imp. D*") bezeichnet wird. Im Allgemeinen wird Oxybutynin (vgl. nachfolgende Strukturformel (1)) in wässrigen Lösungen aufgrund der verbesserten Löslichkeit in wässrigen Medien als Hydrochlorid eingesetzt. Hierbei koordiniert HCl an der freien Aminbase, wobei in sauren Lösungen die Wirksubstanz insbesondere in seiner komplexierten Form gemäß Strukturformel (3) vorliegt (wobei Entsprechendes auch für den Feststoff gilt). In der Zusammensetzung kann sich dabei ein Gleichgewicht des Wirkstoffs gemäß (1) und (3) einstellen, und zwar auch in Abhängigkeit von dem pH-Wert.

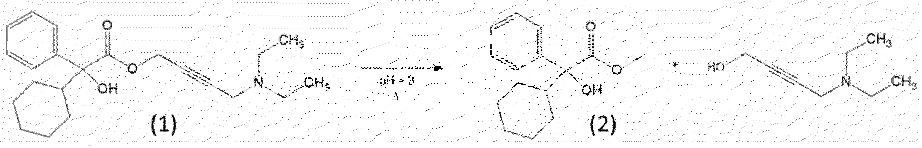

Die obige Abbildung zeigt die Hydrolyse von Oxybutynin (Strukturformel 1) zu der Verunreinigung D in Form von Phenylcyclohexylhydroxyessigsäure (Strukturformel 2).

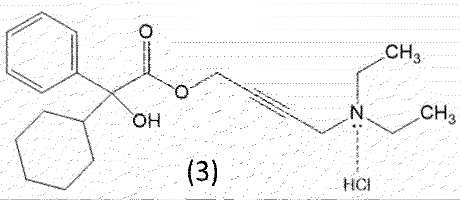

Die obige Strukturformel (3) zeigt die wasserlösliche Form von Oxybutynin, nämlich in Form von Oxybutyninhydrochlorid.

Oxybutynin gemäß Strukturformel (1) und Oxybutyninhydrochlorid gemäß Strukturformel (3) unterscheiden sich - gleichermaßen ohne sich auf diese Theorie beschränken oder berufen zu wollen - auch in ihrer Polarität, Löslichkeit und Affinität zu apolaren Oberflächen. In diesem Zusammenhang hat die Anmelderin gleichermaßen überraschend gefunden, dass insbesondere bei der Hitzesterilisation insbesondere Oxybutynin in Form der Strukturformel (1) verstärkt bzw. maßgeblich in das Spritzenmaterial auf Basis von Polypropylen migriert bzw. hierin eingelagert wird, und zwar insbesondere bedingt durch die geringere Polarität im Vergleich zu Substanz (3), wobei dieser Effekt im Allgemeinen auch gegenüber einem Abbau dominant sein kann, wie gleichermaßen seitens der Anmelderin gefunden.

In diesem Zusammenhang konnte die Anmelderin auch eine Absenkung des pH-Wertes der oxybutyninhaltigen Zusammensetzung bei Migration bzw. Einlagerung bzw. Verlust des Oxybutynins beobachten, welcher - ohne sich auf diese Theorie beschränken oder berufen zu wollen - dadurch zustandekommen kann, dass insbesondere Oxybutynin in Form der freien Aminbase aus der Zusammensetzung entfernt wird.

Weiterhin konnte die Anmelderin zeigen, dass bei geringeren Ausgangs-pH-Werten der zu sterilisierenden Zusammensetzung ein geringerer Verlust des Oxybutynins bzw. eine verringerte Erniedrigung des pH-Wertes im Rahmen der Hitzesterilisation auftritt.

Im Rahmen der vorliegenden Erfindung wird hinsichtlich der verfahrensgemäß durchgeführten Sterilisation der oxybutyninhaltigen Zusammensetzung zudem in ziel- und zweckgerichteter Weise auf eine Hitzesterilisation, vorzugsweise Dampfsterilisation, abgestellt, anhand derer erfindungsgemäß hervorragende Sterilisationsergebnisse bei gleichzeitig hoher Produktstabilität erhalten werden können. Das Verfahren der Hitzesterilisation, insbesondere Dampfsterilisation, zählt dabei zu den thermischen Sterilisationsverfahren, wobei durch den Einsatz von Wärmeenergie eine effiziente Sterilisation gewährleistet wird. Im Hinblick auf die erfindungsgemäß bevorzugte Dampfsterilisation kommt zudem dem damit einhergehenden Einsatz von (Wasser-)Dampf eine große Bedeutung zu, da hierdurch eine gute Energie- bzw. Wärmeübertragung auf die zu sterilisierenden Komponenten bzw. auf das zu sterilisierende Gut gewährleistet wird. Insbesondere wird bei der Kondensation des Dampfes an dem kühleren Sterilisationsgut die Wärmeenergie bzw. -menge auf die zu sterilisierende Kolbenspritze bzw. die zu sterilisierende oxybutyninhaltige Zusammensetzung übertragen, wodurch Mikroorganismen oder dergleichen abgetötet bzw. irreversibel inaktiviert werden. Im Rahmen der erfindungsgemäß eingesetzten Hitzesterilisation, insbesondere Dampfsterilisation, wird somit eine sozusagen vollständige bzw. gemeinsame und zeitgleiche Sterilisation der eingesetzten Kolbenspritze mit der darin befindlichen oxybutyninhaltigen Zusammensetzung gewährleistet, worin ein weiterer Vorteil beispielsweise auch gegenüber einer sterilen Abfüllung oder dergleichen zu sehen ist, da durch die erfindungsgemäß bevorzugt vorgesehene terminale Sterilisation die Sterilisationssicherheit weiterführend erhöht ist.

Im Allgemeinen wird unter dem Begriff der (Hitze-)Sterilisation bzw. (Dampf-) Sterilisation, wie er im Rahmen der vorliegenden Erfindung verwendet wird, insbesondere eine Abtötung bzw. eine irreversible Inaktivierung von Mikroorganismen, Keimen, Viren oder dergleichen, welche sich an bzw. in einem zu sterilisierenden Objekt befinden, einschließlich ihrer Ruhestadien bzw. Überdauerungsformen, wie beispielsweise Endosporen oder dergleichen, verstanden. Da die vollständige Beseitigung bzw. Inaktivierung sämtlicher Erreger an bzw. in einem Gegenstand bzw. Objekt rein statistisch nicht mit vollständiger Sicherheit gewährleistet werden kann, gilt ein Gegenstand bzw. ein Objekt eines Sterilisierguts im Allgemeinen dann als steril, wenn die Wahrscheinlichkeit einer Kontamination mit vermehrungsfähigen Erregern einen bestimmten Wert nicht überschreitet. Diesbezüglich kann insbesondere ein Wert von höchstens 1 : 10⁶ bzw. 10⁻⁶ angeführt werden. Dies bedeutet, dass unter 1 Million Einheiten eines Sterilisierguts höchstens eine Einheit nicht steril ist (beispielsweise wenn diese Einheit mit einer koloniebildenden Einheit (KBE) eines Mikroorganismus bzw. Erregers kontaminiert ist bzw. der Restgehalt an Erregern je Einheit eines Sterilisierguts höchstens 10⁻⁶ KBE beträgt, was dazu führt, dass die Wahrscheinlichkeit für eine nicht sterile Einheit bei 1 : 10⁶ liegt). Ein Gegenstand kann demnach insbesondere dann als steril betrachtet werden, wenn der theoretische Wert von nicht mehr als einem lebenden Mikroorganismus in 10⁶ (1 Million) sterilisierten Einheiten des Endprodukts vorhanden ist. Diesbezüglich wird im Allgemeinen auch von dem *Sterility Assurance Level* (*SAL*) gesprochen. Hierzu kann auch auf die nachfolgenden Ausführungen verwiesen werden.

Im Rahmen der vorliegenden Erfindung wird somit insbesondere derart vorgegangen, dass eine mit der oxybutyninhaltigen Zusammensetzung vorgefüllte Spritze auf Basis von Polypropylen bzw. mit einem Spritzenkörper, insbesondere Spritzenzylinder, aus einem Polypropylen, in welcher bzw. welchem die oxybutyninhaltige Zusammensetzung aufgenommen ist bzw. vorliegt, vorzugsweise terminal hitzesterilisiert, insbesondere dampfsterilisiert, wird. Dies führt zu einer hohen Sterilisationssicherheit und -effizienz.

Im Rahmen der vorliegenden Erfindung wird dabei, wie zuvor angeführt, auf die Verwendung von (Einweg-)Spritzen bzw. Kolbenspritzen auf Basis von Polypropylen abgestellt. Wie zuvor angeführt, ist es erfindungsgemäß vorgesehen, dass die Kolbenspritze, einen Spritzenkörper, insbesondere Spritzenzylinder, einerseits und einen Spritzenkolben mit einem Kolbenstopfen andererseits aufweist, wobei der Spritzenkörper, insbesondere Spritzenzylinder, aus einem Polypropylen (PP) gebildet ist und/oder hieraus besteht. Diesbezüglich können entsprechende Kolbenspritzen bzw. Spritzenkörper bzw. Spritzenzylinder auf Basis eines Polypropylens mit der Produktbezeichnung *ExxonMobil*^{®} *PP 1013 H1* verwendet werden.

Erfindungsgemäß ist die Formulierung, wonach der Spritzenkörper, insbesondere Spritzenzylinder, aus einem Polypropylen (PP) gebildet ist, insbesondere derart zu verstehen, dass der Spritzenkörper, insbesondere Spritzenzylinder, das Polypropylen in einer Menge von mindestens 90 Gew.-%, insbesondere mindestens 95 Gew.-%, vorzugsweise mindestens 99 Gew.-%, bevorzugt mindestens 99,5 Gew.-%, bezogen auf den Spritzenkörper, insbesondere Spritzenzylinder, aufweist und bevorzugt zumindest im Wesentlichen hieraus besteht bzw. vollständig hieraus besteht. Diesbezüglich kann es erfindungsgemäß grundsätzlich vorgesehen sein, dass der Spritzenkörper, insbesondere Spritzenzylinder, geringe Mengen an weiteren Inhaltsstoffen, wie Stabilisatoren, Verarbeitungshilfsmitteln, Farbstoffen, Kunststoffadditiven oder dergleichen, enthält. Erfindungsgemäß kann es insbesondere vorgesehen sein, dass der der Spritzenkörper, insbesondere Spritzenzylinder, das Polypropylen in einer Menge von zumindest im Wesentlichen 100 Gew.-%, bezogen auf den Spritzenkörper, insbesondere Spritzenzylinder, aufweist bzw. hieraus besteht. Besonders bevorzugt ist es also, wenn der Spritzenkörper, insbesondere Spritzenzylinder, zumindest im Wesentlichen, vorzugsweise vollständig, aus Polypropylen besteht.

Was das vorliegend beschriebene Verfahren anbelangt, so kann die im Verfahren, insbesondere in Verfahrensschritt b), auftretende Migration und/oder Einlagerung des Oxybutynins in den Spritzenkörper, insbesondere Spritzenzylinder, und/oder in den Kolbenstopfen, und/oder der im Verfahren, insbesondere in Verfahrensschritt b), auftretende Abbau, insbesondere der reaktive Abbau des Oxybutynins, vorzugsweise der hydrolytisch und/oder oxidativ hervorgerufene Abbau des Oxybutynins, bevorzugt der Abbau zu Phenylcyclohexylhydroxyessigsäure (Verunreinigung D), durch mindestens eine der nachfolgenden Maßnahmen und/oder Schritte (1), (2), (3) und/oder (4) gesteuert und/oder kontrolliert werden, insbesondere verhindert und/oder minimiert bzw. kompensiert werden:
(1) Einstellung und/oder Verwendung einer im Vergleich zur Spezifikations-Konzentration und/oder Spezifikations-Menge des Oxybutynins, bevorzugt in Form des Hydrochlorids, erhöhte Konzentration und/oder Menge (Übereinwaage) an Oxybutynin, bevorzugt in Form des Hydrochlorids, in der Ausgangszusammensetzung;
(2) Einstellung eines im Vergleich zu dem Spezifikations-pH-Wert abweichenden, insbesondere erhöhten pH-Werts in der Ausgangszusammensetzung,
(3) Einstellung und/oder Auswahl der Sterilisationsbedingungen, insbesondere ausgewählt aus der Gruppe von Sterilisationsart, Sterilisationsdauer, Sterilisationstemperatur, Sterilisationsdruck, Sterilisationsatmosphäre sowie deren Kombinationen, vorzugsweise Einstellung und/oder Auswahl des FO-Werts der Sterilisation (Letalitätswert);
(4) Ausrüstung und/oder Behandlung, insbesondere Beschichtung, des Spritzenkörpers, insbesondere Spritzenzylinders, vorzugsweise der Innenwandung des Spritzenkörpers, insbesondere Spritzenzylinders, und/oder des Kolbenstopfens mit mindestens einem migrations- und/oder abbauverringernden Mittel.

Vorliegend ist es dabei besonders bevorzugt, dass zumindest Maßnahme bzw. Schritt (1) durchgeführt wird. Vorliegend kann es somit insbesondere vorgesehen sein, dass zumindest Maßnahme bzw. Schritt (1) und Maßnahme bzw. Schritt (2), gegebenenfalls in Kombination mit Maßnahme bzw. Schritt (3) und/oder (4), durchgeführt wird.

Vorliegend ist es gleichermaßen von Vorteil, wenn zumindest Maßnahme bzw. Schritt (1) und Maßnahme bzw. Schritt (2) durchgeführt werden.

Somit ist es vorliegend weiterhin bevorzugt, dass zumindest Maßnahme bzw. Schritt (1) und Maßnahme bzw. Schritt (2) und Maßnahme bzw. Schritt (3), gegebenenfalls in Kombination mit Maßnahme bzw. Schritt (4), durchgeführt werden.

Was Maßnahme bzw. Schritt (1) anbelangt, so kann im Rahmen der vorliegenden Erfindung insbesondere wie folgt vorgegangen werden:
- So kann erfindungsgemäß, insbesondere für den Fall von Maßnahme und/oder Schritt (1), in der Ausgangszusammensetzung die Konzentration und/oder Menge an Oxybutynin, bevorzugt in Form des Hydrochlorids, um 1 % bis 20 %, insbesondere 2 % bis 15 %, vorzugsweise 2 % bis 15 %, bevorzugt 3 % bis 10 %, besonders bevorzugt 4 % bis 8 %, ganz besonders bevorzugt 4 % bis 6 %, oberhalb der Spezifikations-Konzentration und/oder Spezifikations-Menge an Oxybutynin, bevorzugt in Form des Hydrochlorids, der Endzusammensetzung gewählt bzw. eingestellt werden.
- Zudem kann, insbesondere für den Fall von Maßnahme und/oder Schritt (1), in der Ausgangszusammensetzung die Konzentration und/oder Menge an Oxybutynin, bevorzugt in Form des Hydrochlorids, auf einen Wert im Bereich von 101 % bis 120 %, insbesondere im Bereich von 102 % bis 115 %, vorzugsweise im Bereich von 102 % bis 115 %, bevorzugt im Bereich von 103 % bis 110 %, besonders bevorzugt im Bereich von 104 % bis 108 %, ganz besonders bevorzugt im Bereich von 104 % bis 106 %, der Spezifikations-Konzentration und/oder der Spezifikations-Menge an Oxybutynin, bevorzugt in Form des Hydrochlorids, der Endzusammensetzung eingestellt werden.
- Auch kann, insbesondere für den Fall von Maßnahme und/oder Schritt (1), in der Ausgangszusammensetzung die Konzentration und/oder Menge an Oxybutynin, bevorzugt in Form des Hydrochlorids, auf einen Wert im Bereich von 0,51 mg/ml bis 3,5 mg/ml, insbesondere im Bereich von 0,55 mg/ml bis 3 mg/ml, vorzugsweise im Bereich von 0,6 mg/ml bis 2,5 mg/ml, bevorzugt im Bereich von 0,8 mg/ml bis 2,2 mg/ml, besonders bevorzugt im Bereich von 0,9 mg/ml bis 1,8 mg/ml, noch mehr bevorzugt im Bereich von 1,05 mg/ml bis 1,1 mg/ml, ganz besonders bevorzugt von etwa 1,05 mg/ml oder aber von etwa 1,1 mg/ml, eingestellt werden.

Erfindungsgemäß kann somit insbesondere derart vorgegangen werden, dass in der Ausgangszusammensetzung Oxybutynin, bevorzugt in Form des Hydrochlorids, im Übermaß bzw. im Vergleich zur (End-)Zusammensetzung in einer höheren Menge eingesetzt wird (Übereinwaage), um hierdurch die bei der Hitzesterilisation, insbesondere Dampfsterilisation, auftretende Migration bzw. Einlagerung bzw. den Abbau auszugleichen bzw. zu kompensieren. Dabei ist auch von Beachtung, dass durch die erfindungsgemäßen Maßnahmen ein übermäßiger Abbau verhindert und somit das Entstehen unerwünschter Abbauprodukte trotz höherer Oxybutyninmenge minimiert wird.

Was Maßnahme bzw. Schritt (2) anbelangt, so kann im Rahmen der vorliegenden Erfindung insbesondere wie folgt vorgegangen werden:
- Erfindungsgemäß kann, insbesondere für den Fall von Maßnahme und/oder Schritt (2), der pH-Wert der Ausgangszusammensetzung um 0,02 pH-Werteinheiten bis 2,4 pH-Werteinheiten, insbesondere 0,05 pH-Werteinheiten bis 2,2 pH-Werteinheiten, vorzugsweise 0,1 pH-Werteinheiten bis 2 pH-Werteinheiten, bevorzugt 0,15 pH-Werteinheiten bis 1,8 pH-Werteinheiten, besonders bevorzugt 0,2 pH-Werteinheiten bis 1,6 pH-Werteinheiten, ganz besonders bevorzugt 0,25 pH-Werteinheiten bis 1,4 pH-Werteinheiten oberhalb des Spezifikations-pH-Werts der Endzusammensetzung gewählt und/oder eingestellt werden.
- Zudem kann, insbesondere für den Fall von Maßnahme und/oder Schritt (2), der pH-Wert der Ausgangszusammensetzung um 5 % bis 90 %, insbesondere 5 % bis 85 %, vorzugsweise 10 % bis 80 %, bevorzugt 10 % bis 75 %, besonders bevorzugt 15 % bis 70 %, ganz besonders bevorzugt 20 % bis 60 %, oberhalb des Spezifikations-pH-Werts der Endzusammensetzung gewählt und/oder eingestellt werden.
- Zudem kann, insbesondere für den Fall von Maßnahme und/oder Schritt (2), der pH-Wert der Ausgangszusammensetzung auf einen Wert im Bereich von 2,8 bis 7,5, insbesondere im Bereich von 3,1 bis 7, vorzugsweise im Bereich von 3,4 bis 6,5, bevorzugt im Bereich von 3,5 bis 6, besonders bevorzugt im Bereich von 3,6 bis 5,8, ganz besonders bevorzugt im Bereich von 3,9 bis 5, eingestellt werden.

Im Allgemeinen kann für den Fall von Maßnahme und/oder Schritt (2), der pH-Wert der Ausgangszusammensetzung unter Verwendung von und/oder mittels Salzsäure (HCl) eingestellt werden.

Im Rahmen der vorliegenden Erfindung kann somit im Hinblick auf Maßnahme bzw. Schritt (2) insbesondere derart vorgegangen werden, dass ein im Vergleich zu der Spezifikationsvorgabe der Endzusammensetzung höherer pH-Wert in der Ausgangszusammensetzung eingestellt bzw. vorgegeben wird. Hierdurch kann die im Zusammenhang mit der Migration bzw. Einlagerung des Oxybutynins bzw. dem Abbau des Oxybutynins im Zusammenhang stehende Verringerung des pH-Wertes kompensiert werden.

Was Maßnahme bzw. Schritt (3) anbelangt, so kann im Rahmen der vorliegenden Erfindung insbesondere wie folgt vorgegangen werden:
- So kann, insbesondere für den Fall von Maßnahme und/oder Schritt (3), die Hitzesterilisation, insbesondere Dampfsterilisation, vorzugsweise Wasserdampfsterilisation, bevorzugt terminale (Hitze-, Dampf-, Wasserdampf-) Sterilisation, mit einem FO-Wert im Bereich von 6 min bis 45 min, insbesondere im Bereich von 8 min bis 40 min, vorzugsweise im Bereich von 10 min bis 38 min, bevorzugt im Bereich von 11 min bis 35 min, besonders bevorzugt im Bereich von 15 min bis 32 min, ganz besonders bevorzugt im Bereich von 19 min bis 30 min, durchgeführt werden.
- Weiterhin können, insbesondere für den Fall von Maßnahme und/oder Schritt (3), die Sterilisationsbedingungen auf einen FO-Wert im Bereich von 6 min bis 45 min, insbesondere im Bereich von 8 min bis 40 min, vorzugsweise im Bereich von 10 min bis 38 min, bevorzugt im Bereich von 11 min bis 35 min, besonders bevorzugt im Bereich von 15 min bis 32 min, ganz besonders bevorzugt im Bereich von 19 min bis 30 min, eingestellt oder hieraus ausgewählt werden.

Diesbezüglich konnte die Anmelderin zeigen, dass bei Gewährleistung einer hohen Sterilität zum einen die Migration sowie zum anderen der Abbau weiterführend kontrolliert bzw. minimiert werden kann.

Der FO-Wert der Hitzesterilisation, insbesondere Dampfsterilisation, vorzugsweise Wasserdampfsterilisation, bevorzugt der terminalen (Hitze-, Dampf-, Wasserdampf-) Sterilisation, dient dabei insbesondere zur Bestimmung der thermischen Belastung einer Probe, wobei der FO-Wert die Äquivalenzbelastung der durchgeführten Sterilisation im Vergleich zu einer Sterilisation bei Referenztemperatur von etwa 121 °C darstellt und in Minuten angegeben wird. Im Hinblick auf den FO-Wert werden sozusagen sämtliche letale Effekte der durchgeführten Hitzesterilisation über den Verlauf der Sterilisation bzw. des zugrundeliegenden Sterilisationsprogramms aufsummiert bzw. integriert. Ein FO-Wert von einer Minute entspricht dabei einer thermischen Belastung, welche äquivalent zu einer Minute bei einer Sterilisationstemperatur etwa 121 °C ist. Die Definition und Einstellung bzw. Vorgabe des FO-Werts ist dabei dem Fachmann wohlbekannt, so dass es diesbezüglich keiner weiteren Ausführungen bedarf.

Was Maßnahme bzw. Schritt (4) anbelangt, so kann im Rahmen der vorliegenden Erfindung insbesondere wie folgt vorgegangen werden:
- So kann, insbesondere für den Fall von Maßnahme bzw. Schritt (4), die Ausrüstung und/oder Behandlung, insbesondere Beschichtung, des Spritzenkörpers, insbesondere Spritzenzylinders, vorzugsweise der Innenwandung des Spritzenkörpers, insbesondere Spritzenzylinders, und/oder des Kolbenstopfens mit einem siliciumhaltigen, insbesondere silikon- oder silikatbasierten Mittel, vorzugsweise Silikonöl oder Silikat, insbesondere mittels Silikonisierung oder Silikatisierung, durchgeführt werden.
- Insbesondere für den Fall von Maßnahme und/oder Schritt (4) kann das migrations- und/oder abbauverringernde Mittel folglich ein siliciumhaltiges, insbesondere silikon- oder silikatbasiertes Mittel, vorzugsweise Silikonöl oder Silikat, sein.

Beispielsweise kann eine Beschichtung mit einem Silikonöl auf Basis von Dimeticon erfolgen, beispielsweise unter Verwendung bzw. Einsatz von Silikonöl Ph. Eur. "Dimeticone" 1.000 mPas.

Die diesbezügliche Ausrüstung bzw. Behandlung erfolgt dabei insbesondere vor Befüllung des Spritzenkörpers mit der Ausgangszusammensetzung.

Durch die ziel- bzw. zweckgerichtete Ausrüstung des Spritzenkörpers, insbesondere Spritzenzylinders, kann insbesondere die Migration bzw. Einlagerung des Oxybutynins in den Spritzenkörper bzw. Spritzenzylinder weiterführend verringert werden, wie seitens der Anmelderin überraschend gefunden.

In entsprechender Weise kann im Rahmen der vorliegenden Erfindung grundsätzlich auch das nachfolgend noch beschriebene Verschlusselement dementsprechend behandelt und/oder beschichtet werden, vorzugsweise gleichermaßen mit einem siliciumhaltigen, insbesondere silikon- oder silikatbasierten Mittel, vorzugsweise Silikonöl oder Silikat, insbesondere als migrations- und/oder abbauverringerndes Mittel.

Vorliegend können somit für das Verfahren insbesondere mit einem siliciumhaltigen, insbesondere silikon- oder silikatbasierten Mittel, vorzugsweise Silikonöl oder Silikat, behandelte und/oder beschichtete Kolbenspritzen, insbesondere Spritzenkörper, und/oder Kolbenstopfen und/oder Verschlusselemente eingesetzt werden.

Vorliegend kann insbesondere derart vorgegangen werden, dass die im Verfahren, insbesondere in Verfahrensschritt b), auftretende Migration und/oder Einlagerung des Oxybutynins in den Spritzenkörper, insbesondere Spritzenzylinder, und/oder in den Kolbenstopfen und/oder der im Verfahren, insbesondere in Verfahrensschritt b), auftretende Abbau, insbesondere der reaktive Abbau des Oxybutynins, vorzugsweise der hydrolytisch und/oder oxidativ hervorgerufene Abbau des Oxybutynins, bevorzugt der Abbau zu Phenylcyclohexylhydroxyessigsäure (Verunreinigung D), durch mindestens eine der nachfolgenden Maßnahmen und/oder Schritte (1), (2), (3) und/oder (4) gesteuert und/oder kontrolliert wird, insbesondere verhindert und/oder minimiert und/oder kompensiert werden:
(1) Einstellung und/oder Verwendung einer im Vergleich zur Spezifikations-Konzentration und/oder Spezifikations-Menge des Oxybutynins, bevorzugt in Form des Hydrochlorids, erhöhte Konzentration und/oder Menge (Übereinwaage) an Oxybutynin, bevorzugt in Form des Hydrochlorids, in der Ausgangszusammensetzung,
   insbesondere wobei in der Ausgangszusammensetzung die Konzentration und/oder Menge an Oxybutynin, bevorzugt in Form des Hydrochlorids, um 1 % bis 20 %, insbesondere 2 % bis 15 %, vorzugsweise 2 % bis 15 %, bevorzugt 3 % bis 10 %, besonders bevorzugt 4 % bis 8 %, ganz besonders bevorzugt 4 % bis 6 %, oberhalb der Spezifikations-Konzentration und/oder Spezifikations-Menge an Oxybutynin, bevorzugt in Form des Hydrochlorids, gewählt und/oder eingestellt wird; und/oder
   insbesondere wobei in der Ausgangszusammensetzung die Konzentration und/oder Menge an Oxybutynin, bevorzugt in Form des Hydrochlorids, auf einen Wert im Bereich von 101 % bis 120 %, insbesondere im Bereich von 102 % bis 115 %, vorzugsweise im Bereich von 102 % bis 115 %, bevorzugt im Bereich von 103 % bis 110 %, besonders bevorzugt im Bereich von 104 % bis 108 %, ganz besonders bevorzugt im Bereich von 104 % bis 106 %, der Spezifikations-Konzentration und/oder der Spezifikations-Menge an Oxybutynin, bevorzugt in Form des Hydrochlorids, eingestellt wird; und/oder
   insbesondere wobei in der Ausgangszusammensetzung die Konzentration und/oder Menge an Oxybutynin, bevorzugt in Form des Hydrochlorids, auf einen Wert im Bereich von 0,51 mg/ml bis 3,5 mg/ml, insbesondere im Bereich von 0,55 mg/ml bis 3 mg/ml, vorzugsweise im Bereich von 0,6 mg/ml bis 2,5 mg/ml, bevorzugt im Bereich von 0,8 mg/ml bis 2,2 mg/ml, besonders bevorzugt im Bereich von 0,9 mg/ml bis 1,8 mg/ml, noch mehr bevorzugt im Bereich von 1,05 mg/ml bis 1,1 mg/ml, ganz besonders bevorzugt von etwa 1,05 mg/ml oder aber von etwa 1,1 mg/ml, eingestellt wird;
(2) Einstellung eines im Vergleich zu dem Spezifikations-pH-Wert abweichenden, insbesondere erhöhten pH-Werts der Ausgangszusammensetzung,
   insbesondere wobei der pH-Wert der Ausgangszusammensetzung um 0,02 pH-Werteinheiten bis 2,4 pH-Werteinheiten, insbesondere 0,05 pH-Werteinheiten bis 2,2 pH-Werteinheiten, vorzugsweise 0,1 pH-Werteinheiten bis 2 pH-Werteinheiten, bevorzugt 0,15 pH-Werteinheiten bis 1,8 pH-Werteinheiten, besonders bevorzugt 0,2 pH-Werteinheiten bis 1,6 pH-Werteinheiten, ganz besonders bevorzugt 0,25 pH-Werteinheiten bis 1,4 pH-Werteinheiten oberhalb des Spezifikations-pH-Werts der Endzusammensetzung gewählt und/oder eingestellt wird; und/oder
   insbesondere wobei der pH-Wert der Ausgangszusammensetzung um 5 % bis 90 %, insbesondere 5 % bis 85 %, vorzugsweise 10 % bis 80 %, bevorzugt 10 % bis 75 %, besonders bevorzugt 15 % bis 70 %, ganz besonders bevorzugt 20 % bis 60 %, oberhalb des Spezifikations-pH-Werts gewählt und/oder eingestellt wird; und/oder
   insbesondere wobei der pH-Wert der Ausgangszusammensetzung auf einen Wert im Bereich von 2,8 bis 7,5, insbesondere im Bereich von 3,1 bis 7, vorzugsweise im Bereich von 3,4 bis 6,5, bevorzugt im Bereich von 3,5 bis 6, besonders bevorzugt im Bereich von 3,6 bis 5,8, ganz besonders bevorzugt im Bereich von 3,9 bis 5, eingestellt wird;
   wobei der pH-Wert der Ausgangszusammensetzung unter Verwendung von und/oder mittels Salzsäure (HCl) eingestellt wird;
(3) Einstellung und/oder Auswahl der Sterilisationsbedingungen, insbesondere ausgewählt aus der Gruppe von Sterilisationsart, Sterilisationsdauer, Sterilisationstemperatur, Sterilisationsdruck, Sterilisationsatmosphäre sowie deren Kombinationen, vorzugsweise Einstellung und/oder Auswahl des FO-Werts der Sterilisation (Letalitätswert),
   insbesondere wobei die Hitzesterilisation, insbesondere Dampfsterilisation, vorzugsweise Wasserdampfsterilisation, bevorzugt terminale (Hitze-, Dampf-, Wasserdampf-)Sterilisation mit einem FO-Wert im Bereich von 6 min bis 45 min, insbesondere im Bereich von 8 min bis 40 min, vorzugsweise im Bereich von 10 min bis 38 min, bevorzugt im Bereich von 11 min bis 35 min, besonders bevorzugt im Bereich von 15 min bis 32 min, ganz besonders bevorzugt im Bereich von 19 min bis 30 min, durchgeführt wird;
(4) Ausrüstung und/oder Behandlung, insbesondere Beschichtung, des Spritzenkörpers, insbesondere Spritzenzylinders, vorzugsweise der Innenwandung des Spritzenkörpers, insbesondere Spritzenzylinders, und/oder des Kolbenstopfens mit mindestens einem migrations- und/oder abbauverringernden Mittel, bevorzugt einem siliciumhaltigen, insbesondere silikon- oder silikatbasierten Mittel, vorzugsweise Silikonöl oder Silikat, insbesondere mittels Silikonisierung oder Silikatisierung, und/oder wobei das migrations- und/oder abbauverringernde Mittel ein siliciumhaltiges, insbesondere silikon- oder silikatbasiertes Mittel, vorzugsweise Silikonöl oder Silikat, ist;

insbesondere wobei zumindest Maßnahme und/oder Schritt (1), gegebenenfalls in Kombination mit Maßnahme und/oder Schritt (2), (3) und/oder (4), durchgeführt wird; und/oder
insbesondere wobei zumindest Maßnahme und/oder Schritt (1) und Maßnahme und/oder Schritt (2), gegebenenfalls in Kombination mit Maßnahme und/oder Schritt (3) und/oder (4), durchgeführt werden; und/oder
insbesondere wobei zumindest Maßnahme und/oder Schritt (1) und Maßnahme und/oder Schritt (2) und Maßnahme und/oder Schritt (3), gegebenenfalls in Kombination mit Maßnahme und/oder Schritt (4), durchgeführt werden.

Im Rahmen der vorliegenden Erfindung kann es sich insbesondere derart verhalten, dass die im Verfahren, insbesondere in Verfahrensschritt b), auftretende Migration und/oder Einlagerung in den Spritzenkörper, insbesondere Spritzenzylinder, und/oder in den Kolbenstopfen durch Oxybutynin in Form der freien Base, insbesondere Oxybutynin in Form der freien Aminbase, erfolgt und/oder hierdurch hervorgerufen ist. Hierzu kann auch auf obige Ausführungen verwiesen werden.

In diesem Zusammenhang kann es sich insbesondere derart verhalten, dass infolge der Migration und/oder Einlagerung von Oxybutynin in Form der freien Base, insbesondere Oxybutynin in Form der freien Aminbase, in den Spritzenkörper, insbesondere Spritzenzylinder, der pH-Wert gesenkt bzw. verringert ist.

Wie gleichermaßen zuvor angeführt und ohne sich auf die diesbezügliche Theorie berufen oder beschränken zu wollen, weist Oxybutynin in Form der freien Base (vgl. obige Strukturformel (1)) eine geringere Polarität als Oxybutynin in Form des Hydrochlorids auf (vgl. obige Strukturformel (3)), wodurch eine bevorzugte Migration in das apolare Spritzenmaterial resultieren kann. Infolge des Entfernens bzw. der Verringerung von Oxybutynin in Form der freien Base in der Zusammensetzung resultiert eine entsprechende Verringerung des pH-Wertes der zugrundeliegenden Zusammensetzung. Wie zuvor angeführt, können durch die zuvor angeführten Maßnahmen diese Effekte in entsprechender Weise kompensiert bzw. ausgeglichen werden, um hierdurch die gleichermaßen zuvor angeführten Spezifikationsvorgaben in Bezug auf die nach dem Verfahren erhältliche Endzusammensetzung zu erreichen bzw. zu gewährleisten.

Erfindungsgemäß kann es sich zudem derart verhalten, dass die im Verfahren, insbesondere in Verfahrensschritt b), auftretende Migration und/oder Einlagerung des Oxybutynins in den Spritzenkörper, insbesondere Spritzenzylinder, und/oder in den Kolbenstopfen und/oder der im Verfahren, insbesondere in Verfahrensschritt b), auftretende Abbau, insbesondere der reaktive Abbau des Oxybutynins, vorzugsweise der hydrolytisch und/oder oxidativ hervorgerufene Abbau des Oxybutynins, bevorzugt der Abbau zu Phenylcyclohexylhydroxyessigsäure (Verunreinigung D), durch Einstellung des Verhältnisses von (i) Oxybutynin in Form der freien Base, insbesondere Oxybutynin in Form der freien Aminbase, einerseits und (ii) Oxybutynin in Form der komplexierten und/oder koordinierten und/oder versalzenen und/oder protonierten Base, insbesondere Oxybutynin in Form der komplexierten und/oder koordinierten und/oder versalzenen und/oder protonierten Aminbase, vorzugsweise Oxybutynin in Form des Hydrochlorids, gesteuert und/oder kontrolliert wird, insbesondere verhindert und/oder minimiert und/oder kompensiert wird.

In diesem Zusammenhang kann es sich insbesondere derart verhalten, dass das Verhältnis in Richtung von (ii) Oxybutynin in Form der komplexierten und/oder koordinierten und/oder versalzenen und/oder protonierten Base, insbesondere Oxybutynin in Form der komplexierten und/oder koordinierten und/oder versalzenen und/oder protonierten Aminbase, vorzugsweise Oxybutynin in Form des Hydrochlorids, verschoben wird, vorzugsweise durch Einstellung, insbesondere Absenkung und/oder Verringerung, des pH-Werts und/oder durch Einstellung eines insbesondere niedrigen pH-Werts, insbesondere auf einen pH-Wert im Bereich von 2,8 bis 5, vorzugsweise im Bereich von 3,1 bis 4,5, bevorzugt im Bereich von 3,2 bis 4,2, in der Ausgangzusammensetzung und/oder durch Zugabe einer Säure in die Ausgangszusammensetzung.

Erfindungsgemäß kann es sich somit insbesondere derart verhalten, dass durch entsprechend niedrige pH-Werte der Ausgangszusammensetzung die Migration bzw. die Einlagerung von (i) Oxybutynin in Form der freien Base in den Spritzenkörper bzw. Spritzenzylinder insofern verringert wird, als in der Ausgangszusammensetzung eine Verschiebung in Richtung von (ii) Oxybutynin in Form der komplexierten bzw. koordinierten bzw. versalzenen bzw. protonierten Base vorliegt. Hiermit geht insbesondere auch ein geringerer Rückgang des pH-Werts bzw. geringerer pH-Verlust bei der Sterilisation, insbesondere Dampfsterilisation, einher.

Im Rahmen der vorliegenden Erfindung kann es sich weiterhin insbesondere derart verhalten, dass die im Verfahren, insbesondere in Verfahrensschritt b), auftretende Migration und/oder Einlagerung des Oxybutynins in den Spritzenkörper, insbesondere Spritzenzylinder, und/oder in den Kolbenstopfen, auf einen Wert von höchstens 20 Gew.-%, insbesondere höchstens 15 Gew.-%, vorzugsweise höchstens 10 Gew.- %, bevorzugt höchstens 8 Gew.-%, besonders bevorzugt höchstens 5 Gew.-%, der in der Ausgangslösung bzw. Ausgangszusammensetzung vorliegenden Konzentration und/oder Menge an Oxybutynin, bevorzugt in Form des Hydrochlorids, beschränkt und/oder eingestellt wird.

Erfindungsgemäß kann es sich zudem insbesondere derart verhalten, dass die Endzusammensetzung bzw. die nach dem erfindungsgemäßen Verfahren, insbesondere die nach Verfahrensschritt (b), erhaltene Endzusammensetzung einen niedrigeren pH-Wert als die Ausgangszusammensetzung aufweist. Dabei kann - gleichermaßen ohne sich auf diese Theorie beschränken oder berufen zu wollen - die pH-Wert-Absenkung durch die Einlagerung bzw. Migration von Oxybutynin insbesondere in Form der freien Base in den Spritzenkörper bzw. Spritzenzylinder, hervorgerufen werden bzw. hiermit im Zusammenhang stehen. Insbesondere verhält es sich im Rahmen der vorliegenden Erfindung derart, dass die Absenkung bzw. Verringerung des pH-Werts (pH-Verlust) im Verfahren, insbesondere in Verfahrensschritt b), auftritt.

Im Rahmen des vorliegend beschriebenen Verfahrens kann es insbesondere der Fall sein, dass die Absenkung bzw. Verringerung des pH-Werts (pH-Verlust) der Endzusammensetzung im Vergleich zu der Ausgangszusammensetzung auf höchstens 2 pH-Werteinheiten, insbesondere höchstens 1,6 pH-Werteinheiten, vorzugsweise höchstens 1,2 pH-Werteinheiten, bevorzugt höchstens 0,8 pH-Werteinheiten, besonders bevorzugt höchstens 0,6 pH-Werteinheiten, ganz besonders bevorzugt höchstens 0,4 pH-Werteinheiten, eingestellt und/oder beschränkt wird.

Gleichermaßen kann es erfindungsgemäß vorgesehen sein, dass die Absenkung bzw. Verringerung des pH-Werts (pH-Verlust) der Endzusammensetzung im Vergleich zu der Ausgangszusammensetzung auf höchstens 30 %, insbesondere höchstens 25 %, vorzugsweise höchstens 20 %, bevorzugt höchstens 15 %, besonders bevorzugt höchstens 10 %, ganz besonders bevorzugt höchstens 5 %, des pH-Werts der Ausgangszusammensetzung beschränkt bzw. eingestellt wird.

Was die nach Abschluss des Verfahrens erhaltene oxybutyninhaltige Endzusammensetzung anbelangt, so kann diese weitere Spezifikationsvorgaben aufweisen bzw. erfüllen:
- Insbesondere kann die Ausgangszusammensetzung als wässrige Zusammensetzung ausgebildet sein.
- So kann die nach Abschluss des Verfahrens, insbesondere nach Verfahrensschritt b), erhaltene oxybutyninhaltige Endzusammensetzung als weitere Spezifikationsvorgabe (iv) mindestens einen Elektrolyten, insbesondere Alkalimetallsalz, vorzugsweise Alkalimetallchlorid, bevorzugt Natriumchlorid (NaCl), aufweisen, insbesondere in einer vorgegebenen Spezifikations-Menge im Bereich von 0,1 Gew.-% bis 2 Gew.-%, insbesondere im Bereich von 0,5 Gew.-% bis 1,5 Gew.-%, vorzugsweise im Bereich von 0,7 Gew.-% bis 1,2 Gew.-%, bevorzugt von etwa 0,9 Gew.-%, bezogen auf die Endzusammensetzung.
- Insbesondere kann die nach Abschluss des Verfahrens, insbesondere nach Verfahrensschritt b), erhaltene oxybutyninhaltige Endzusammensetzung als weitere Spezifikationsvorgabe (iv) Natriumchlorid, berechnet als Natrium, in einer vorgegebenen Spezifikations-Konzentration von (3,5 ± 0,5) mg/ml, insbesondere (3,5 ± 0,3) mg/ml, vorzugsweise (3,5 ± 0,1) mg/ml, bevorzugt etwa 3,5 mg/ml, enthalten.
- Zudem kann die nach Abschluss des Verfahrens, insbesondere nach Verfahrensschritt b), erhaltene oxybutyninhaltige Endzusammensetzung als weitere Spezifikationsvorgabe (v) eine vorgegebene Spezifikations-Menge an Gesamt-Abbauprodukten von höchstens 1 Gew.-%, insbesondere höchstens 0,5 Gew.-%, bezogen auf die Gesamtmenge an eingesetztem Oxybutynin, bevorzugt Oxybutyninhydrochlorid, aufweisen. Die Gesamt-Abbauprodukte umfassen dabei insbesondere auch die vorgenannte Verunreinigung D.

Im Hinblick auf das vorliegend beschriebene Verfahren kann die Ausgangszusammensetzung insbesondere auch im Hinblick auf die angeführten Spezifikationsvorgaben der Endzusammensetzung wie folgt ausgebildet sein:
- So kann die Ausgangszusammensetzung Oxybutynin, bevorzugt in Form des Hydrochlorids, enthalten, vorzugsweise in einer Konzentration bzw. Menge im Bereich von 0,51 mg/ml bis 3,5 mg/ml, insbesondere im Bereich von 0,55 mg/ml bis 3 mg/ml, vorzugsweise im Bereich von 0,6 mg/ml bis 2,5 mg/ml, bevorzugt im Bereich von 0,8 mg/ml bis 2,2 mg/ml, besonders bevorzugt im Bereich von 0,9 mg/ml bis 1,8 mg/ml, noch mehr bevorzugt im Bereich von 1,05 mg/ml bis 1,1 mg/ml, ganz besonders bevorzugt von etwa 1,05 mg/ml oder aber von etwa 1,1 mg/ml.
- Zudem kann die Ausgangszusammensetzung einen pH-Wert im Bereich von 2,8 bis 7,5, insbesondere im Bereich von 3,1 bis 7, vorzugsweise im Bereich von 3,4 bis 6,5, bevorzugt im Bereich von 3,5 bis 6, besonders bevorzugt im Bereich von 3,6 bis 5,8, ganz besonders bevorzugt im Bereich von 3,9 bis 5, aufweisen. In diesem Zusammenhang kann der pH-Wert unter Verwendung von bzw. mittels Salzsäure (HCl) eingestellt werden.
- Zudem kann die Ausgangszusammensetzung gegebenenfalls mindestens einen Elektrolyten, insbesondere Alkalimetallsalz, vorzugsweise Alkalimetallchlorid, bevorzugt Natriumchlorid (NaCl), enthalten, insbesondere in einer Menge im Bereich von 0,1 Gew.-% bis 2 Gew.-%, insbesondere im Bereich von 0,5 Gew.-% bis 1,5 Gew.-%, vorzugsweise im Bereich von 0,7 Gew.-% bis 1,2 Gew.-%, bevorzugt von etwa 0,9 Gew.-%, bezogen auf die Ausgangszusammensetzung, und/oder wobei die Ausgangszusammensetzung gegebenenfalls Natriumchlorid, berechnet als Natrium, in einer vorgegebenen Spezifikations-Konzentration von (3,5 ± 0,5) mg/ml, insbesondere (3,5 ± 0,3) mg/ml, vorzugsweise (3,5 ± 0,1) mg/ml, aufweisen kann.

Zudem kann die Ausgangszusammensetzung wie folgt ausgebildet werden:
So kann die Zusammensetzung als wässrige Zusammensetzung ausgebildet werden;
wobei die Ausgangszusammensetzung Oxybutynin, bevorzugt in Form des Hydrochlorids, enthält, vorzugsweise in einer Menge im Bereich von 0,02 Gew-% bis 0,25 Gew-%, insbesondere im Bereich von 0,05 Gew-% bis 0,2 Gew-%, vorzugsweise im Bereich von 0,08 Gew-% bis 0,15 Gew-%, bevorzugt von etwa 0,15 Gew-%, bezogen auf die Ausgangszusammensetzung;
wobei die Ausgangszusammensetzung gegebenenfalls mindestens einen Elektrolyten, insbesondere Alkalimetallsalz, vorzugsweise Alkalimetallchlorid, bevorzugt Natriumchlorid (NaCl), enthält, insbesondere in einer Menge im Bereich von 0,1 Gew.- % bis 2 Gew.-%, insbesondere im Bereich von 0,5 Gew.-% bis 1,5 Gew.-%, vorzugsweise im Bereich von 0,7 Gew.-% bis 1,2 Gew.-%, bevorzugt von etwa 0,9 Gew.-%, bezogen auf die Ausgangszusammensetzung; und
wobei die Ausgangszusammensetzung gegebenenfalls Salzsäure, insbesondere zur Einstellung des pH-Werts, aufweist, insbesondere eingesetzt in Form von 10 %-iger Salzsäure und in einer Menge im Bereich von 0,001 Gew.-% bis 0,01 Gew.-%, insbesondere 0,003 Gew.-% bis 0,008 Gew.-%, bezogen auf die Ausgangszusammensetzung.

Darüber hinaus kann die im Rahmen des vorliegend beschriebenen Verfahrens eingesetzte Kolbenspritze wie folgt ausgebildet sein:
- So kann die Kolbenspritze, bevorzugt der Spritzenkörper, insbesondere Spritzenzylinder, ein Volumen, insbesondere ein Aufnahmevolumen für die oxybutyninhaltige Zusammensetzung, im Bereich von 1 ml bis 100 ml, insbesondere im Bereich von 2 ml bis 50 ml, vorzugsweise im Bereich von 3 ml bis 25 ml, bevorzugt im Bereich von 4 ml bis 25 ml, besonders bevorzugt im Bereich von 6 ml bis 20 ml, weiter bevorzugt im Bereich von 8 ml bis 15 ml, nochmals weiter bevorzugt im Bereich von 9 ml bis 12 ml, ganz besonders bevorzugt von etwa 10 ml, aufweisen.
- Insbesondere kann die Kolbenspritze, bevorzugt der Spritzenkörper, insbesondere Spritzenzylinder, die oxybutyninhaltige Zusammensetzung in einer Menge im Bereich von 1 ml bis 100 ml, insbesondere im Bereich von 2 ml bis 50 ml, vorzugsweise im Bereich von 3 ml bis 25 ml, bevorzugt im Bereich von 4 ml bis 25 ml, besonders bevorzugt im Bereich von 6 ml bis 20 ml, weiter bevorzugt im Bereich von 8 ml bis 15 ml, nochmals weiter bevorzugt im Bereich von 9 ml bis 12 ml, ganz besonders bevorzugt von etwa 10 ml, aufweisen.

Wie zuvor angeführt, dient der Spritzenkörper, insbesondere Spritzenzylinder, insbesondere der Aufnahme der zugrundeliegenden oxybutyninhaltigen Zusammensetzung.

Was darüber hinaus den Spritzenkolben der Kolbenspritze anbelangt, so kann dieser gleichermaßen aus Polypropylen (PP) gebildet sein bzw. hieraus bestehen. Diesbezüglich kann gleichermaßen ein Polypropylen auf Basis bzw. in Form von *ExxonMobil*^{®} *PP 1013 H1* eingesetzt werden.

Was weiterhin den Kolbenstopfen der Kolbenspritze anbelangt, so kann dieser im Allgemeinen aus einem elastomeren Material, insbesondere aus einem Kautschukmaterial und/oder Gummimaterial, vorzugsweise Kautschukmaterial, gebildet sein bzw. hieraus bestehen.

Erfindungsgemäß ist es jedoch bevorzugt, wenn der Kolbenstopfen und/oder das nachfolgend noch beschriebene Verschlusselement aus einem Halogenbutylkautschuk, insbesondere aus einem Chlorbutylkautschuk oder aus einem Brombutylkautschuk, vorzugsweise aus einem Brombutylkautschuk, gebildet ist bzw. hieraus besteht. In diesem Zusammenhang hat die Anmelderin gefunden, dass diesbezüglich insbesondere im Rahmen der Hitzesterilisation eine höhere Kompatibilität zu Oxybutynin bzw. Oxybutyninhydrochlorid vorliegt, so dass der Verlust an Oxybutynin bzw. Oxybutyninhydrochlorid in der Zusammensetzung insbesondere infolge einer geringeren Migration in die Kolbenstopfen bzw. in das Verschlusselement weiterführend verringert werden kann. Zudem weist Halogenbutylkautschuk, insbesondere Brombutylkautschuk, wie er im Rahmen der vorliegenden Erfindung in bevorzugter Weise für den Kolbenstopfen eingesetzt wird, weitere Vorteile auf, und zwar insbesondere was dessen hohe Hitzestabilität, verbesserte Alterungseigenschaften und geringe Gaspermeabilität anbelangt. **In** Bezug auf den Kolbenstopfen können markt- bzw. handelsübliche Produkte eingesetzt werden (wie z. B. Dätwyler V9340FM257/2 brombutyl-compound).

Erfindungsgemäß ist die Formulierung, wonach der Kolbenstopfen bzw. das Verschlusselement aus einem Halogenbutylkautschuk, insbesondere aus einem Chlorbutylkautschuk oder aus einem Brombutylkautschuk, vorzugsweise aus einem Brombutylkautschuk, gebildet ist, insbesondere derart zu verstehen, dass der Kolbenstopfen bzw. das Verschlusselement den Halogenbutylkautschuk, insbesondere den Chlorbutylkautschuk oder den Brombutylkautschuk, vorzugsweise den Brombutylkautschuk, in einer Menge von mindestens 90 Gew.-%, insbesondere mindestens 95 Gew.-%, vorzugsweise mindestens 99 Gew.-%, bevorzugt mindestens 99,5 Gew.-%, bezogen auf den Kolbenstopfen bzw. das Verschlusselement, aufweist und bevorzugt zumindest im Wesentlichen hieraus besteht bzw. vollständig hieraus besteht. Diesbezüglich kann es erfindungsgemäß grundsätzlich vorgesehen sein, dass der Kolbenstopfen bzw. das Verschlusselement geringe Mengen an weiteren Inhaltsstoffen, wie Stabilisatoren, Verarbeitungshilfsmitteln, Farbstoffen, Kunststoffadditiven oder dergleichen, enthält. Erfindungsgemäß kann es insbesondere vorgesehen sein, dass der Kolbenstopfen bzw. das Verschlusselement den Halogenbutylkautschuk, insbesondere den Chlorbutylkautschuk oder den Brombutylkautschuk, vorzugsweise den Brombutylkautschuk, in einer Menge von zumindest im Wesentlichen 100 Gew.-%, bezogen auf den der Kolbenstopfen bzw. das Verschlusselement, aufweist bzw. hieraus besteht. Besonders bevorzugt ist es also, wenn der Kolbenstopfen bzw. das Verschlusselement zumindest im Wesentlichen, vorzugsweise vollständig, aus Halogenbutylkautschuk, insbesondere Chlorbutylkautschuk oder Brombutylkautschuk, vorzugsweise den Brombutylkautschuk, besteht.

Gemäß einer alternativen Ausführungsform kann es erfindungsgemäß grundsätzlich auch vorgesehen sein, dass der Spritzenkolben mit dem Kolbenstopfen einstückig ausgebildet ist. In diesem Zusammenhang kann der Spritzenkolben mit dem Kolbenstopfen aus Polypropylen gebildet sein bzw. hieraus bestehen.

Im Allgemeinen weist die Kolbenspritze, insbesondere der Spritzenkörper, vorzugsweise der Spritzenzylinder, eine Aufnahme- und/oder Befüllöffnung auf, insbesondere zum Befüllen des Spritzenkörpers, insbesondere des Spritzenzylinders, mit der oxybutyninhaltigen Zusammensetzung und/oder insbesondere zur vorzugsweise abdichtenden Aufnahme des Spritzenkolbens mit dem Kolbenstopfen und/oder insbesondere wobei die Aufnahme- und/oder Befüllöffnung mit dem Spritzenkolben, insbesondere mit dem Kolbenstopfen, verschließbar ist und/oder verschließbar ausgebildet ist bzw. verschlossen ist.

Zudem kann es erfindungsgemäß vorgesehen sein, dass der Spritzenkörper einen Adapter, insbesondere zum Anschluss einer Instillationsvorrichtung und/oder zum Austragen und/oder Applizieren der oxybutyninhaltigen Zusammensetzung, aufweist (Anschluss- und/oder Austragsadapter).

In diesem Zusammenhang kann der Spritzenkörper einstückig (einteilig) ausgebildet sein, d. h. insbesondere eine mit dem Adapter einstückig bzw. zusammenhängende Einheit ausbilden. Insbesondere kann der Adapter mit dem Spritzenkörper, insbesondere Spritzenzylinder, eine verbundene, insbesondere fest und/oder unlösbar verbundene, vorzugsweise stoffschlüssig verbundene, Einheit ausbilden. In diesem Zusammenhang kann es sich im Rahmen der vorliegenden Erfindung gleichermaßen derart verhalten, dass der Adapter mit dem Spritzenkörper, insbesondere Spritzenzylinder, verbunden, insbesondere fest und/oder unlösbar verbunden, vorzugsweise stoffschlüssig verbunden, ist. In diesem Zusammenhang kann es sich erfindungsgemäß beispielsweise derart verhalten, dass der Adapter mit dem Spritzenkörper mittels Gießen, insbesondere Spritzgießen, Gießformen, Extrudieren oder dergleichen, verbunden, insbesondere fest bzw. unlösbar verbunden, vorzugsweise stoffschlüssig verbunden, ist.

Im Rahmen der vorliegenden Erfindung ist der Begriff "einstückig" bzw. "einteilig" bzw. "verbunden, insbesondere fest und/oder unlösbar verbunden" im Hinblick auf die bevorzugte Ausgestaltung des Spritzenkörpers bzw. Spritzenzylinders einerseits mit dem Adapter andererseits insbesondere derart zu verstehen, dass der Spritzenkörper als eine untrennbare bzw. fest verbundene Einheit auf Basis des Spritzenzylinders und Adapters als den Spritzenkolben und den Adapter aufweisendes bzw. integriertes Teil vorliegt. Mit anderen Worten verhält es sich im Rahmen der vorliegenden Erfindung somit insbesondere derart, dass der Adapter einerseits und der Spritzenzylinder andererseits zur Ausbildung des Spritzenkörpers eine zusammengehörende und fest verbundene bzw. dauerhafte Einheit ausbilden können. Insbesondere verhält es sich erfindungsgemäß derart, dass der Adapter einerseits und der Spritzenzylinder andererseits zur Ausbildung des Spritzenkörpers fest zusammenhängend bzw. nicht voneinander lösbar bzw. nicht voneinander trennbar verbunden sein kann. Insbesondere sind die vorgenannten Begriffe derart zu verstehen, dass Spritzenzylinder einerseits und Adapter andererseits nicht bzw. nicht zerstörungsfrei bzw. nur unter Zerstörung voneinander trennbar bzw. lösbar sind. Insbesondere können der Spritzenzylinder einerseits und der Adapter andererseits als Spritzenkörper eine konstruktive bzw. körperliche Einheit ohne diesbezügliche zerstörungsfreie Möglichkeit des Lösens des Adapters bzw. des Trennens dieser Komponenten bilden.

Diesbezüglich kann es sich im Rahmen der vorliegenden Erfindung somit insbesondere derart verhalten, dass der Spritzenzylinder und der Adapter aus identischen Materialien gebildet sind bzw. dass der Spritzenzylinder und der Adapter aus demselben Material, nämlich Polypropylen, bestehen und insbesondere einen einheitlichen, untrennbar zusammenhängenden Körper ausbilden.

Wie nachfolgend noch angeführt, kann es im Rahmen der vorliegenden Erfindung diesbezüglich insbesondere derart vorgesehen sein, dass der einstückig mit dem Spritzenzylinder ausgebildete Adapter stufenkegelförmig ausgebildet ist. Hierzu kann auch auf nachfolgende Ausführungen verwiesen werden.

Darüber hinaus kann der Adapter der im Rahmen des vorliegend beschriebenen Verfahrens eingesetzten Kolbenspritze wie folgt ausgebildet sein:
Der Adapter kann eine Auslassöffnung, insbesondere am freien Ende des Adapters und/oder insbesondere zum Austragen und/oder Applizieren der oxybutyninhaltigen Zusammensetzung, aufweisen. In diesem Zusammenhang kann der Adapter eine Einlassöffnung, insbesondere im Übergangsbereich zwischen Adapter und Spritzenzylinder und/oder insbesondere zur Aufnahme der oxybutyninhaltigen Zusammensetzung aus dem Spritzenzylinder, aufweisen. Zudem kann der Adapter hohl ausgebildet sein. Darüber hinaus kann der Adapter einen zwischen der Auslassöffnung des Adapters und der Einlassöffnung des Adapters befindlichen Hohlraum, insbesondere Durchlasskanal, insbesondere für den Auslass der oxybutyninhaltigen Zusammensetzung, aufweisen, insbesondere wobei sich der Hohlraum, insbesondere Durchlasskanal, von der Einlassöffnung des Adapters zu der Auslassöffnung des Adapters erstreckt und/oder die Einlassöffnung mit der Auslassöffnung verbinden.

Der Adapter kann weiterhin in Auslassrichtung und/oder in Richtung der Auslassöffnung des Adapters verjüngt bzw. zulaufend ausgebildet sein. Zudem kann der Adapter in Auslassrichtung und/oder in Richtung der Auslassöffnung des Adapters einen abnehmenden und/oder sich verringernden Umfang, insbesondere Außenumfang, und/oder einen abnehmenden und/oder sich verringernden Durchmesser, insbesondere Außendurchmesser, aufweisen.

Der Adapter kann im Rahmen der vorliegenden Erfindung insbesondere wie folgt ausgebildet sein:
- Erfindungsgemäß kann der Adapter düsenförmig bzw. als Düse, insbesondere als konus- oder zylinderförmige Düse, vorzugsweise ohne Gewinde, bevorzugt als Luer-Slip, oder aber mit Gewinde, insbesondere Schraubgewinde, bevorzugt als Luer-Lock, ausgebildet sein. Diesbezüglich kann auch auf die entsprechenden Normen gemäß der DIN EN 80369-7 sowie auf die DIN EN 20594-1 verwiesen werden.
- Weiterhin kann der Adapter konisch oder stufenkegelförmig, vorzugsweise stufenkegelförmig, ausgebildet sein. In diesem Zusammenhang kann der Adapter derart gebildet sein, dass der Umfang, insbesondere Außenumfang, bzw. der Durchmesser, insbesondere Außendurchmesser, des Adapters in Aulassrichtung bzw. in Richtung der Auslassöffnung des Adapters abnimmt.
- Insbesondere kann der Adapter als Stufenkegeladapter ausgebildet sein. In diesem Zusammenhang kann der Adapter eine Anzahl an Stufen im Bereich von 2 Stufen bis 40 Stufen, insbesondere im Bereich von 3 Stufen bis 30 Stufen, vorzugsweise im Bereich von 5 Stufen bis 25 Stufen, bevorzugt im Bereich von 8 Stufen bis 20 Stufen, besonders bevorzugt im Bereich von 10 Stufen bis 15 Stufen, aufweisen. Weiterhin kann in diesem Zusammenhang der Umfang, insbesondere Außenumfang, und/oder der Durchmesser, insbesondere Außendurchmesser, der Stufen des Adapters in Auslassrichtung und/oder in Richtung der Auslassöffnung des Adapters abnehmen.
- Zudem kann in diesem Zusammenhang das Verhältnis des Durchmessers, insbesondere Außendurchmessers, des Adapters an der Einlassöffnung des Adapters zu dem Durchmesser, insbesondere Außendurchmesser, des Adapters an der Auslassöffnung des Adapters in einem Bereich von 1,1 bis 15, insbesondere in einem Bereich von 1,25 bis 10, vorzugsweise in einem Bereich von 1,5 bis 5, bevorzugt in einem Bereich von 2 bis 4, besonders bevorzugt in einem Bereich von 2,25 bis 3, liegen.

Erfindungsgemäß wird unter dem Begriff "stufenkegelförmig" insbesondere eine Ausbildung des Adapters derart verstanden, dass sich der Umfang, insbesondere Außenumfang, bzw. der Durchmesser, insbesondere Außendurchmesser, des Adapters in Austragsrichtung der Zusammensetzung ausgehend von der Einlassöffnung in Richtung der Auslassöffnung durch Ausbildung einzelner bzw. aufeinander abfolgender Stufen bzw. stufenförmig verringert.

Erfindungsgemäß kann es somit vorgesehen sein, dass der Adapter als Stufenkegeladapter ausgebildet ist. Wie zuvor angeführt, kann es sich hierbei insbesondere um einen einstückig mit dem Spritzenkörper ausgebildeten Stufenkegeladapter handeln.

Zudem kann der Umfang, insbesondere Außenumfang, und/oder der Durchmesser, insbesondere Außendurchmesser, der Stufen des Adapters in Auslassrichtung und/oder in Richtung der Auslassöffnung des Adapters abnehmen.

Im Rahmen der vorliegenden Erfindung kann somit eine im Hinblick auf den Adapter vielseitig ausgebildete Kolbenspritze eingesetzt werden, wobei die konkrete Ausgestaltung insbesondere auch vor dem Hintergrund des zugrundeliegenden Anwendungs- bzw. Einsatzspektrums sozusagen maßgeschneidert werden kann, wobei im Rahmen der vorliegenden Erfindung auch die Möglichkeit der Verbindung bzw. des Anschlusses einer Vielzahl verschiedener Applikations- bzw. Instillationsvorrichtungen, insbesondere in Form von Installationsschläuchen bzw. (Harnblasen-)Kathetern, mit der erfindungsgemäß eingesetzten Kolbenspritze verbunden werden können. Die erfindungsgemäß gegebenenfalls vorgesehene einstückige Ausbildung von Spritzenkörper bzw. Spritzenzylinder und Adapter geht dabei mit dem Vorteil einer verbesserten Handhabbarkeit der Kolbenspritze bei gleichzeitig verringerter Gefahr einer Kontamination insbesondere im Bereich des Adapters bzw. im Hinblick auf die in der Kolbenspritze befindliche oxybutyninhaltige Zusammensetzung einher, zumal der Schritt des der Anwendung vorangehenden Anbringens bzw. Befestigens eines separaten Adapters an den Spritzenzylinder entfällt und somit die Applikations- bzw. Instillationsvorrichtung sozusagen direkt mit der Kolbenspritze verbunden werden kann. Hierdurch wird auch das Risiko einer Fehlbedienung reduziert. Durch die erfindungsgemäß gleichermaßen bevorzugte stufenförmige Ausbildung des Adapters ist bei gleichzeitig verbesserter Handhabbarkeit eine gute Befestigung bzw. Fixierung der Applikations- bzw. Instillationsvorrichtung gewährleistet, welche gegebenenfalls über ein entsprechendes Gegenstück über den Adapter geführt bzw. der Adapter in die insbesondere schlauchförmig ausgebildete Applikations- bzw. Instillationsvorrichtung geführt wird.

Weiterhin kann die Kolbenspritze ein insbesondere abnehmbares und/oder entfernbares und/oder lösbares Verschlusselement, insbesondere eine Verschlusskappe (Verschluss- und/oder Dichtkappe), vorzugsweise zum insbesondere abdichtenden Anliegen an den Adapter und/oder vorzugsweise zum insbesondere abdichtenden Aufsetzen auf den Adapter und/oder vorzugsweise zum abdichtenden Verschließen der Auslassöffnung und/oder des Adapters, aufweisen.

Die Auslassöffnung bzw. der Adapter kann dabei mit dem Verschlusselement verschlossen sein. Insbesondere ist es im Rahmen der vorliegenden Erfindung vorgesehen, dass die Auslassöffnung bzw. der Adapter zumindest bei der Sterilisation und vorzugsweise auch bei der Befüllung der Kolbenspritze mit den Zusammensetzungen bzw. bei Bereitstellung der Zusammensetzungen in der Kolbenspritze verschlossen ist.

Zudem kann das Verschlusselement den Adapter, insbesondere mitsamt der Auslassöffnung, zumindest im Wesentlichen vollständig und/oder abdichtend umschließen bzw. abdecken.

Im Allgemeinen kann das Verschlusselement somit die Auslassöffnung bzw. den Adapter verschließen. Insbesondere verhält es sich erfindungsgemäß derart, dass im Rahmen des vorliegend beschriebenen Verfahrens die Kolbenspritze bzw. die Auslassöffnung mit dem Verschlusselement verschlossen ist.

Was das Verschlusselement weiterhin anbelangt, so kann dieses auch wie folgt ausgebildet sein:
- Insbesondere kann das Verschlusselement zum vorzugsweise abdichtenden Anliegen an den Adapter und/oder zum insbesondere abdichtenden Aufsetzen auf den Adapter und/oder vorzugsweise zum abdichtenden Verschließen der Auslassöffnung des Adapters und/oder zum im Wesentlichen vollständigen und/oder abdichtenden Umschließen des Adapters ausgebildet sein.
- Erfindungsgemäß kann das Verschlusselement insbesondere kappen- bzw. hutförmig ausgebildet sein.
- Im Allgemeinen kann das Verschlusselement einen Aufnahmeraum, insbesondere zur zumindest im Wesentlichen vollständigen Aufnahme und/oder zum zumindest im Wesentlichen vollständigen Umschließen des Adapters, aufweisen. Insbesondere kann es sich erfindungsgemäß derart verhalten, dass das Verschlusselement den insbesondere stufenkegelförmig ausgebildeten Adapter zumindest im Wesentlichen vollständig umschließt.
- Zudem kann das Verschlusselement form- und/oder kraftschlüssig, insbesondere reibschlüssig, an dem Adapter anliegen und/oder anliegbar ausgebildet sein. Weiterhin kann das Verschlusselement den Adapter form- und/oder kraftschlüssig, insbesondere reibschlüssig, umschließen bzw. umschließbar ausgebildet sein.
- Das Verschlusselement kann im Allgemeinen im Rahmen der vorliegenden Erfindung aus einem elastomeren Material, insbesondere aus einem Gummimaterial und/oder Kautschukmaterial, gebildet sein.
- In bevorzugter Weise kann das Verschlusselement aus einem Halogenbutylkautschuk, insbesondere aus einem Chlorbutylkautschuk oder aus einem Brombutylkautschuk, vorzugsweise aus einem Brombutylkautschuk, gebildet sein bzw. hieraus bestehen. Hierzu kann auch auf die obigen Ausführungen zu dem Kolbenstopfen verwiesen werden.
- Erfindungsgemäß kann das Verschlusselement weiterhin eine Abnehm- und/oder Entfernungseinrichtung, insbesondere Abzieheinrichtung, vorzugsweise in Form eines insbesondere nippelförmig ausgebildeten Fortsatzes und/oder vorzugsweise zum insbesondere manuellen Abnehmen, insbesondere Abziehen, des Verschlusselements von dem Adapter, aufweisen. In diesem Zusammenhang kann die Abnehm- und/oder Entfernungseinrichtung an dem und/oder im Bereich des geschlossenen Endes und/oder an der Spitze des Verschlusselements und/oder in Verlängerung der Längsachse des Verschlusselements angeordnet sein.
- Zudem kann das Verschlusselement an dessen Außenseite mindestens eine Vertiefung, insbesondere Einkerbung, Einbuchtung und/oder Materialverjüngung, aufweisen. In diesem Zusammenhang kann die Vertiefung längsförmig und/oder linienförmig und/oder gradlinig verlaufend ausgebildet sein und zudem kann die Vertiefung zumindest im Wesentlichen in Längsrichtung und/oder zumindest im Wesentlichen parallel zur Längsachse des Verschlusselements (7) verlaufend angeordnet sein.
- Zudem kann das Verschlusselement zwei, drei, vier, fünf oder mehr, insbesondere zwei, drei oder vier, bevorzugt vier, Vertiefungen aufweisen. In diesem Zusammenhang können die Vertiefungen zumindest im Wesentlichen äquidistant zueinander verlaufend und/oder zumindest im Wesentlichen parallel zueinander verlaufend auf der Außenseite des Verschlusselements angeordnet sein.

Erfindungsgemäß kann es insbesondere vorgesehen sein, dass die Kolbenspritze bzw. der Adapter bzw. das Verschlusselement körperlich (d. h. mit Ausnahme des Materials, aus dem die Kolbenspritze gebildet ist, d. h. nicht die stoffliche Beschaffenheit der Kolbenspritze) gemäß der DE 10 2020 130 032 A1 und/oder gemäß der DE 20 2020 106 531 U1 ausgebildet sein.

Wie zuvor angeführt, kann es sich erfindungsgemäß auch derart verhalten, dass auch die im Verfahren, insbesondere in Verfahrensschritt b), auftretende Migration und/oder Einlagerung des Oxybutynins in das Verschlusselement gesteuert und/oder kompensiert werden kann, und zwar mit der vorgenannten Maßgabe und/oder unter Heranziehung der vorgenannten Maßnahmen und/oder Schritte (1), (2), (3) und/oder (4). Dieser Gesichtspunkt ist jedoch signifikant weniger relevant, da im Allgemeinen die Kontaktfläche zu der Zusammensetzung nur äußerst gering bzw. minimal ist. Dies belegen auch die Untersuchungen der Anmelderin.

Zusammenfassend kann es sich erfindungsgemäß insbesondere derart verhalten, dass die die im Verfahren, insbesondere in Verfahrensschritt b), auftretende Migration und/oder Einlagerung des Oxybutynins in den Spritzenkörper, insbesondere Spritzenzylinder, und/oder in den Kolbenstopfen und/oder in das Verschlusselement, und/oder der im Verfahren, insbesondere in Verfahrensschritt b), auftretende Abbau des Oxybutynins, insbesondere der reaktive Abbau des Oxybutynins, vorzugsweise der hydrolytisch und/oder oxidativ hervorgerufene Abbau, bevorzugt der Abbau zu Phenylcyclohexylhydroxyessigsäure (Verunreinigung D), gesteuert und/oder kompensiert werden kann, und zwar gleichermaßen mit der vorgenannten Maßgabe und/oder unter Heranziehung der vorgenannten Maßnahmen und/oder Schritte (1), (2), (3) und/oder (4).

Was weiterhin die in Verfahrensschritt (b) vorgesehene Hitzesterilisation anbelangt, so kann diese insbesondere wie folgt ausgebildet bzw. durchgeführt werden.

Im Allgemeinen kann die Hitzesterilisation, insbesondere Dampfsterilisation, vorzugsweise Wasserdampfsterilisation, bevorzugt terminale (Hitze-, Dampf-, Wasserdampf-)Sterilisation, erfindungsgemäß unter Temperaturbeaufschlagung (Erwärmung) durchgeführt werden.

Erfindungsgemäß kann die Hitzesterilisation, insbesondere Dampfsterilisation, vorzugsweise Wasserdampfsterilisation, bevorzugt terminale (Hitze-, Dampf-, Wasserdampf-)Sterilisation, bei Temperaturen von mindestens 105 °C, insbesondere mindestens 110 °C, vorzugsweise mindestens 115 °C, bevorzugt mindestens 120 °C, durchgeführt werden.

Insbesondere kann die Hitzesterilisation, insbesondere Dampfsterilisation, vorzugsweise Wasserdampfsterilisation, bevorzugt terminale (Hitze-, Dampf-, Wasserdampf-)Sterilisation, bei Temperaturen von höchstens 160 °C, insbesondere höchstens 150 °C, vorzugsweise höchstens 140° C bevorzugt höchstens 130° C, durchgeführt werden.

Erfindungsgemäß kann die Hitzesterilisation, insbesondere Dampfsterilisation, vorzugsweise Wasserdampfsterilisation, bevorzugt terminale (Hitze-, Dampf-, Wasserdampf-)Sterilisation, bei Temperaturen im Bereich von 105 °C bis 160 °C, insbesondere im Bereich von 110 °C bis 150 °C, vorzugsweise im Bereich von 115 °C bis 140 °C, bevorzugt im Bereich von 120 °C bis 130° C, besonders bevorzugt bei etwa 121 °C, durchgeführt werden.

Wie zuvor angeführt, kann die Hitzesterilisation erfindungsgemäß mit einem F0-Wert im Bereich von 6 min bis 45 min, insbesondere im Bereich von 8 min bis 40 min, vorzugsweise im Bereich von 10 min bis 38 min, bevorzugt im Bereich von 11 min bis 35 min, besonders bevorzugt im Bereich von 15 min bis 32 min, ganz besonders bevorzugt im Bereich von 19 min bis 30 min, durchgeführt werden. Hierzu kann auch auf obige Ausführungen verwiesen werden.

Insbesondere kann die Hitzesterilisation, insbesondere Dampfsterilisation, vorzugsweise Wasserdampfsterilisation, bevorzugt terminale (Hitze-, Dampf-, Wasserdampf-)Sterilisation, für einen (Gesamt-)Zeitraum von mindestens 1 min, insbesondere von mindestens 2 min, vorzugsweise von mindestens 3 min, bevorzugt von mindestens 4 min, besonders bevorzugt von mindestens 5 min, ganz besonders bevorzugt von mindestens 6 min, durchgeführt werden.

Zudem kann die Hitzesterilisation, insbesondere Dampfsterilisation, vorzugsweise Wasserdampfsterilisation, bevorzugt terminale (Hitze-, Dampf-, Wasserdampf-) Sterilisation, für einen (Gesamt-)Zeitraum von höchstens 600 min, insbesondere von höchstens 500 min, vorzugsweise von höchstens 400 min, bevorzugt von höchstens 350 min, besonders bevorzugt von höchstens 300 min, ganz besonders bevorzugt von höchstens 250 min, durchgeführt werden.

Gleichermaßen kann die Hitzesterilisation, insbesondere Dampfsterilisation, vorzugsweise Wasserdampfsterilisation, bevorzugt terminale (Hitze-, Dampf-, Wasserdampf-)Sterilisation, für einen (Gesamt-)Zeitraum im Bereich von 1 min bis 600 min, insbesondere im Bereich von 2 min bis 500 min, vorzugsweise im Bereich von 3 min bis 400 min, bevorzugt im Bereich von 4 min bis 350 min, besonders bevorzugt im Bereich von 5 min bis 300 min, ganz besonders bevorzugt im Bereich von 6 min bis 240 min, durchgeführt werden.

Erfindungsgemäß kann die Hitzesterilisation, insbesondere Dampfsterilisation, vorzugsweise Wasserdampfsterilisation, bevorzugt terminale (Hitze-, Dampf-, Wasserdampf-)Sterilisation, unter Druckbeaufschlagung durchgeführt werden.

Erfindungsgemäß kann es zudem vorgesehen sein, dass die Hitzesterilisation, insbesondere Dampfsterilisation, vorzugsweise Wasserdampfsterilisation, bevorzugt terminale (Hitze-, Dampf-, Wasserdampf-)Sterilisation, bei einem Relativdruck im Bereich von 0,1 bar bis 10 bar, insbesondere im Bereich von 0,5 bar bis 5 bar, vorzugsweise im Bereich von 1 bar bis 3,5 bar, bevorzugt im Bereich von 1,5 bar bis 3 bar, durchgeführt wird.

Insbesondere kann die Hitzesterilisation, insbesondere Dampfsterilisation, vorzugsweise Wasserdampfsterilisation, bevorzugt terminale (Hitze-, Dampf-, Wasserdampf-)Sterilisation, bei einem Absolutdruck im Bereich von 1,1 bar bis 10 bar, insbesondere im Bereich von 1,5 bar bis 6 bar, vorzugsweise im Bereich von 2 bar bis 4,5 bar, durchgeführt werden.

Gleichermaßen kann die Hitzesterilisation in Gegenwart einer insbesondere reinen Wasserdampf enthaltenden Atmosphäre und/oder als Sattdampfverfahren durchgeführt werden oder aber wobei die Dampfsterilisation in Gegenwart eines Wasserdampf/Gas-Gemisches, insbesondere eines Gemisches von Wasserdampf mit Inertgas, insbesondere Stickstoff, und/oder Sauerstoff, vorzugsweise eines Wasserdampf/Luft-Gemisches, durchgeführt werden.

Zudem kann die Hitzesterilisation, insbesondere Dampfsterilisation, vorzugsweise Wasserdampfsterilisation, bevorzugt terminale (Hitze-, Dampf-, Wasserdampf-) Sterilisation, in einer vorzugsweise geschlossenen Sterilisationsvorrichtung, insbesondere in einem gasdicht verschlossenen Druckbehälter, vorzugsweise in einer Autoklaviervorrichtung (Autoklav), durchgeführt werden.

In diesem Zusammenhang kann die Sterilisationsatmosphäre durch Injektion von Wasserdampf und/oder Wasserdampf/Druckluft-Gemischen, vorzugsweise Wasserdampf/Druckluft-Gemischen, in die Sterilisationseinrichtung erzeugt werden.

Zudem kann die Sterilisationsatmosphäre in diesem Zusammenhang durch Injektion von bereits vorgemischten Wasserdampf/Druckluft-Gemischen und/oder durch jeweils separate Injektionen von Wasserdampf und Druckluft in die Sterilisationseinrichtung erzeugt werden.

Erfindungsgemäß kann es zudem vorgesehen sein, dass bei der Hitzesterilisation, insbesondere Dampfsterilisation, vorzugsweise Wasserdampfsterilisation, bevorzugt terminale (Hitze-, Dampf-, Wasserdampf-)Sterilisation, der Wasserdampf aus destilliertem und/oder vollentsalztem Wasser erzeugt wird.

Zudem kann es erfindungsgemäß vorgesehen sein, dass bei der Hitzesterilisation, insbesondere Dampfsterilisation, vorzugsweise Wasserdampfsterilisation, bevorzugt terminale (Hitze-, Dampf-, Wasserdampf-)Sterilisation, die Druckluft aus steriler Luft erzeugt wird, insbesondere wobei die Luft mittels Durchströmen von Filtern gereinigt und/oder sterilisiert wird.

Erfindungsgemäß kann die Hitzesterilisation, insbesondere Dampfsterilisation, vorzugsweise Wasserdampfsterilisation, bevorzugt terminale (Hitze-, Dampf-, Wasserdampf-)Sterilisation, unter *overkill*-Bedingungen und/oder bei etwa 121 °C und/oder für einen Zeitraum von mindestens 6 min, vorzugsweise von etwa 6 min, oder von mindestens 20 min, vorzugsweise von etwa 20 min, durchgeführt werden.

Zudem kann die Hitzesterilisation, insbesondere Dampfsterilisation, vorzugsweise Wasserdampfsterilisation, bevorzugt terminale (Hitze-, Dampf-, Wasserdampf-) Sterilisation, gemäß Pharmacopoea Europaea (Ph. Eur.), Kapitel 5.1.1 (07/2017:50101) durchgeführt werden, insbesondere unter *overkill*-Bedingungen und/oder bei etwa 121 °C und/oder für einen Zeitraum von mindestens 15 min, insbesondere von mindestens 20 min, vorzugsweise von etwa 20 min.

Erfindungsgemäß hat es sich zudem als vorteilhaft erwiesen, wenn die Hitzesterilisation, insbesondere Dampfsterilisation, vorzugsweise Wasserdampfsterilisation, bevorzugt terminale (Hitze-, Dampf-, Wasserdampf-)Sterilisation, mehrzyklisch durchgeführt wird, insbesondere wobei zumindest der abschließende und/oder terminale Zyklus unter mindestens einer der vorgenannten Bedingungen durchgeführt wird. Diesbezüglich kann es insbesondere vorgesehen sein, dass der abschließende und/oder terminale Zyklus unter *overkill*-Bedingungen und/oder bei etwa 121 °C und/oder für einen Zeitraum von mindestens 6 min, vorzugsweise von etwa 6 min, oder von mindestens 20 min, vorzugsweise von etwa 20 min, durchgeführt wird.

Erfindungsgemäß kann es zudem vorgesehen sein, dass die mit der oxybutyninhaltigen Zusammensetzung befüllte Kolbenspritze, insbesondere vor Durchführung der Hitzesterilisation, insbesondere Dampfsterilisation, vorzugsweise Wasserdampfsterilisation, bevorzugt terminalen (Hitze-, Dampf-, Wasserdampf-) Sterilisation, in eine insbesondere wasser- und/oder keimdicht verschlossene und/oder versieaelte Verpackung, insbesondere Bereitschaftspackung, eingebracht ist bzw. worden ist. Diesbezüglich kann insbesondere ein Teil der Verpackung wenigstens wasserdampfdurchlässig, insbesondere wasserdampf- und/oder gasdurchlässig, ausgebildet sein. Erfindungsgemäß kann es sich insbesondere derart verhalten, dass die Verpackung bei Durchführung der Dampfsterilisation gleichermaßen sterilisiert wird.

Insbesondere im Hinblick auf eine großtechnische Anwendung kann es erfindungsgemäß vorgesehen sein, dass eine Vielzahl von mit der oxybutyninhaltigen Zusammensetzung befüllte Kolbenspritzen gleichzeitig sterilisiert werden. In diesem Zusammenhang kann es sich insbesondere derart verhalten, dass vorzugsweise während der Durchführung der Dampfsterilisation mehrere Kolbenspritzen auf einem (Spritzen-)Träger zusammengeführt sind.

Die nach Abschluss des Verfahrens, insbesondere nach Verfahrensschritt b), erhaltene sterile oxybutyninhaltige Zusammensetzung (Endzusammensetzung) und/oder die mit der oxybutyninhaltigen Zusammensetzung (Endzusammensetzung) befüllte, nach Abschluss des Verfahrens, insbesondere nach Verfahrensschritt b), erhaltene sterile Kolbenspritze kann insbesondere einen SAL-Wert (*Sterility Assurance Level*) von mindestens 10⁻⁵, insbesondere mindestens 10⁻⁶, vorzugsweise mindestens 10⁻⁷, aufweisen.

Insbesondere kann es erfindungsgemäß vorgesehen sein, dass die sterilisierte bzw. sterile oxybutyninhaltige Zusammensetzung (Endzusammensetzung) bzw. die im Rahmen des erfindungsgemäßen Verfahrens erhaltene und mit der oxybutyninhaltigen Zusammensetzung befüllte Kolbenspritze steril gemäß der DIN EN 566-1 ist.

Für weitere Ausführungen zu der dem erfindungsgemäßen Verfahren zugrundeliegenden Hitzesterilisation, insbesondere Dampfsterilisation kann insbesondere auf die DE 10 2017 104 931 A1 sowie auf die zu derselben Patentfamilie gehörende WO 2018/145799 A1 verwiesen werden, wobei der diesbezügliche Offenbarungsgehalt hiermit durch Bezugnahme vollumfänglich eingeschlossen ist. Zudem kann auch auf die DE 10 2011 105 840 A1 sowie auf die zu derselben Patentfamilie gehörende WO 2012/136313 A2 bzw. auf die US 2014/093422 A1 verwiesen werden.

Wie zuvor angeführt, wird im Rahmen des erfindungsgemäßen Verfahrens eine sterile oxybutyninhaltige Zusammensetzung mit hoher Stabilität bereitgestellt. Insbesondere weist die sterile oxybutyninhaltige Zusammensetzung einen geringen Gehalt an Verunreinigungen bzw. Abbauprodukten auf.

Insbesondere erfüllt die sterile oxybutyninhaltige Zusammensetzung (Endzusammensetzung) die zuvor definierten Spezifikationsvorgaben, und zwar auch nach längerer Lagerungszeit.

In diesem Zusammenhang kann es sich erfindungsgemäß insbesondere wie folgt verhalten:
- So kann es sich erfindungsgemäß insbesondere derart verhalten, dass die nach Abschluss des Verfahrens, insbesondere nach Verfahrensschritt b), erhaltene sterile oxybutyninhaltige Endzusammensetzung die vorgegebenen Spezifikationsvorgaben (i) sowie (ii) und/oder (iii), insbesondere (i), (ii) und (iii), auch nach Lagerung der mit der oxybutyninhaltigen Endzusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweist und/oder erfüllt.
- Zudem kann die nach Abschluss des Verfahrens, insbesondere nach Verfahrensschritt b), erhaltene sterile oxybutyninhaltige Endzusammensetzung die nachfolgend angeführten Spezifikationsvorgaben (i) sowie (ii) und/oder (iii), insbesondere (i), (ii) und (iii), auch nach Lagerung der mit der oxybutyninhaltigen Endzusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweisen bzw. erfüllen:
   (i) vorgegebene Spezifikations-Konzentration von Oxybutynin, bevorzugt in Form des Hydrochlorids (Oxybutyninhydrochlorid, Oxybutynin-HCl):
      0,5 mg/ml bis 2 mg/ml mit maximaler Abweichung von ± 5 %, bezogen auf die Konzentration von Oxybutynin, vorzugsweise 1 mg/ml mit maximaler Abweichung von ± 0,05 mg/ml,
   (ii) vorgegebener Spezifikations-pH-Wert:
      2,8 bis 5,2, insbesondere 3 bis 5, vorzugsweise 3,2 bis 4,8, bevorzugt 3,5 bis 4,5, besonders bevorzugt 3,5 bis 4,2, ganz besonders bevorzugt 3,8 bis 4,2,
   (iii) vorgegebene Spezifikations-Menge an Oxybutynin-Abbauprodukt(en), insbesondere reaktiv hervorgerufenen Oxybutynin-Abbauprodukt(en), vorzugsweise hydrolytisch und/oder oxidativ hervorgerufenen Oxybutynin-Abbauprodukt(en), vorzugsweise Phenylcyclohexylhydroxyessigsäure (Verunreinigung D):
      höchstens 0,5 Gew.-%, insbesondere höchstens 0,2 Gew.-%, bezogen auf die Gesamtmenge an eingesetztem Oxybutynin, bevorzugt Oxybutyninhydrochlorid.
- Zudem kann die nach Abschluss des Verfahrens, insbesondere nach Verfahrensschritt b), erhaltene sterile oxybutyninhaltige Endzusammensetzung die vorgegebenen Spezifikationsvorgabe (iv), insbesondere die vorgegebenen Spezifikations-Menge (iv) an Elektrolyten, insbesondere Alkalimetallsalz, vorzugsweise Alkalimetallchlorid, bevorzugt Natriumchlorid (NaCl), im Bereich von 0,1 Gew.-% bis 2 Gew.-%, insbesondere im Bereich von 0,5 Gew.-% bis 1,5 Gew.-%, vorzugsweise im Bereich von 0,7 Gew.-% bis 1,2 Gew.-%, bevorzugt von etwa 0,9 Gew.-%, bezogen auf die Endzusammensetzung, und/oder die vorgegebenen Spezifikations-Konzentration (iv) an Natriumchlorid, berechnet als Natrium, von (3,5 ± 0,5) mg/ml, insbesondere (3,5 ± 0,3) mg/ml, vorzugsweise (3,5 ± 0,1) mg/ml, bevorzugt etwa 3,5 mg/ml, auch nach Lagerung der mit der oxybutyninhaltigen Endzusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweisen bzw. erfüllen.
- Zudem kann es sich erfindungsgemäß derart verhalten, dass die nach Abschluss des Verfahrens, insbesondere nach Verfahrensschritt b), erhaltene sterile oxybutyninhaltige Endzusammensetzung die vorgegebenen Spezifikationsvorgabe (v), insbesondere die vorgegebene Spezifikations-Menge an Gesamt-Abbauprodukten von höchstens 1 Gew.-%, insbesondere höchstens 0,5 Gew.-%, bezogen auf die Gesamtmenge an eingesetztem Oxybutynin, bevorzugt Oxybutyninhydrochlorid, auch nach Lagerung der mit der oxybutyninhaltigen Endzusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweist bzw. erfüllt.

In Bezug auf die Ausgestaltung des vorliegend beschriebenen Verfahrens gemäß dem vorliegenden Aspekt kann auch auf die nachfolgenden Ausführungen zu den weiteren erfindungsgemäßen bzw. vorliegend beschriebenen Aspekten verwiesen werden, welche entsprechend gelten.

Darüber hinaus ist vorliegend beschrieben - gemäß einem vorliegend beschriebenen Aspekt - auch die sterile oxybutyninhaltige Zusammensetzung (Endzusammensetzung), insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen,
wobei die oxybutyninhaltige Zusammensetzung nach dem vorliegend beschrieben Verfahren erhältlich bzw. erhalten ist; und/oder
wobei die oxybutyninhaltige Zusammensetzung nach dem vorliegend beschriebenen Verfahren hitzesterilisiert, insbesondere dampfsterilisiert, vorzugsweise wasserdampfsterilisiert, bevorzugt terminal (hitze-, dampf-, wasserdampf-) sterilisiert, ist bzw. worden ist.

Gemäß diesem Aspekt ist vorliegend weiterhin auch beschrieben die sterile oxybutyninhaltige Zusammensetzung (Endzusammensetzung), insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere die zuvor definierte Zusammensetzung,
wobei die oxybutyninhaltige Zusammensetzung in einer vorzugsweise anwendungsfertigen Kolbenspritze, insbesondere Einweg-Kolbenspritze, vorliegt,
wobei die Kolbenspritze einen Spritzenkörper, insbesondere Spritzenzylinder, einerseits und einen Spritzenkolben mit einem Kolbenstopfen andererseits aufweist, wobei der Spritzenkörper, insbesondere Spritzenzylinder, aus einem Polypropylen (PP) gebildet ist, und
wobei die oxybutyninhaltige Zusammensetzung als sterile wässrige Zusammensetzung, insbesondere terminal sterilisierte Zusammensetzung, ausgebildet ist und die nachfolgend angeführten Spezifikationsvorgaben (i) sowie (ii) und/oder (iii), vorzugsweise (i), (ii) und (iii), aufweist:
   (i) vorgegebene Spezifikations-Konzentration von Oxybutynin, bevorzugt in Form des Hydrochlorids (Oxybutyninhydrochlorid, Oxybutynin-HCl):
      0,5 mg/ml bis 2 mg/ml mit maximaler Abweichung von ± 5 %, bezogen auf die Konzentration von Oxybutynin, vorzugsweise 1 mg/ml mit maximaler Abweichung von ± 0,05 mg/ml,
   (ii) vorgegebener Spezifikations-pH-Wert:
      2,8 bis 5,2, insbesondere 3 bis 5, vorzugsweise 3,2 bis 4,8, bevorzugt 3,5 bis 4,5, besonders bevorzugt 3,5 bis 4,2, ganz besonders bevorzugt 3,8 bis 4,2,
   (iii) vorgegebene Spezifikations-Menge an Oxybutynin-Abbauprodukt(en), insbesondere reaktiv hervorgerufenen Oxybutynin-Abbauprodukt(en), vorzugsweise hydrolytisch und/oder oxidativ hervorgerufenen Oxybutynin-Abbauprodukt(en), vorzugsweise Phenylcyclohexylhydroxyessigsäure (Verunreinigung D):
      höchstens 0,5 Gew.-%, insbesondere höchstens 0,2 Gew.-%, bezogen auf die Gesamtmenge an eingesetztem Oxybutynin, bevorzugt Oxybutyninhydrochlorid;
wobei die in der Kolbenspritze enthaltene oxybutyninhaltige Zusammensetzung einschließlich der Kolbenspritze hitzesterilisiert, insbesondere dampfsterilisiert, vorzugsweise wasserdampfsterilisiert, bevorzugt terminal (hitze-, dampf-, wasserdampf-)sterilisiert, worden ist.

Die vorliegend beschriebene oxybutyninhaltige Zusammensetzung kann zudem als weitere Spezifikationsvorgabe (iv) mindestens einen Elektrolyten, insbesondere Alkalimetallsalz, vorzugsweise Alkalimetallchlorid, bevorzugt Natriumchlorid (NaCl), aufweisen, insbesondere in einer vorgegebenen Spezifikations-Menge im Bereich von 0,1 Gew.-% bis 2 Gew.-%, insbesondere im Bereich von 0,5 Gew.-% bis 1,5 Gew.-%, vorzugsweise im Bereich von 0,7 Gew.-% bis 1,2 Gew.-%, bevorzugt von etwa 0,9 Gew.-%, bezogen auf die Endzusammensetzung.

Insbesondere kann die Oxybutyninzusammensetzung als weitere Spezifikationsvorgabe (iv) Natriumchlorid, berechnet als Natrium, in einer vorgegebenen Spezifikations-Konzentration von (3,5 ± 0,5) mg/ml, insbesondere (3,5 ± 0,3) mg/ml, vorzugsweise (3,5 ± 0,1) mg/ml, bevorzugt etwa 3,5 mg/ml, enthalten.

Weiterhin kann die oxybutyninhaltige Zusammensetzung als weitere Spezifikationsvorgabe (v) eine vorgegebene Spezifikations-Menge an Gesamt-Abbauprodukten von höchstens 1 Gew.-%, insbesondere höchstens 0,5 Gew.-%, bezogen auf die Gesamtmenge an eingesetztem Oxybutynin, bevorzugt Oxybutyninhydrochlorid, aufweisen. Wie zuvor angeführt, umfasst die Spezifikations-Menge an Gesamt-Abbauprodukten auch die Verunreinigung D.

Erfindungsgemäß ist es insbesondere vorgesehen, dass der Kolbenstopfen aus einem elastomeren Material, insbesondere aus einem Kautschukmaterial und/oder Gummimaterial, vorzugsweise Kautschukmaterial, gebildet ist. Erfindungsgemäß ist es bevorzugt, dass der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist bzw. hieraus besteht.

Was die vorliegend beschriebene Zusammensetzung anbelangt, so kann diese unter mindestens einer der zuvor für das vorliegend beschriebene Verfahren definierten Bedingungen hitzesterilisiert, insbesondere dampfsterilisiert, vorzugsweise wasserdampfsterilisiert, bevorzugt terminal (hitze-, dampf-, wasserdampf-) sterilisiert, sein bzw. worden sein.

Erfindungsgemäß kann es insbesondere vorgesehen sein, dass die oxybutyninhaltige Zusammensetzung mit einem F0-Wert im Bereich von 6 min bis 45 min, insbesondere im Bereich von 8 min bis 40 min, vorzugsweise im Bereich von 10 min bis 38 min, bevorzugt im Bereich von 11 min bis 35 min, besonders bevorzugt im Bereich von 15 min bis 32 min, ganz besonders bevorzugt im Bereich von 19 min bis 30 min, hitzesterilisiert, insbesondere dampfsterilisiert, vorzugsweise wasserdampfsterilisiert, bevorzugt terminal (hitze-, dampf-, wasserdampf-)sterilisiert, ist bzw. worden ist.

Insbesondere kann die Zusammensetzung unter *overkill*-Bedingungen und/oder bei etwa 121 °C und/oder für einen Zeitraum von mindestens 6 min, vorzugsweise von etwa 6 min, oder von mindestens 20 min, vorzugsweise von etwa 20 min, hitzesterilisiert, insbesondere dampfsterilisiert, vorzugsweise wasserdampfsterilisiert, bevorzugt terminal (hitze-, dampf-, wasserdampf-)sterilisiert, sein bzw. worden sein.

Erfindungsgemäß kann es insbesondere vorgesehen sein, dass die Zusammensetzung gemäß Pharmacopoea Europaea (Ph. Eur.), Kapitel 5.1.1 (07/2017:50101), insbesondere unter *overkill*-Bedingungen und/oder bei etwa 121 °C und/oder für einen Zeitraum von mindestens 15 min, insbesondere von mindestens 20 min, vorzugsweise von etwa 20 min, hitzesterilisiert, insbesondere dampfsterilisiert, vorzugsweise wasserdampfsterilisiert, bevorzugt terminal (hitze-, dampf-, wasserdampf-)sterilisiert, ist bzw. worden ist.

Im Hinblick auf die vorliegend beschriebene Zusammensetzung verhält es sich insbesondere derart, dass diese einschließlich der diesbezüglichen Kolbenspritze hitzesterilisiert, insbesondere dampfsterilisiert, vorzugsweise wasserdampfsterilisiert, bevorzugt terminal (hitze-, dampf-, wasserdampf-)sterilisiert, ist bzw. worden ist.

Die vorliegend beschriebene Zusammensetzung zeichnet sich insgesamt durch definierte Spezifikationsvorgaben und deren Ein- bzw. Aufrechterhaltung (und zwar auch für längere Lagerzeiten) aus, und zwar insbesondere auch als Folge des vorliegend beschriebenen Verfahrens zu deren Herstellung, so dass das vorliegend beschriebene Verfahren seinen unmittelbaren Niederschlag auch in der vorliegend beschriebenen Zusammensetzung findet.

Insbesondere zeichnet sich die Zusammensetzung auch über eine hohe (Lager-) Stabilität aus:
So kann es sich erfindungsgemäß insbesondere derart verhalten, dass die oxybutyninhaltige Zusammensetzung die vorgegebenen Spezifikationsvorgaben (i) sowie (ii) und/oder (iii), insbesondere (i), (ii) und (iii), auch nach Lagerung der mit der oxybutyninhaltigen Zusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweist bzw. erfüllt.

Zudem kann es sich erfindungsgemäß auch derart verhalten, dass die oxybutyninhaltige Zusammensetzung die nachfolgend angeführten Spezifikationsvorgaben (i) sowie (ii) und/oder (iii), insbesondere (i), (ii) und (iii), auch nach Lagerung der mit der oxybutyninhaltigen Zusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweist bzw. erfüllt:
(i) vorgegebene Spezifikations-Konzentration von Oxybutynin, bevorzugt in Form des Hydrochlorids (Oxybutyninhydrochlorid, Oxybutynin-HCl):
   0,5 mg/ml bis 2 mg/ml mit maximaler Abweichung von ± 5 %, bezogen auf die Konzentration von Oxybutynin, vorzugsweise 1 mg/ml mit maximaler Abweichung von ± 0,05 mg/ml,
(ii) vorgegebener Spezifikations-pH-Wert:
   2,8 bis 5,2, insbesondere 3 bis 5, vorzugsweise 3,2 bis 4,8, bevorzugt 3,5 bis 4,5, besonders bevorzugt 3,5 bis 4,2, ganz besonders bevorzugt 3,8 bis 4,2,
(iii) vorgegebene Spezifikations-Menge an Oxybutynin-Abbauprodukt(en), insbesondere reaktiv hervorgerufenen Oxybutynin-Abbauprodukt(en), vorzugsweise hydrolytisch und/oder oxidativ hervorgerufenen Oxybutynin-Abbauprodukt(en), vorzugsweise Phenylcyclohexylhydroxyessigsäure (Verunreinigung D):
   höchstens 0,5 Gew.-%, insbesondere höchstens 0,2 Gew.-%, bezogen auf die Gesamtmenge an eingesetztem Oxybutynin, bevorzugt Oxybutyninhydrochlorid.

Insbesondere kann die oxybutyninhaltige Zusammensetzung die vorgegebene Spezifikationsvorgabe (iv), insbesondere die vorgegebenen Spezifikations-Menge (iv) an Elektrolyten, insbesondere Alkalimetallsalz, vorzugsweise Alkalimetallchlorid, bevorzugt Natriumchlorid (NaCl), im Bereich von 0,1 Gew.-% bis 2 Gew.-%, insbesondere im Bereich von 0,5 Gew.-% bis 1,5 Gew.-%, vorzugsweise im Bereich von 0,7 Gew.-% bis 1,2 Gew.-%, bevorzugt von etwa 0,9 Gew.-%, bezogen auf die Endzusammensetzung, und/oder die vorgegebenen Spezifikations-Konzentration (iv) an Natriumchlorid, berechnet als Natrium, von (3,5 ± 0,5) mg/ml, insbesondere (3,5 ± 0,3) mg/ml, vorzugsweise (3,5 ± 0,1) mg/ml, bevorzugt etwa 3,5 mg/ml, auch nach Lagerung der mit der oxybutyninhaltigen Zusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweisen bzw. erfüllen.

Gleichermaßen kann die oxybutyninhaltige Zusammensetzung die vorgegebene Spezifikationsvorgabe (v), insbesondere die vorgegebene Spezifikations-Menge an Gesamt-Abbauprodukten von höchstens 1 Gew.-%, insbesondere höchstens 0,5 Gew.-%, bezogen auf die Gesamtmenge an eingesetztem Oxybutynin, bevorzugt Oxybutyninhydrochlorid, auch nach Lagerung der mit der oxybutyninhaltigen Zusammensetzung befüllten sterilen Kolbenspritze bei Temperaturen im Bereich von 25 °C bis 40 °C, bei einer relativen Luftfeuchtigkeit im Bereich von 60 % bis 75 % und bei einem Druck von 1.013,25 mbar von mindestens 10 Tagen, insbesondere mindestens 30 Tagen, vorzugsweise mindestens 3 Monaten, bevorzugt mindestens 6 Monaten, besonders bevorzugt mindestens 12 Monaten, aufweisen bzw. erfüllen.

Zudem kann die oxybutyninhaltige Zusammensetzung und/oder die mit der oxybutyninhaltigen Zusammensetzung befüllte sterile Kolbenspritze einen SAL-Wert (*Sterility* Assurance) von mindestens 10⁻⁵, insbesondere mindestens 10⁻⁶, vorzugsweise mindestens 10⁻⁷, aufweisen.

Erfindungsgemäß verhält es sich im Allgemeinen insbesondere derart, dass die neurogenen Blasenfunktionsstörungen mit einer Detrusorüberaktivität und/oder Detrusor-Sphinkter-Dyssynergie, insbesondere mit überaktivem Detrusor, einhergehen bzw. hierdurch gekennzeichnet sind.

Insbesondere können die neurogenen Blasenfunktionsstörungen mit einer Rückenmarksverletzung, insbesondere einer Querschnittlähmung, Spina bifida, Diabetes, Multiple Sklerose, Schlaganfall oder Morbus Parkinson im Zusammenhang stehen bzw. hierdurch verursacht sein.

Vorliegend beschrieben ist gleichermaßen auch die sterile oxybutyninhaltige Zusammensetzung zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere wobei die neurogenen Blasenfunktionsstörungen mit einer Detrusorüberaktivität und/oder Detrusor-Sphinkter-Dyssynergie, insbesondere mit überaktivem Detrusor, einhergehen und/oder hierdurch gekennzeichnet sind und/oder insbesondere wobei die neurogenen Blasenfunktionsstörungen mit einer Rückenmarksverletzung, insbesondere einer Querschnittlähmung, Spina bifida, Diabetes, Multiple Sklerose, Schlaganfall oder Morbus Parkinson im Zusammenhang stehen oder hierdurch verursacht sind.

Insbesondere kann die vorliegend beschriebene Zusammensetzung zur Instillation bzw. zur vorzugsweise topischen Applikation in den Urogenitalbereich, insbesondere in die Harnblase, vorgesehen oder hergerichtet sein.

Zudem kann die oxybutyninhaltige Zusammensetzung nach der Erfindung zur Verabreichung bzw. zur Instillation bzw. zur vorzugsweise topischen Applikation in den Urogenitaltrakt, insbesondere in die Harnblase, vorgesehen bzw. hergerichtet sein. Insbesondere kann die oxybutyninhaltige Zusammensetzung mittels Instillation bzw. mittels topischer Applikation in den Urogenitaltrakt, insbesondere in die Harnblase, verabreicht werden.

Im Hinblick auf weitere Ausgestaltungen der vorliegend beschriebene Zusammensetzung kann auch auf die Ausführungen zu den weiteren erfindungsgemäßen bzw. vorliegend beschriebenen Aspekten verwiesen werden, welche vorliegend entsprechend gelten.

Die vorliegende Erfindung betrifft weiterhin - gemäß einem wiederum **weiteren** Aspekt der vorliegenden Erfindung - auch die erfindungsgemäße Verpackungseinheit, enthaltend mindestens eine Verpackung und mindestens eine Kolbenspritze, insbesondere Einweg-Kolbenspritze, wie zuvor definiert, wobei die Kolbenspritze in die Verpackung eingebracht ist bzw. in der Verpackung vorliegt.

In diesem Zusammenhang kann es sich erfindungsgemäß insbesondere derart verhalten, dass die erfindungsgemäße Verpackungseinheit bzw. die diesbezügliche Verpackung mit der diesbezüglichen Kolbenspritze und der oxybutyninhaltigen Zusammensetzung gemäß dem vorliegend beschriebenen Verfahren, wie zuvor definiert, hitzesterilisiert, insbesondere dampfsterilisiert, worden ist. Darüber hinaus kann die Verpackungseinheit nach der Erfindung im Allgemeinen als Bereitschaftspackung ausgebildet sein. Insbesondere kann die Verpackungseinheit nach der Erfindung wasser- und/oder keimdicht verschlossen bzw. versiegelt sein. Weiterhin kann zumindest ein Teil der Verpackung wenigstens wasserdampfdurchlässig, insbesondere wasserdampf- und/oder gasdurchlässig ausgebildet sein.

Für weitere Ausführungen bzw. Ausgestaltungen zu der erfindungsgemäßen Verpackungseinheit kann zudem auf die weiteren Ausführungen zu den weiteren Aspekten der vorliegenden Erfindung bzw. zu den vorliegend beschriebenen Aspekten verwiesen werden, welche entsprechend gelten.

Wiederum weiterer Gegenstand der vorliegenden Erfindung - gemäß einem **weiteren** Aspekt der vorliegenden Erfindung - ist zudem das erfindungsgemäße Kit, insbesondere Applikations- und/oder Instillationssystem, umfassend (i) mindestens eine in einer Kolbenspritze, insbesondere Einweg-Kolbenspritze, insbesondere wie zuvor definiert, vorliegende oxybutyninhaltige Zusammensetzung, wie zuvor definiert; (ii) (a) mindestens eine an die Kolbenspritze anschließbare Applikations- und/oder Instillationsvorrichtung, insbesondere in Form eines Instillationsschlauches oder dergleichen, vorzugsweise in Form eines (Harnblasen-)Katheters, und/oder (b) mindestens eine an die Kolbenspritze anschließbare Anschlussvorrichtung zum Anschließen an eine Applikations- und/oder Instillationsvorrichtung, insbesondere in Form eines Instillationsschlauches oder dergleichen, vorzugsweise in Form eines (Harn-blasen-)Katheters; und gegebenenfalls (iii) mindestens eine Applikations- und/oder Instillationsanleitung.

Gemäß diesem Aspekt betrifft die vorliegende Erfindung auch das weitere erfindungsgemäße Kit, insbesondere Applikations- und/oder Instillationssystem, umfassend (i) mindestens eine in einer Kolbenspritze, insbesondere Einweg-Kolbenspritze, insbesondere wie zuvor definiert, vorliegende oxybutyninhaltige Zusammensetzung, insbesondere wie zuvor definiert; (ii) (a) mindestens eine an die Kolbenspritze, insbesondere an den Adapter der Kolbenspritze, anschließbare Applikations- und/oder Instillationsvorrichtung, insbesondere in Form eines Instillationsschlauches oder dergleichen, vorzugsweise in Form eines (Harn-blasen-)-Katheters; gegebenenfalls (iii) ein Adapter-Gegenstück zum Anschließen der Applikations- und/oder Instillationsvorrichtung an die Kolbenspritze insbesondere an den Adapter der Kolbenspritze; und gegebenenfalls (iv) mindestens eine Applikations- und/oder Instillationsanleitung.

Dabei kann die erfindungsgemäße Kolbenspritze mit der diesbezüglichen sterilen oxybutyninhaltigen Zusammensetzung, wie zuvor definiert, auch in Form der zuvor beschriebenen Verpackungseinheit eingesetzt werden.

Hinsichtlich weiterer diesbezüglicher Ausgestaltungen kann zudem auf die Ausführungen zu den weiteren erfindungsgemäßen bzw. vorliegend beschriebenen Aspekten verwiesen werden, welche entsprechend gelten.

Nochmals weiter ist vorliegend beschrieben- gemäß einem vorliegend beschriebenen Aspekt-zudem die Verwendung einer Kolbenspritze, insbesondere Einweg-Kolbenspritze, in einem Verfahren zur Herstellung einer sterilen oxybutyninhaltigen Zusammensetzung, insbesondere wie zuvor definiert. Hinsichtlich der körperlichen Ausbildung der Kolbenspritze kann auf obige Ausführungen verwiesen werden, welche entsprechend gelten. Die erfindungsgemäß verwendete Kolbenspritze weist dabei einen Spritzenkörper, insbesondere Spritzenzylinder, einerseits und einen Spritzenkolben mit einem Kolbenstopfen andererseits auf, wobei der Spritzenkörper insbesondere Spritzenzylinder, aus einem Polypropylen (PP) gebildet ist.

Diesbezüglich kann auch auf die Ausführungen zu den weiteren erfindungsgemäßen bzw. vorliegend beschriebenen Aspekten verwiesen werden, welche vorliegend entsprechend gelten.

Zudem ist vorliegend beschrieben - gemäß einem vorliegend beschriebenen Aspekt - zudem die Verwendung einer Hitzesterilisation, insbesondere Dampfsterilisation, vorzugsweise Wasserdampfsterilisation, bevorzugt terminalen (Hitze-, Dampf-, Wasserdampf-)-Sterilisation, in einem Verfahren zur Herstellung einer sterilen oxybutyninhaltigen Zusammensetzung, insbesondere wie zuvor definiert, in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze, insbesondere wie zuvor definiert.

Hinsichtlich der körperlichen Ausbildung der Kolbenspritze kann auf die obigen Ausführungen verwiesen werden, welche entsprechend gelten. Die erfindungsgemäß verwendete Kolbenspritze weist dabei einen Spritzenkörper, insbesondere Spritzenzylinder, einerseits und einen Spritzenkolben mit einem Kolbenstopfen andererseits auf, wobei der Spritzenkörper insbesondere Spritzenzylinder, aus einem Polypropylen (PP) gebildet ist.

Für weitere diesbezügliche Ausführungen zu der vorliegend beschriebenen Verwendung gemäß diesem Aspekt kann auch auf die Ausführungen zu den weiteren erfindungsgemäßen bzw. vorliegend Aspekten verwiesen werden, welche vorliegend entsprechend gelten.

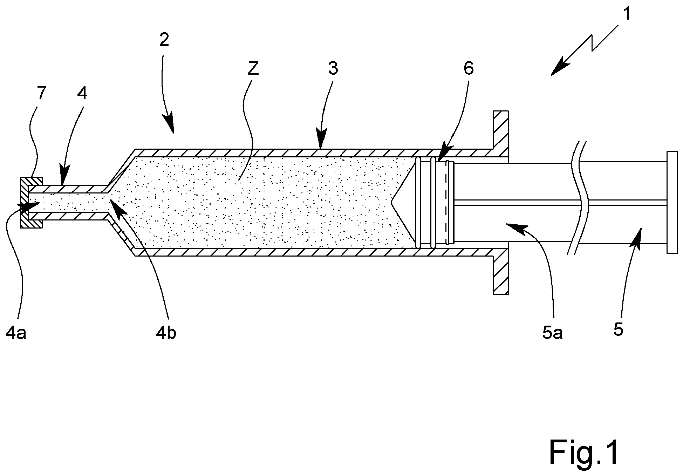

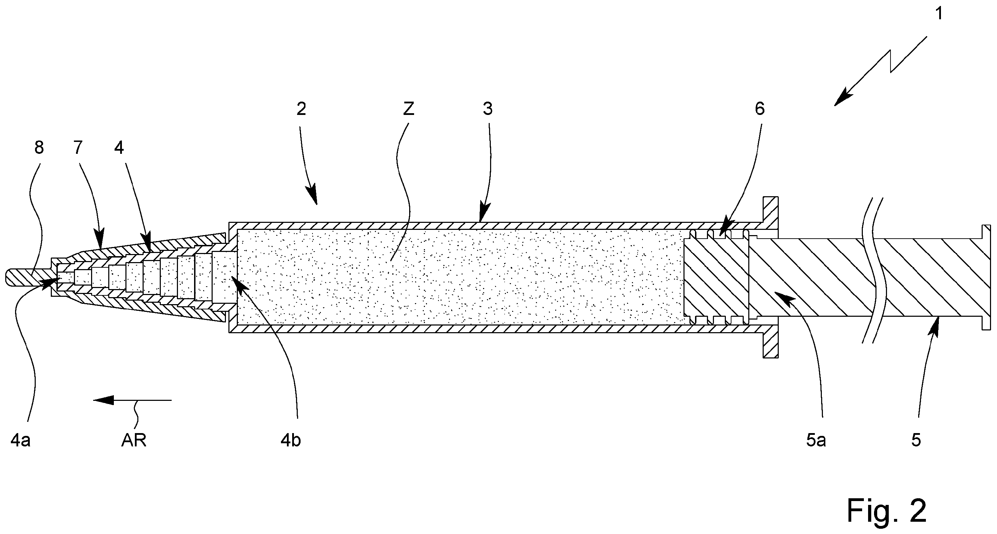

Die vorliegende Erfindung wird nachfolgend auch anhand von bevorzugte Ausführungsformen bzw. Ausgestaltungen darstellenden Figurendarstellungen beschrieben. Im Zusammenhang mit der Erläuterung dieser bevorzugten Ausführungsform bzw. Ausgestaltung der vorliegenden Erfindung, welche jedoch in Bezug auf die vorliegende Erfindung keinesfalls beschränkend ist, werden auch weitergehende Vorteile, Eigenschaften, Aspekte und Merkmale der vorliegenden Erfindung aufgezeigt. Zudem kann für die in Fig. 1 und Fig. 2 dargestellten Bezugszeichen auf die nachfolgende Bezugszeichenliste verwiesen werden.

In den Figurendarstellungen zeigt:
- Fig. 1: eine schematische Schnittdarstellung einer im Rahmen des vorliegend beschriebenen Verfahrens erhaltenen Kolbenspritze bzw. einer mit der sterilen oxybutyninhaltigen Zusammensetzung befüllten Kolbenspritze nach der Erfindung;
- Fig. 2: eine schematische Schnittdarstellung einer weiteren im Rahmen des vorliegend beschriebenen Verfahrens verwendbaren bzw. erhaltenen Kolbenspritze bzw. einer mit der sterilen oxybutyninhaltigen Zusammensetzung befüllten Kolbenspritze nach der Erfindung; Fig. 2 zeigt dabei eine bevorzugte Ausführungsform der vorliegenden Erfindung, wonach die Kolbenspritze einen stufenkegelförmig ausgebildeten Adapter bzw. einen Stufenkegeladapter aufweist, wobei der Spritzenkörper mit dem Adapter einstückig ausgebildet ist bzw. wonach der Adapter mit dem Spritzenzylinder verbunden, insbesondere fest bzw. unlösbar verbunden, vorzugsweise stoffschlüssig verbunden, ist; Fig. 2 zeigt auch eine weitere erfindungsgemäße Ausführungsform, wonach der Adapter einschließlich der Auslassöffnung mit einem Verschlusselement in Form einer Verschlusskappe verschlossen bzw. zumindest im Wesentlichen vollständig bedeckt ist;

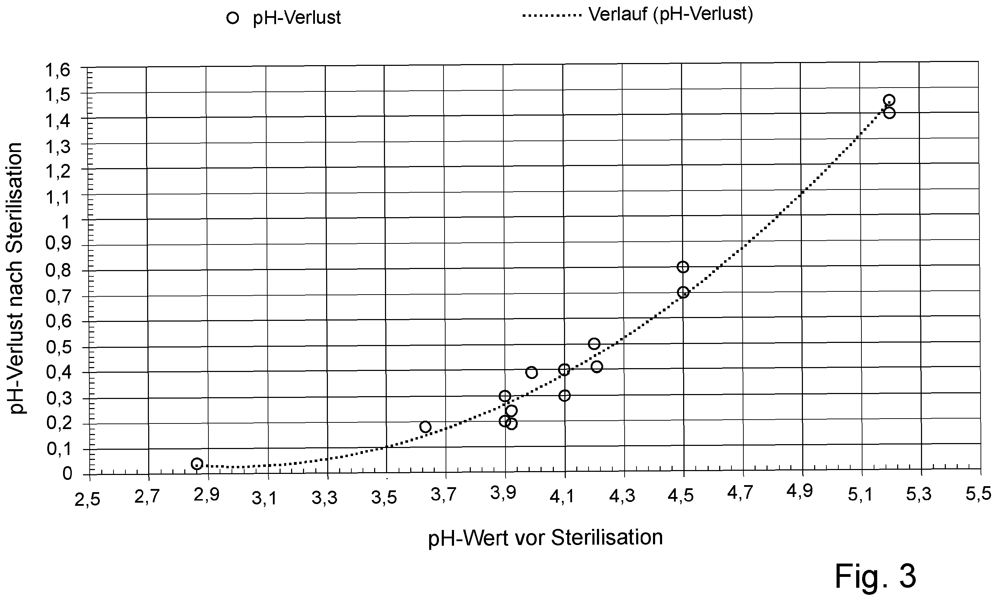

- Fig. 3: eine grafische Darstellung der Abhängigkeit der für sterile oxybutyninhaltige Zusammensetzungen (Endzusammensetzungen) nach Durchführung des vorliegend beschriebenen Verfahrens bzw. nach Hitzesterilisation vorliegende Verringerung des pH-Werts (pH-Verlust) in Abhängigkeit des Ausgangs-pH-Werts (Start-pH-Wert) der Ausgangszusammensetzung vor Wärmesterilisation;

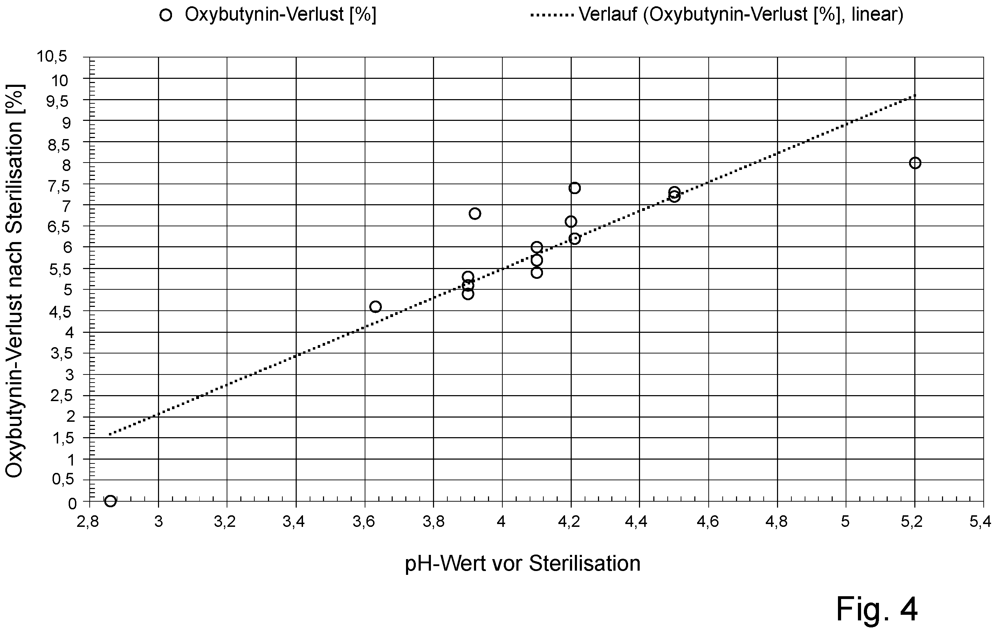

- Fig. 4: eine grafische Darstellung der Abhängigkeit der Migrations- bzw. Einlagerungsrate von Oxybutynin (Verlust an Oxybutynin nach Wärmesterilisation) in Abhängigkeit des pH-Werts der Ausgangszusammensetzung (Zusammensetzung bzw. Lösung vor Wärmesterilisation).

Für weitergehende Einzelheiten zu in den Figurendarstellungen dargestellten Ausführungsformen und Ausführungsbeispiele kann zur Vermeidung unnötiger Wiederholungen auf die obigen Ausführungen sowie auf die nachfolgenden ergänzenden Ausführungen zur vorliegenden Erfindung und auf die Ausführungen zu den Ausführungsbeispielen verwiesen werden, welche in Bezug auf die Figurendarstellungen entsprechend gelten. Zudem kann für die in Fig. 1 und Fig. 2 dargestellten Bezugszeichen auf die nachfolgende Bezugszeichenliste verwiesen werden.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE

### UND WEITERE AUSFÜHRUNGEN ZUR VORLIEGENDEN ERFINDUNG:

### Zielsetzung der nachfolgenden Versuchsreihen

Vorliegend soll eine sterile oxybutyninhaltige Zusammensetzung her- bzw. bereitgestellt werden, welche die in Tabelle 1 angeführten Spezifikationsvorgaben erfüllt.

In diesem Zusammenhang wird eine 0,1 %ige Oxybutyninlösung verwendet. Die sterile oxybutyninhaltige Zusammensetzung soll in einer anwendungsfertigen Einweg-Kolbenspritze aus Polypropylen bereitgestellt werden, wobei die Einweg-Kolbenspritze und die oxybutyninhaltige Zusammensetzung gleichzeitig thermisch sterilisiert werden sollen. Der Wirkstoff Oxybutynin wird in den nachfolgenden wässrigen Zusammensetzung aufgrund seiner Löslichkeit als Hydrochlorid (Oxybutyninhydrochlorid bzw. Oxybutynin-HCl, Strukturformel (3)) eingesetzt.

**Tabelle 1: Spezifikationsvorgaben der sterilen oxybutyninhaltigen Zusammensetzung nach Sterilisation in der Kolbenspritze (Verunreinigung D = Phenylcyclohexylhydroxyessigsäure)**

| **Parameter** | **Spezifikation** |
|---|---|
| Gehalt Oxybutynin | 95 - 105 % |
| Verunreinigung D | ≤ 0,2 Gew.-% |
| weitere Verunreinigungen | ≤ 0,2 Gew.-% |
| Summe der Verunreinigungen | ≤ 0,5 Gew.-% |
| pH-Wert | 3,5 - 4,2 |

### Hintergrund

Die Vorversuche der Anmelderin, welche nicht auf Basis des vorliegend beschriebenen Verfahrens durchgeführt werden, deuten darauf hin, dass eine unsterile oxybutyninhaltige Lösung in einem durch terminale, thermische Sterilisation behandelten Zylinder nicht innerhalb der vorgegebenen Spezifikation liegt, wogegen bei Primärpackmitteln bzw. Zylindern aus Cycloolefin-Copolymer (COC) die angeführten Werte im Allgemeinen erreicht werden (vgl. Tabelle 1).

Die Vorversuche zeigen einen Verlust von etwa 10 % des Oxybutynins nach Sterilisation in einer Spritze aus Polypropylen PP sowie die Bildung des Hydrolyseprodukts Phenylcyclohexylhydroxyessigsäure (Verunreinigung D, Strukturformel 2) auf 0,7 %. Folglich werden die in Tabelle 1 definierten Spezifikationsvorgaben nicht eingehalten. Dagegen weist die Lösung nach Sterilisation in COC einen geringeren Verlust auf.

### Oxybutynin in Polypropylen-Aufbewahrungsvorrichtungen (Kolbenspritzen)

Die obigen Vorversuche können als Hintergrund bzw. Grundlage für die nachfolgend angeführten erfindungsgemäßen Versuche und Fragestellungen herangezogen werden.

Dabei sind insbesondere drei Aspekte von Relevanz, nämlich Verständnis des Verlustes der Oxybutynin-Konzentration nach Sterilisation, Abhängigkeit der Bildung des Hydrolyseproduktes (Verunreinigung D), Einfluss der Größe der thermischen Belastung auf die vorgenannten Punkte in Abhängigkeit von den Sterilisationsbedingungen, Einfluss des pH-Wertes auf in Tabelle 1 angeführte Parameter.

Als Standardzusammensetzung wird eine oxybutyninhaltige Zusammensetzung mit den in Tabelle 2 spezifizierten Mengen eingesetzt.

**Tabelle 2: Oxybutyninhaltige Zusammensetzung**

| **Rohstoff** | **Menge** |
|---|---|
| Natriumchlorid | 0,9 Gew.-% |
| 10 %-ige Salzsäure | 0,0052 Gew.-% |
| Oxybutynin-HCl | 0,1 Gew.-% |
| Wasser | 98,9948 Gew.-% |

### Verhalten der Spezifikationsparameter bei unterschiedlicher Lagerung ohne Sterilisation

Die Spezifikationsparameter von oxybutyninhaltigen Zusammensetzungen in Polypropylen-Kolbenspritzen werden bei zwei unterschiedlichen Lagerungsbedingungen dokumentiert. Dabei werden die Änderungen der Spezifikationsparameter über die Zeit von oxybutyninhaltigen Zusammensetzungen mit einem Anfangs-pH-Wert von 3,8 sowie mit einem Anfangs-pH-Wert von 4,2 ermittelt (die Anfangs-pH-Werte stellen Werte im oberen und unteren Bereich der Spezifikationsvorgaben dar). Die oxybutyninhaltigen Zusammensetzungen mit den unterschiedlichen Anfangs-pH-Werten werden jeweils bei 25 °C und 60 % relativer Luftfeuchtigkeit (RH) sowie bei 40 °C und 75 % relativer Luftfeuchtigkeit gelagert. Die Spezifikationsparameter werden nach 10 Tagen, 30 Tagen und 3 Monaten ermittelt und sind in Tabelle 3 aufgelistet.

Die nachfolgende Tabelle 3 zeigt das Verhalten der Spezifikationsparameter bei unterschiedlicher Lagerung ohne Sterilisation von oxybutyninhaltigen Zusammensetzungen in Polypropylen-Kolbenspritzen.

**Tabelle 3: Spezifikationsangaben**

| | **Bedingung** | **Dauer** | **pH** | **Oxybutynin [%]** | **Oxybutynin-Verlust [%]** | **Verunreinigung D [%]** | **weitere Verunreinigungen [%]** | **Summe der Verunreinigungen [%]** |
|---|---|---|---|---|---|---|---|---|
| **untere pH-Wert Grenze** | t0 | 0 | 3,92 | 100,9 | | n.b. | n.a | n.a |
| | 25/60 | 10 Tage | 3,93 | 100,6 | 0,3 | n.b. | 0,03 | 0,03 |
| | 40/75 | 10 Tage | 3,91 | 100,6 | 0,3 | n.b. | 0,03 | 0,03 |
| | 25160 | 30 Tage | 4 | 100,3 | 0,6 | n.b. | 0,03 | 0,03 |
| | 40/75 | 30 Tage | 4,01 | 100 | 0,9 | n.b. | 0,03 | 0,03 |
| | 40/75 | 3 Monate | - | 99,9 | 1 | 0,01 | 0,08 | 0,09 |
| **obere pH-Wert Grenze** | t0 | 0 | 4,21 | 99,7 | | n.b. | n.a | n.a |
| | 25/60 | 10 Tage | - | 99,8 | -0,1 | n.b. | 0,03 | 0,03 |
| | 40/75 | 10 Tage | - | 99,8 | -0,1 | n.b. | 0,03 | 0,03 |
| | 25/60 | 30 Tage | - | 99,2 | 0,5 | n.b. | n.a | n.a |
| | 40/75 | 30 Tage | - | 97,1 | 2,6 | 0,016 | 0,025 | 0,041 |

| | | | | | | | | |
|---|---|---|---|---|---|---|---|---|
| n.b. = nicht bestimmt | | | | | | | | |

Es zeigt sich, dass bei Lagerung der unsterilen oxybutyninhaltigen Zusammensetzung in einer Polypropylen-Kolbenspritze ohne vorangehende Sterilisation die Spezifikationsvorgaben über den gesamten Zeitraum eingehalten werden.

Weiterhin weisen die in Tabelle 3 angeführten Daten darauf hin, dass bei höheren pH-Werten und intensiveren Lagerungsbedingungen (obere pH-Wert Grenze, 40 °C, 75 % relative Luftfeuchtigkeit, 30 Tage) ein gewisser Verlust von Oxybutynin zu beobachten ist, welcher jedoch nicht mit einer Zunahme des Abbauproduktes D einhergeht.

### Verhalten der Spezifikationsparameter bei unterschiedlicher Lagerung nach Sterilisation

Darüber hinaus werden auch die Änderungen der Spezifikationsparameter nach thermischer Sterilisation einer oxybutyninhaltigen Zusammensetzung mit einem Anfangs-pH-Wert von 3,8 (Anfangs-pH-Wert im unteren Bereich der Spezifikationsvorgaben) ermittelt.

Die Spezifikationsparameter werden nach 10 Tagen, 30 Tagen und 3 Monaten ermittelt und sind in Tabelle 4 aufgelistet.

Die nachfolgende Tabelle 4 zeigt das Verhalten der Spezifikationsparameter bei unterschiedlicher Lagerung von oxybutyninhaltigen Zusammensetzungen in Polypropylen-Kolbenspritzen nach Sterilisation (im Vergleich zu den Ausgangswerten vor Sterilisation).

**Tabelle 4: Spezifikationsangaben**

| | **Bedingung** | **Dauer** | **pH** | **Oxybutynin [%]** | **Oxybutynin-Verlust [%]** | **Verunreinigung D [%]** | **weitere Verunreinigungen** [%] | **Summe der Verunreinigungen** [%] |
|---|---|---|---|---|---|---|---|---|
| **unsteril** | t0 | 0 | 3,92 | 100,9 | - | n.b. | n.b. | n.b. |
| **steril** | t0 | 0 | 3,73 | 91,1 | 9,8 | n.b. | n.b. | n.b. |
| | 25/60 | 10 Tage | 3,69 | 94,4 | 6,5 | 0,018 | 0,028 | 0,046 |
| | 40/75 | 10 Tage | 3,7 | 93,7 | 7,2 | n.b | 0,02 | 0,02 |
| | 25/60 | 30 Tage | 3,76 | 92,6 | 8,3 | 0,015 | 0,028 | 0,043 |
| | 40/75 | 30 Tage | 3,8 | 93,8 | 7,1 | 0,008 | 0,023 | 0,031 |
| | 25/60 | 3 Monate | 3,75 | 92,3 | 8,6 | 0,01 | 0,028 | 0,038 |
| | 40/75 | 3 Monate | 3,8 | 94 | 6,9 | 0,014 | 0,111 | 0,125 |

| | | | | | | | | |
|---|---|---|---|---|---|---|---|---|
| n.b. = nicht bestimmt | | | | | | | | |

Aus Tabelle 4 geht hervor, dass bei einem Anfangs-pH-Wert im unteren Bereich der Spezifikationsvorgaben (ca. 3,8) eine Reduktion bzw. ein Verlust von 9,8 % Oxybutynin nach Sterilisation zu beobachten ist. Im gleichen Maß geht ein Abfall des pH-Werts der Lösung um 0,19 Einheiten direkt nach der Sterilisation zum Zeitpunkt t0 einher. Die Lösung ist somit nicht spezifikationskonform. Bei der Lagerung über einen Zeitraum von 3 Monaten bei gestressten Bedingungen (40 °C, 75 % relative Luftfeuchtigkeit) ist ersichtlich, dass der sich Abfall der Oxybutynin-Konzentration auf ca. 7 % einpendelt. In diesem Zusammenhang wird eine Rückkehr zu einem spezifikationskonformen pH-Wert von 3,8 erreicht.

Im Vergleich zu den unsterilen Zusammensetzungen ist einen Anstieg des Hydrolyseprodukts Verunreinigung D nach 10 Tagen Lagerung bei 25 °C und 60 % relativer Luftfeuchtigkeit von 0,018 % erkennbar. Dieselbe Größenordnung wird allerdings auch bei einer unsterilen Lösung nach 30 Tagen bei 40 °C und 75 % relativer Luftfeuchtigkeit beobachtet (vgl. Tabelle 3). Hieraus kann geschlossen werden, dass die Hydrolyse ein thermisch induzierter Prozess ist, welcher jedoch insgesamt von geringerer Bedeutung ist. Wie auch aus Tabelle 3 hervorgeht, liegen auch die unbekannten Verunreinigungen auf einem Niveau zwischen steriler und unsteriler Lösung.

### Mechanismus des Verlustes an Oxybutynin nach Sterilisation

Wie zuvor bereits beschrieben und aus Tabelle 4 ersichtlich, kommt es bei der Sterilisation einer wässrigen Oxybutynin-Lösung mit einem pH-Wert von 3,92 zu einem Oxybutynin-Verlust von 9,8 %. Dieser Verlust korreliert jedoch nicht unmittelbar mit dem gleichzeitigen Anstieg der Verunreinigung D (Hydrolyseprodukt von Oxybutynin) oder anderen unbekannten Verunreinigungen.

Das Oxybutynin könnte, wie nachfolgend bestätigt, insbesondere im Rahmen der Sterilisation aus der wässrigen Lösung in das Packmittel PP (d. h. in die Polypropylen-Kolbenspritze) migrieren. Dies hätte zur Folge, dass die Reduktion bzw. der Verlust von Oxybutynin thermisch bedingt ist. Ein solcher bislang nicht beschriebener Effekt wäre für den Fachmann dabei völlig überraschend.

Die vorstehende Annahme der Migration von Oxybutynin in das Packmittel kann vor allem für den Fall bestätigt bestätigt werden, wonach
(i) eine Migration der Verunreinigung D in das Packmittel und ein somit einhergehender Verlust in der Lösung ausgeschlossen werden kann; und
(ii) die Reduktion von Oxybutynin auf einer unbekannten thermisch induzierten Abbaureaktion beruht, dessen Abbauprodukte mit der validierten HPLC-Methode nicht nachgewiesen werden können, ausgeschlossen werden kann.

Um auszuschließen, dass eine thermisch induzierte Abbaureaktion von Oxybutynin-HCl zur Bildung eines unbekannten Nebenproduktes, welches nicht mittels validierter HPLC-Methode zu detektieren ist, oder dass eine Migration des Hydrolyseprodukts D stattfindet, wird das verwendete Packmittel (Polypropylen-Zylinder und Gummistopfen) nach der Sterilisation einer Extraktion mit THF unterzogen und der resultierende Extrakt wird sowohl qualitativ mittels Dünnschichtchromatographie als auch quantitativ mittels HPLC untersucht.

Des Weiteren wird der Extrakt einer wässrigen Mikroaufarbeitung mit Diethylether bei pH 4 unterzogen, um nachzuweisen, dass Oxybutynin bei pH 4 als freie Aminbase (Strukturformel (1)) in der organischen Phase (Diethylether) oder als Hydrochlorid in der wässrigen Phase vorliegt.

Die Gehaltsergebnisse nach Sterilisation zeigen, dass es zu einem Abfall von Oxybutynin in der Lösung von bis zu 9 % bei einem pH-Wert von 3,8 kommt. Es kann davon ausgegangen werden, dass bei pH 4 der Großteil von Oxybutynin in der Diethyletherphase als freie Aminbase vorliegt.

Es werden die Extrakte der Packmittel mit den jeweiligen analytischen Referenzsubstanzen von Oxybutynin und Verunreinigung D auf der Dünnschichtchromatographie- bzw. DC-Platte mit dem Laufmittel Cyclohexan/Essigester 1:6 analysiert.

Aus weiterführenden Versuchen ergibt sich, dass im Extrakt der Gummiteile kein Oxybutynin enthalten ist, während im Extrakt des Polypropylen-Zylinders Oxybutynin enthalten ist.

Durch eine saure, wässrige Aufarbeitung des Extraktes des Polypropylen-Zylinders kann qualitativ nachgewiesen werden, dass Oxybutynin in der organischen Diethyletherphase vorliegt, aber nicht in der wässrigen Phase.

In diesem Zusammenhang werden die Proben der Mikroaufarbeitung auch mittels HPLC-Analyse analysiert, wodurch folgende qualitative Nachweise erfolgen können:
- Migration von Oxybutynin in das Packmittel
- Affinität der Migration in den Polypropylen-Zylinder im Vergleich zu dem Butylkautschuk der Gummiteile
- Vergleich mit einem Blankomaterial (nicht migriert)
- Ausschluss der Migration der Verunreinigung D in den Polypropylen-Zylinder und die Gummiteile

### Berechnung der maximal nachweisbaren Konzentration von Oxybutynin in den Proben

Wie zuvor ausgeführt, sind direkt nach der Sterilisation 9,8 % des Oxybutynins nicht mehr wiederzufinden bzw. nicht mehr in der oxybutyninhaltigen Zusammensetzung nachweisbar, wobei eine durchschnittliche Migrationsrate über sämtliche vorliegend verwendete Packmittel von 7,8 % berechnet werden kann.

Bei einer Oxybutynin-Konzentration von 0,1 % migrieren demnach 0,78 mg/Spritze in das Packmittel. Bei vier verwendeten Packmitteln entspricht dies einer Masse von etwa 3,1 mg, welche bei quantitativer Aufarbeitung wiederzufinden sind (vgl. Tabelle 5).

**Tabelle 5: Berechnung der theoretisch migrierten Menge Oxybutynin anhand der verbleibenden Konzentration in der Lösung nach Sterilisation**

| | |
|---|---|
| Konzentration der Lösung | 0,1 % |
| Volumen der Lösung | 10 ml |
| Oxybutynin/Spritze | 10 mg |
| durchschnittliche Migrationsrate | 7,84 % |
| migrierte Menge/Spritze | 0,78 mg |
| Menge in 4 Spritzen bei quantitativem Nachweis | 3,1 mg |

Es werden folgende Proben mittels HPLC analysiert:
- Gummiteile (wässrige, organische Phase)
- Polypropylen-Zylinder (wässrige, organische Phase)
- Blanko Gummiteile (wässrige, organische Phase)
- Blanko Polypropylen-Zylinder (wässrige, organische Phase)

In Tabelle 6 wird die Oxybutynin-Menge in den vermessenen Proben aufgelistet (es wird jeweils keine Verunreinigung D gefunden).

**Tabelle 6: HPLC Ergebnisse der Extraktionsversuche der Gummiteile sowie Polypropylen-Zylinder**

| | **Oxybutynin [µg/ml]** | **weitere Werte** |
|---|---|---|
| Gummiteil organische Phase Blanko | n.b. | |
| Gummiteil organische Phase | 8,71 | |
| Gummiteil wässrige Phase Blanko | n.b. | |
| Gummiteil wässrige Phase | 2,71 | |
| Zylinder organische Phase Blanko | n.b. | |
| Zylinder organische Phase | 71,78 | |
| Zylinder wässrige Phase Blanko | n.b. | |
| Zylinder wässrige Phase | 13,39 | |
| Summe Gummiteil | 11,43 | |
| Summe Zylinder | 85,17 | |
| Summe Packmittel | **96,59** | |
| Menge/Gummiteil | 2,86 | |
| Menge/Zylinder | 21,29 | |
| Menge/Spritze | 24,15 | |
| Verhältnis von Oxybutynin Zylinder / Oxybutynin Gummiteil (wässrige Phase) | | 4,94 |
| Verhältnis von Oxybutynin Zylinder / Oxybutynin Gummiteil (organische Phase) | | 8,24 |
| Verhältnis von Oxybutynin Zylinder / Oxybutynin Gummiteil (Summe) | | 7,45 |
| Oxybutynin in HPLC Probe | 96,59 | |
| Faktor Probenverdünnung | | 10 |
| Oxybutynin in Extraktionsprobe | | 965,92 µg |
| Oxybutynin/Spritze | | 0,24 mg |
| | | |
| Migration Gummiteil | | 11,83 % |
| Migration Zylinder | | 88,17 % |
| Migration wässrig Zylinder | | 15,72 % |
| Migration organisch Zylinder | | 84,28 % |

Die Verunreinigung D kann weder in der wässrigen noch in der organischen Phase nachgewiesen werden, so dass die Substanz weder in den Zylinder noch in die Gummiteile migriert, wie auch durch die Dünnschichtchromatographie bestätigt. Dieser Befund bestätigt nochmals die Annahme, dass die Hydrolyse von Oxybutynin und eine folgende Migration von Phenylcyclohexylhydroxyessigsäure (Verunreinigung D bzw. das Hydrolyseprodukt von Oxybutynin) nicht für den Rückgang der Oxybutynin-Konzentration verantwortlich sind.

Der Vergleich der wässrigen und organischen Proben der Mikroaufarbeitung bestätigen den qualitativen Befund der Dünnschichtchromatographie, welcher zeigt, dass Oxybutynin hauptsächlich (84 %) in den organischen Proben nachzuweisen ist. Der Wert für die wässrige Aufarbeitung der Zylinder liegt bei 13,4 µg/ml im Vergleich zur organischen Aufarbeiten von 71,8 µg/ml. Dies untermauert die Aussage, dass Oxybutynin bei pH 4 größtenteils als neutrale Aminbase vorliegt und somit in Diethylether löslich ist (84 %). Die Mengen von Oxybutynin werden aus den wässrigen und organischen Proben addiert, um die Gesamtmenge an Oxybutynin nach der Festphasenextraktion zu bestimmen. Oxybutynin migriert zu 88 % in das Zylindermaterial, nur 12 % migrieren in die Gummiteile.

Da die Versuche nicht mit exakten Mengen an Lösungsmittel und nicht unter Verwendung eines externen Standards durchgeführt wurden, sind im Vergleich nur 30 % der migrierten Oxybutyninmenge gefunden worden. Ebenso kann die geringe Wiederfindungsrate durch eine möglicherweise unvollständige Festphasenextraktion mit THF erklärt werden. Die Resultate der HPLC Messungen sind in der aus den Stabilitätseinlagerungsversuchen erwarteten Bereichen. Aus Tabelle 6 ist ersichtlich, dass 965,92 µg aus vier Packmittel nach Probenvorbereitung nachgewiesen werden, was einer Menge von 0,24 mg/Spritze entspricht.

Die Löslichkeit von Oxybutynin in wässriger Umgebung ist eingeschränkt, so dass die Substanz in Form ihres Hydrochlorids in wässrigen Systemen zum Einsatz kommt. Sowohl die qualitativen Ergebnisse der Dünnschichtchromatographie als auch die quantitativen Werte der HPLC zeigen, dass es zu einer Migration von Oxybutynin in Polypropylen nach thermischer Belastung kommt. Oxybutynin migriert in seiner apolaren Form in das Packmittel und kann durch Extraktion mittels THF wieder als freie Aminbase nachgewiesen werden.

Folglich wird angenommen, dass bei steigenden pH-Werten in der wässrigen Lösung Oxybutynin in Form seiner freien, unkomplexierten Form vorliegt, welche in das Packmittel migriert. Bei verringerten, stark sauren pH-Werten ist Oxybutynin durch das Hydrochlorid komplexiert und wird dann durch seine Polarität gehemmt, in Polypropylen zu migrieren. Somit liegt eine Erhöhung der Migrationsrate von Oxybutynin nach Sterilisation mit steigendem pH-Wert der Ausgangslösung sowie eine Reduktion der Migrationsrate nach Sterilisation bei Absenken des pH-Werts vor. Des Weiteren kann bei Migration der unkomplexierten freien Aminbase ein Abfall des pH-Wertes in der Lösung nach Sterilisation zu beobachten werden, da das Hydrochlorid als freie Säure vorliegt.

Diese Annahmen werden durch einen Abfall der Oxybutynin-Konzentration von 9,8 % und einer pH-Wert Reduktion um 0,19 phänomenologisch unterstützt (vgl. Tabelle 4).

### Auswirkungen des Anfanqs-pH-Wertes auf den Oxybutynin-Verlust

Um die zuvor dargestellte pH-Wert abhängige Migration zu verifizieren, wird die Menge an HCl in der oxybutyninhaltigen Zusammensetzung (vgl. Tabelle 2) derart angepasst, dass pH-Werte von pH 3 und pH 5 erreicht werden. Um den Oxybutynin-Verlust nach Sterilisation bei pH 5 auszugleichen wird Oxybutynin mit einer Konzentration von 105 % eingesetzt. Die Lösungen werden in einer Polypropylen-Kolbenspritze sterilisiert und Spezifikationsparameter nach thermischen Eintrag ermittelt (vgl. Tabelle 7).

Die nachfolgende Tabelle 7 zeigt das Verhalten der Spezifikationsparameter von oxybutyninhaltigen Zusammensetzungen in Polypropylen-Kolbenspritzen für unterschiedliche Anfangs-pH-Werte (pH 3 bzw. pH 5) nach Sterilisation (im Vergleich zu den Ausgangswerten vor Sterilisation).

**Tabelle 7: Spezifikationsparameter bei pH 3 und pH 5 vor und nach Sterilisation**

| | **Bedingung** | **Dauer** | **pH** | **Oxybutynin [%]** | **Oxybutynin-Verlust [%]** | **Verunreinigung D [%]** | **weitere Verunreinigungen [%]** | **Summe der Verunreinigungen [%]** |
|---|---|---|---|---|---|---|---|---|
| **100 % Oxybutynin, pH 3, unsteril** | t0 | 0 | 2,86 | 99,5 | - | 0,008 | 0 | 0,084 |
| **100 % Oxybutynin, pH 3, steril** | t0 | 0 | 2,82 | 100,3 | -0,8 | 0,03 | 0 | 0,034 |
| | 25/60 | 10 Tage | 2,84 | 98,9 | 0,6 | 0,05 | 0 | 0,089 |
| | 40/75 | 10 Tage | 2,86 | 98,9 | 0,6 | 0,058 | 0 | 0,094 |
| | 25/60 | 30 Tage | 2,82 | 98 | 1,5 | 0,023 | 0 | 0,023 |
| | 40/75 | 30 Tage | 2,81 | 98,8 | 0,7 | 0,063 | 0 | 0,063 |
| | 25/60 | 3 Monate | 2,84 | 99 | 0,5 | 0,025 | 0 | 0,031 |
| | 40/75 | 3 Monate | 2,86 | 99,2 | 0,3 | 0,029 | 0,16 | 0,189 |
| **105 % Oxybutynin, pH 5, unsteril** | t0 | 0 | 5,2 | 103,9 | - | 0,01 | 0 | 0,01 |
| **105 % Oxybutynin, pH 5, steril** | t0 | 0 | 3,8 | 95,9 | 8 | 0,06 | 0 | 0,06 |

Aus Tabelle 7 ist ersichtlich, dass ein Ausgangs-pH-Wert von pH 3 zu spezifikationskonformen Ergebnissen nach Sterilisation führt und die Migrationsrate von 9,8 % (vgl. Tabelle 4) auf 0 % gesenkt werden kann. Darüber hinaus kommt es nach Sterilisation lediglich zu einem Abfall des pH-Wertes von 0,04 (d.h. um 0,15 im Vergleich zu den Ergebnissen bei einem Start pH-Wert von 3,92; vgl. Tabelle 4). Auch diese Ergebnisse unterstützen die Annahme, dass bei geringeren pH-Werten Oxybutynin hauptsächlich in seiner polaren Form vorliegt und so eine Migration in Polypropylen überraschend reduziert werden kann, wodurch gleichermaßen der pH-Wert nach Sterilisation nur wenig reduziert ist. Nach drei Monaten Lagerung bei erschwerten Bedingungen (40/75) liegt eine maximale Migrationsrate von 0,3 % sowie ein stabiler pH-Wert von ca. 2,8 vor.

Des Weiteren wird gezeigt, dass die Erhöhung des Ausgangs-pH-Werts auf pH 5 zwar einen erhöhten Verlust von Oxybutynin (8 %) zur Folge hat, dieser Verlust jedoch überraschend mit der Aufstockung der Oxybutynin-Konzentration auf 105 % ausgeglichen werden kann. Hier können nach Sterilisation spezifikationskonforme Werte von bis zu 95,9 % Oxybutynin und ein pH-Wert von 3,8 erzielt werden.

Die Versuche werden mit weiteren unterschiedlichen Ausgangs-pH-Werten wiederholt (Einstellung des pH-Werts durch Anpassung der HCl-Konzentration). In diesem Zusammenhang werden pH-Werte im Bereich von 2,86 bis 5,2 betrachtet und die Abhängigkeit zum pH-Wert Abfall nach Sterilisation dokumentiert (vgl. Fig. 3). Diese Abhängigkeit kann in einer nichtlineare Korrelation abgebildet werden, welche sich mittels eines Polynoms zweiten Grades und einem Bestimmtheitsmaß von R² = 0,9799 (y = 0,2898x² - 1,7344x + 2,6217) beschreiben lässt. Auf Grundlage dieser Funktion können nun weitere pH-Verluste interpoliert sowie exemplarisch pH-Werte extrapoliert werden. Der Verlauf des Graphen zeigt einen quadratischen ansteigenden pH-Wert Verlust bei steigendem Anfangs-pH-Wert. Daraus kann abgeleitet werden, dass bei einer pH-Wert-Spezifikationsvorgabe von 3,5 bis 4,2 ein maximaler Anfangs-pH-Wert von 5,2 und ein minimaler Anfangs-pH-Wert von 4,2 eingesetzt werden sollte.

In Fig. 4 ist die Abhängigkeit des Oxybutynin-Verlusts nach Sterilisation vom Ausgangs-pH-Wert des Lösung visualisiert. Außerdem ist erkennbar, dass die beobachtete Migrationsrate einen linearen Trend im pH-Wert Bereich von 2,86 bis 5,2 aufweist. Die daraus abgeleitete lineare Korrelation zeigt ein Bestimmtheitsmaß von R² = 0,8078 (y = 3,4196x - 8,1923) und verdeutlicht, dass im pH-Bereich von 3,8 bis 4,2 ein durchschnittlicher Oxybutynin-Verlust von 5 % nach thermischem Eintrag zu erwarten ist. Die Migration kann bei pH-Werten von 3 im Wesentlichen unterdrückt werden und steigt bei pH Werten über 5 auf einen Migrationsverlust auf etwa 8 % an.

### Auswirkung der Oxybutynin-Konzentration auf die weiteren Spezifizierungsparameter

Durch die Einstellung des pH-Werts der oxybutyninhaltigen Zusammensetzung vor Sterilisation zu sauren Bedingungen (pH-Wert von ca. 3) können die Spezifikationsvorgaben durch Unterdrückung bzw. Verringerung der Migration erreicht werden (vgl. Fig. 4). Jedoch ist die physiologische Anwendbarkeit von derart sauren Lösungen (pH ca. 3) mitunter nicht gegeben. Der Verlust von Oxybutynin nach Sterilisation innerhalb des spezifizierten pH-Wert Bereichs kann aber in überraschender Weise durch Verwendung höherer Startkonzentrationen von Oxybutynin ausgeglichen werden.

Außerdem zeigen die Versuchsreihen, dass eine Erhöhung des Anfangs-pH-Werts eine Zunahme der Migrationsrate zur Folge hat (vgl. Fig. 4). Bei einem Anfangs-pH-Wert von 5 kommt es zu einer Migration von 8 %, wohingegen sehr saure Lösungen die Migration inhibieren. Im pH-Bereich zwischen 3,8 und 4,2 findet eine durchschnittliche Migration von 5,5 % statt. Unter Verwendung der linearen Korrelation lassen sich Grenzwerte zur Definition einer möglichen Überdosierung bzw. Übereinwaage in Abhängigkeit vom Ausgangs-pH-Wert ableiten. Von einem unteren pH-Wert von 2,86 ausgehend ist es möglich, bis zu einem pH-Wert von 3,8 ohne Überdosierung den Oxybutyningehalt spezifikationskonform zu halten (100 % bis 105 % Oxybutynin). Ab einem pH-Wert von 3,8 kann mit mindestens 5 % Überdosierung gearbeitet werden (Grenzwertschwelle). Hier kann mit einer Oxybutynin-Konzentration von 105 % bis 110 % gearbeitet werden. Folglich kann durch die Einstellung des pH-Wertes ein spezifikationskonformer Gehalt an Oxybutynin nach Sterilisation erhalten werden.

Weitere Versuchsreihen, bei denen 105 % bzw. 110 % Oxybutynin eingesetzt werden (vgl. Tabelle 8), zeigen, dass eine Spezifikationskonformität des Oxybutyningehalts nach Sterilisation realisiert werden kann, jedoch der pH-Wert außerhalb der spezifizierten Werte im Bereich von 3,8 bis 4,2 liegt. Eine Erhöhung des Oxybutyningehalts auf 105 % ohne Anpassung der HCl-Menge hat einen Anfangs-pH-Wert der unsterilisierten Lösung von 3,63 zur Folge, welcher nach der Sterilisation auf 3,45 absinkt. Die Migration von 4,6 % kann hier durch die Überdosierung ausgeglichen werden. Durch die Erhöhung auf 110 % Oxybutynin und die Anpassung des pH-Werts mittels HCl-Dosierung, kann ein Start pH-Wert von 3,99 eingestellt werden. Nach der Sterilisation fällt der Oxybutyningehalt auf 99,9 % ab. Gleichzeitig sinkt der pH Wert auf 3,6. Die Erhöhung des Oxybutyningehalts auf 115 % mit gleichzeitiger Einstellung des pH-Werts auf 4,2 führt zu einer Migrationsrate von 6,6 % und zu einem Abfall des pH-Werts auf 3,7.

Die nachfolgende Tabelle 8 zeigt das Verhalten der Spezifikationsparameter von oxybutyninhaltigen Zusammensetzungen in Polypropylen-Kolbenspritzen für unterschiedliche Oxybutynin-Anfangsgehalte (bei einem Anfangs-pH-Wert von pH 3) nach Sterilisation (im Vergleich zu den Ausgangswerten vor Sterilisation).

**Tabelle 8: Spezifikationsparameter bei pH 3 und unterschiedlichem Oxybutynin-Anfangsgehalt**

| **Beschreibung** | **Bedingung** | **Dauer** | **pH** | **Oxybutynin** | **Oxybutynin-Verlust [%]** | **Verunreinigung D [%]** | **weitere Verunreinigungen [%]** | **Summe der Verunreinigungen [%]** |
|---|---|---|---|---|---|---|---|---|
| **pH 3, unsteril** | t0 | 0 | 2,86 | 99,5 | - | 0,008 | 0 | 0,084 |
| **pH 3**, **steril** | t0 | 0 | 2,82 | 100,3 | -0,8 | 0,03 | 0 | 0,034 |
| | 25/60 | 10 Tage | 2,84 | 98,9 | 0,6 | 0,05 | 0 | 0,089 |
| | 40/75 | 10 Tage | 2,86 | 98,9 | 0,6 | 0,058 | 0 | 0,094 |
| | 25/60 | 30 Tage | 2,82 | 98 | 1,5 | 0,023 | 0 | 0,023 |
| | 40/75 | 30 Tage | 2,81 | 98,8 | 0,7 | 0,063 | 0 | 0,063 |
| | 25/60 | 3 Monate | 2,84 | 99 | 0,5 | 0,025 | 0 | 0,031 |
| | 40/75 | 3 Monate | 2,86 | 99,2 | 0,3 | 0,029 | 0,16 | 0,189 |
| **105 % Oxybutynin, unsteril** | t0 | 0 | 3,63 | 104,4 | - | 0,008 | 0 | 0,103 |
| **105 % Oxybutynin, steril** | t0 | 0 | 3,45 | 99,8 | 4,6 | 0,028 | 0 | 0,036 |
| | 25/60 | 10 Tage | 3,49 | 100,4 | 4 | 0,054 | 0 | 0,09 |
| | 40/75 | 10 Tage | 3,5 | 100,6 | 3,8 | 0,039 | 0 | 0,064 |
| | 25/60 | 30 Tage | 3,42 | 100,1 | 4,3 | 0,024 | 0 | 0,024 |
| | 40/75 | 30 Tage | 3,47 | 99,2 | 5,2 | 0,016 | 0 | 0,016 |
| | 25/60 | 3 Monate | 3,54 | 99,8 | 4,6 | 0,016 | 0 | 0,021 |
| | 40/75 | 3 Monate | 3,51 | 99,2 | 5,2 | 0,024 | 0,17 | 0,2 |
| **110 % Oxybutynin, unsteril** | t0 | 0 | 3,99 | 110 | - | 0,008 | 0 | 0,093 |
| **110 % Oxybutynin, steril** | t0 | 0 | 3,6 | 99,9 | 10,1 | 0,028 | 0 | 0,034 |
| | 25/60 | 10 Tage | 3,62 | 103,2 | 6,8 | 0,05 | 0 | 0,075 |
| | 40/75 | 10 Tage | 3,61 | 103,5 | 6,5 | 0,043 | 0 | 0,068 |
| | 25/60 | 30 Tage | 3,6 | 102,1 | 7,9 | 0,032 | 0 | 0,032 |
| | 40/75 | 30 Tage | 3,59 | 102,8 | 7,2 | 0,024 | 0 | 0,024 |
| | 25/60 | 3 Monate | 3,61 | 104 | 6 | 0,015 | 0 | 0,02 |
| | 40/75 | 3 Monate | 3,72 | 102,3 | 7,7 | 0,015 | 0,167 | 0,181 |
| **115 % Oxybutynin, unsteril** | t0 | 0 | 4,2 | 115 | - | 0,02 | - | 0,045 |
| **115 % Oxybutynin, steril** | t0 | 0 | 3,7 | 108,4 | 6,6 | 0,0055 | - | 0,078 |

Aus den Versuchen geht hervor, dass die prozentuale Menge des überraschend migrierten Oxybutynins in gleichermaßen überraschender Weise durch höhere Startkonzentrationen ausgeglichen werden kann, wobei zudem der Abfall des pH-Werts auch ein kritischer Prozessparameter ist, welcher - gleichermaßen überraschend - durch eine Kombination aus pH-Wert-Anpassungen und Konzentrationserhöhung des Oxybutynins kontrolliert bzw. kompensiert werden kann.

### Anpassung des pH-Werts und der Oxybutynin-Konzentration

Wie zuvor ausgeführt, kann die Migration von Oxybutynin in das Packmittel Polypropylen nach Sterilisation durch die Erhöhung der Oxybutynin-Konzentration überraschend ausgeglichen werden, gegebenenfalls können dadurch nicht die pH-Wert Grenzen eingehalten werden (vgl. Tabelle 8).

Wie zuvor bereits ausgeführt und in Fig. 3 dargestellt, liegt ein überraschend aufgefundener Zusammenhang zwischen dem Ausgangs-pH-Wert der oxybutyninhaltigen Zusammensetzung und der pH-Wert Reduktion nach Sterilisation vor. Bei einem Anfangs-pH-Wert von 4,5 kommt es zu einer pH-Wert-Reduktion von 0,75, wodurch ein pH-Wert nach Sterilisation von ca. 3,75 vorliegt. Folglich kann durch eine Kombination aus erhöhter Oxybutynin-Konzentration und Anpassung des Anfangs-pH-Werts ein pH-Wert nach Sterilisation von 3,7 erreicht werden. Insbesondere kann der Ausgangs-pH-Wert vor Sterilisation auf 4,5 eingestellt werden (insbesondere bei einer Oxybutynin-Konzentration von 105 %). Hierdurch können auch mitunter nicht-spezifikationskonforme pH-Werte beispielsweise im großtechnischen Maßstab vermieden werden, was auch mit einer optimalen Prozessführung einhergeht.

Die nachfolgende Tabelle 9 zeigt das Verhalten der Spezifikationsparameter von oxybutyninhaltigen Zusammensetzungen in Polypropylen-Kolbenspritzen für einen Oxybutynin-Anfangsgehalt von 105 % (bei einem Anfangs-pH-Wert von pH 4,5) nach Sterilisation (im Vergleich zu den Ausgangswerten vor Sterilisation).

**Tabelle 9: Spezifikationsparameter bei pH 4,5 und 105 % Oxybutynin**

| **Beschreibung** | **Bedingung** | **Dauer** | **pH** | **Oxybutynin [%]** | **Oxybutynin-Verlust [%]** | **Verunreinigung D [%]** | **weitere Verunreinigungen [%]** | **Summe der Verunreinigungen [%]** |
|---|---|---|---|---|---|---|---|---|
| 105 % Oxybutynin, pH 4,5, unsteril | t0 | 0 | 4,5 | 104,2 | - | 0,01 | 0 | 0,01 |
| 105 % Oxybutynin, pH 4,5, steril | t0 | 0 | 3,7 | 96,9 | 7,3 | 0,05 | 0 | 0,05 |
| | 25/60 | 10 Tage | 3,8 | 99,5 | 4,7 | 0,04 | 0 | 0,05 |
| | 40/75 | 10 Tage | 3,84 | 97,6 | 6,6 | 0,04 | 0 | 0,04 |

Bei Verwendung von 105 % Oxybutynin bei einem Anfangs-pH-Wert von 4,5 liegt eine Migration von 7,3 % vor, und folglich liegen sowohl der Oxybutyningehalt nach Sterilisation als auch der pH-Wert innerhalb der Spezifikationsvorgaben.

### Einfluss der Sterilisationsparameter

Wie zuvor bereits beschrieben, ist die Migration von Oxybutynin in das Packmittel Polypropylen insbesondere thermisch induziert. Die durchschnittliche Migrationsrate nach thermischer Belastung durch Autoklavierung liegt bei 5 %. Dieser Zusammenhang geht gleichermaßen auch aus den Stabilitätsdaten unsteriler Zusammensetzungen hervor (vgl. Tabelle 3): es erfolgt bereits eine Migration von 2,6 % über einen Zeitraum von 3 Monaten bei 40 °C und 75 % relative Luftfeuchtigkeit.

Um den Einfluss der gewählten Sterilisationsparameter auf die Migrationsrate zu ermitteln, werden zwei unterschiedliche Sterilisationsprogramme (A bzw. B) getestet und die relevanten Spezifikationsparameter nach thermischem Eintrag ermittelt (vgl. Tabelle 10).

Die Sterilisationsprogramme A und B unterscheiden sich in der thermischen Belastung, die auf die Probe ausgeübt wird. Als Maß zur Bestimmung der thermischen Belastung dient hierbei der F0-Wert, welcher die Äquivalenzbelastung des Sterilisationsprogramm im Vergleich zu einer Sterilisation bei einer Referenztemperatur (121,1 °C) darstellt und in min angegeben wird. Er dient dazu, sämtliche letalen Effekte eines Sterilisationsprogramms (Aufheizen, Sterilisieren, Abkühlen) über den Verlauf des Programms aufzusummieren und so eine Vergleichbarkeit zu ermöglichen. Ein F0-Wert von 1 min entspricht folglich einer thermischen Belastung, welche äquivalent zu 1 min bei 121,1 °C ist.

Das Sterilisationsprogramm A erzielt FO-Werte im Bereich von 19 bis 23 min, bei einer effektiven Sterilisationshaltezeit von mindestens 6 min bei 121,5 °C. Sterilisationsprogramm B weist dabei zusätzlich eine Anpassung zur Entsprechung von EP 5.1.1 (European Pharmacopoeia) bei typischen FO-Werte von 28 bis 31 min und einer Sterilisationshaltezeit von mindestens 20 min bei 121,5 °C.

Die Sterilisationsprogramm weisen einen deutlichen Unterschied in ihrer Länge der Plateauphase (Sterilisationszeit) auf, welcher der thermischen Belastung bei < 121 °C entspricht. Es wird ein höherer Verlust an Oxybutynin nach der Sterilisation mit Programm B erwartet.

Tabelle 10 zeigt die Reduktion des pH-Werts und des Oxybutyningehalts an drei verschiedenen Versuchsreihen, wobei die drei Versuchsreihen sich in der Oxybutynin-Konzentration (Standardrezeptur bzw. 105 %) und dem Anfangs-pH-Wert (3,9 bis 4,5) unterscheiden.

**Tabelle 10: Vergleich der Spezifikationsparameter nach den Sterilisationsprogrammen A und B**

| **Beschreibung** | **Sterilisationsprogramm** | **pH** | **Oxybutynin [%]** | **Oxybutynin-Verlust [%]** |
|---|---|---|---|---|
| **Standardrezeptur** | unsteril | 3,92 | 100,9 | - |
| **Standardrezeptur** | A | 3,73 | 91,1 | 9,8 |
| **Standardrezeptur** | B | 3,68 | 94,1 | 6,8 |
| **Standardrezeptur** | unsteril | 4,21 | 99,7 | |
| **Standardrezeptur** | A | 3,8 | 92,3 | 7,4 |
| **Standardrezeptur** | B | 3,8 | 93,5 | 6,2 |
| **105 % Oxybutynin, pH 4,5** | unsteril | 4,5 | 104,2 | - |
| **105 % Oxybutynin, pH 4,5** | A | 3,8 | 97 | 7,2 |
| **105 % Oxybutynin, pH 4,5** | B | 3,7 | 96,9 | 7,3 |

Die Tabelle zeigt, dass für sämtliche Zusammensetzungen mit 105 % Oxybutynin und einem pH-Wert von 4,5 spezifikationskonforme Werte erhalten werden. In diesem Zusammenhang ermöglichen beide Sterilisationsprogramme eine effektive Sterilisation, welche in spezifikationskonformen Zusammensetzungen, wie zuvor angeführt, resultiert. Zudem kann kein signifikanter Einfluss der erhöhten FO-Werte und der längeren Sterilisationshaltezeit auf die Reduktion des Oxybutyningehalts beobachtet werden. Weder für den pH-Wert noch für den Verlust des Oxybutynins liegen nennenswerte Unterschiede zwischen beiden Sterilisationsprogrammen vor (vgl. Tabelle 10). Lediglich bei der Zusammensetzung mit einem Anfangs-pH-Wert von 3,92 liegen geringfügig höhere Abweichung im Oxybutynin-Verlust (9,8 %) vor, welche bei der anspruchsvolleren Sterilisation (Sterilisationsprogramm B) sogar niedriger ausfällt (6,8 %), so dass nicht auf eine erhöhte Produktbelastung geschlossen werden kann. Bei einer Anfangskonzentration von 105 % und einem pH-Wert von 4,5 liegen die Migrationswerte bei 7,3 % bzw. 7,2 %. Aus den Ergebnissen kann abgeleitet werden, dass FO-Werte im Bereich von 19 bis 31 min angewandt werden können ohne die Migrationsrate des Oxybutynin sowie den pH Verlust signifikant zu beeinflussen.

### Zusammenfassung der vorangehenden Ergebnisse

Der Gehalt an Oxybutynin in einer wässrigen 0,1 %igen Oxybutyninlösung fällt nach terminaler Dampfsterilisation in einer Polypropylen-Kolbenspritze ab. Weiterhin sinkt der pH-Wert der Lösung nach thermischer Belastung. Durch Extraktionsversuche des verwendeten Polypropylen-Packmittels und nachfolgender qualitativer Analyse mittels Dünnschichtchromatographie sowie quantitativer Analyse mittels HPLC kann nachgewiesen werden, dass der Abfall der Oxybutyninkonzentration im Wesentlichen nicht bzw. nicht vorrangig durch Hydrolyse des Oxybutynins oder durch Bildung einer unbekannten Verunreinigung resultiert, sondern auf eine Migration von Oxybutynin in unkomplexierter Form (d. h. als freie Aminbase) zurückzuführen ist. Dieser Prozess ist insbesondere thermisch induziert.

Darüber hinaus führt eine terminale Dampfsterilisation mit F0-Werten im Bereich von 19 min bis 31 min zu vergleichbaren Migrationsraten von Oxybutynin sowie pH-Wert Verlusten, so dass diese gleichermaßen verwendet werden können.

Des Weiteren kann die Migration von Oxybutynin über den pH-Wert der Lösung vor der Sterilisation gesteuert werden - geringere pH-Werte führen zu einer reduzierten Migrationen. Die Migrationsrate bei höheren pH-Werten kann durch eine Erhöhung der Oxybutynin-Konzentration in der Lösung vor Sterilisation ausgeglichen werden.

Die gefundene Relation zwischen Anfangs-pH-Wert und pH-Wert nach Sterilisation kann mit einer Polynomfunktion zweiten Grades beschrieben werden. In Kombination mit der Überdosierung können pH-Werte ermittelt werden, welche zu spezifikationskonformen Ergebnissen führen.

Aus den vorliegenden Versuchen können somit die in Tabelle 11 aufgelisteten pH-Wert- und Oxybutynin-Konzentrationskorridore aufgezeigt werden (A-E). Die Spezifikationsvorgabe des Oxybutyningehalts von 95 bis 105 % wird in allen Kombinationen (A-E) eingehalten. Für die Kombinationen A und B bei sauren pH-Werten kann die sterilisierte Lösung mit einer pH-Wert Spezifikation ab 2,8 erreicht werden (physiologische Aspekte werden in diesem Fall nicht betrachtet). Die Kombination C und D sind spezifikationskonform und weisen finale pH-Werte von 3,5 bis 4,2 auf.

Die Kombination E als eine optimale Kombination basiert auf einer Überdosierung von Oxybutynin von 105 % sowie einem Ausgangs-pH-Wert von 4,5, woraus eine finale produktionstechnisch abbildbare pH-Wert Spezifikation im Bereich von 3,5 bis 4,2 erreicht wird.

Die nachfolgende Tabelle 11 zeigt zusammenfassend die aufgezeigten pH-Wert- und Oxybutynin-Konzentrationskorridore.

**Tabelle 11: Korridore für pH-Werte und Oxybutyninkonzentrationen**

| **Kombination** | **pH-Wert vor Sterilisation** | **Oxybutynin-Konzentration vor Sterilisation [%]** | **pH-Verlust nach Sterilisation** | **Oxybutynin-Verlust nach Sterilisation [%]** | **pH-Wert nach Sterilisation** | **Oxybutynin-Konzentration nach Sterilisation [%]** | **Spezifikation pH-Wert nach Sterilisation** |
|---|---|---|---|---|---|---|---|
| **A** | 2,9 - 3,8 | 100 | 0,02 - 0,22 | 1.7 - 4,8 | 2,88 - 3,58 | 95,2 - 98,3 | 2,8 - 4,2 |
| **B** | 2,9 - 3,8 | 105 | 0,02 - 0,22 | 1,7 -4,8 | 2,88 - 3,58 | 100,2 - 103,2 | 2,8 - 4,2 |
| **C** | 3,8 - 5,2 | 105 | 0,22 - 1,44 | 4,8 - 9,6 | 3,58 - 3,76 | 95,4 - 100,2 | 3,5 - 4,2 |
| **D** | 3,9 - 5,2 | 110 | 0,27 **-** 1,44 | 5,1 - 9,6 | 3,53 - 3,76 | 100,4 - 104,9 | 3,5 - 4,2 |
| **E** | 4,5 | I 105 | 0,69 | 7,2 | 3,81 | 97,8 | 3,5 - 4,2 |

Die vorangehend zusammenfassend angeführten überraschenden Zusammenhänge konnten von der Anmelderin im Rahmen der vorgenannten Versuchsreihen ermittelt werden. Diesbezüglich ist es im Rahmen der vorliegenden Erfindung völlig überraschend gelungen, ein effizientes Konzept zur Bereitstellung steriler oxybutyninhaltiger Zusammensetzungen aufzuzeigen, anhand dessen insbesondere Zusammensetzungen mit definiertem Wirkstoffgehalt und pH-Wert bei gleichzeitig geringer Verunreinigungsmenge erhalten werden. Auf dieser Basis kann somit ein effizientes wie auch ökonomisches Verfahren bereitgestellt werden.

### Experimentelle Durchführung

### Verwendete Materialien

### Dünnschichtchromatogramme

Dünnschichtchromatogramme werden auf mit Kieselgel 60 F254 beschichteten Aluminiumplatten durchgeführt. Deren Detektion erfolgt mit KMNO₄ Lösung und anschließendem Erwärmen auf einer Heizplatte.

### HPLC Freigabeanalytik und Stabilitätsuntersuchungen

| **Gerät** | HPLC System (Pumpe, Probengeber, DAD Detektor, Säulenofen) | | |
|---|---|---|---|
| **Detektion** | 210 nm | | |
| | DAD-Spektrum (200-350 nm) | | |
| **Stationäre Phase** | C18 Säule (z.B.: Kinetex) | | |
| | Länge: 50 mm; Innendurchmesser: 4,6 mm; Partikelgröße: 2,6 µm | | |

| | Zeit / [min] | Eluent A [%] | Eluent B [%] |
|---|---|---|---|
| | 0,0 | 65 | 35 |
| **Mobile Phase (Gradient)** | 0,7 | 65 | 35 |
| | 6,7 | 20 | 80 |
| | 7,7 | 20 | 80 |
| | 8,0 | 65 | 35 |
| | 12,0 | 65 | 35 |
| **Säulentemperatur** | 25 °C | | |
| **Flussrate** | 1,3 ml/min | | |
| **Injektionsvolumen** / | 5 µl Prüflösung für die Gehaltsbestimmung | | |
| | 40 µl Prüflösung für die Verunreinigungsbestimmung | | |
| | 40 µl Kalibrierlösung | | |
| **Laufzeit /** | 12 Minuten | | |

### HPLC zur Auswertung der Migrationsversuche

| **Gerät** | Ultmate 3000 von thermo fisher scientific | |
|---|---|---|
| **Säule:** | Kinetex C18 Evo 150x4,6 mm, 2,6 µm | |
| **EluentA**: | 0,5%ige wässrige TFA-Lösung | |
| **EluentB:** | Acetonitril | |

| **Gradient:** | **Time** | **%B** |
|---|---|---|
| | -5 | 22 |
| | 0 | 22 |
| | 0,7 | 22 |
| | 6,7 | 78 |
| | 10 | 78 |
| **Fluss:** | 1 ml/min | |
| **Detektion:** | 215 nm | |
| **Temperatur:** | 30°C | |
| **Laufzeit:** | 10 min | |
| **Injektionsvol:** | 20µl | |

### Packmittel

### Gummidichtkappe

Material: Brombutylkautschuk
Silikonöl : Ph. Eur. 1.000 mPas
Silikonmenge: 0,005 - 0,010 mg/cm²

### Gummistopfen

Material: Brombutylkautschuk
Silikonöl : Ph. Eur. 1.000 mPas
Silikonmenge: 0,040 - 0,080 mg/cm2

### Zylinder

Material: ExxonMobil PP 1013 H1 natur
Silikonöl: Ph. Eur."Dimeticone" 1.000 mPas

### Herstellung der Laborchargen

50% des Wasser werden direkt in ein geeignetes Becherglas eingewogen. Das Natriumchlorid und das Oxybutynin-HCl werden in geeignete Gefäße (Glas) eingewogen. Anschließend werden die Komponenten unter Rühren in das Becherglas überführt. Die Einwaagegefäße werden nachgespült.

Anschließend wird ein Großteil der Restmenge Wasser unter Rühren in das Becherglas eingewogen. Anschließend wird der pH-Wert gemessen und dokumentiert. Für Rezepturen, deren pH Wert eingestellt wird (3; 4,5; 5) wird die Salzsäure (0,001%) zugegeben bis der Ziel pH-Wert erreicht ist. Die Lösung wird dann für 30 min bei 250 U/min mittels Magnetrührer gerührt.

Die erhaltene Lösung wird händisch in dem PP Spritzenzylinder aufgezogen, mit der Gummidichtkappe versehen und die gefüllten Spritzenzylinder zunächst verblistert und darauffolgend der Dampfsterilisation unterzogen.

### Sterilisationsprozess

Zur Dampfsterilisation wird der Dampf-Luft Großraum-Autoklav SDR-12.12.60/2 der Firma SBM/Bosch eingesetzt. Für die vorliegenden Versuche werden aber zur Prozessoptimierung nur Leerkammerläufe durchgeführt, bei welchen die Versuchsspritzen auf einer Palette in die Kammermitte gefahren werden und dort ohne Füllbeladung und die weiteren Paletten autoklaviert werden.

Die verblisterten und in Faltschachteln gepackten Spritzen werden dabei in Mengen von 5 bis 25 Faltschachteln (je nach Versuchsgröße) auf einer Leerpalette platziert und mit Gutfühlern zu Temperaturmessung und Steuerung der Programme versehen.

Bei allen Versuchen deren Ergebnisse vorliegend diskutiert werden, handelt es sich daher nicht um Vollkammerversuche.

### Sterilisationsprogramm A

Das Sterilisationsprogramm A enthält zu Beginn eine Entlüftungsphase zur Reduktion der Luftbelastung in den Blistern und Faltschachteln im Wechsel mit Dampfstößen zum langsamen Druckaufbau. Sobald diese Schritte abgeschlossen sind, schließt sich eine Sterilisation unter Stützdruck über die Mindestzeit von 6 min bei etwa 121 °C an. Abschließend wird kontrolliert und mehrstufig abgekühlt unter stetiger Aufrechterhaltung des Stützdruckes. Nach erfolgter Abkühlung erfolgt eine mehrstufige Trocknung über die internen Wärmetauscher. Dies wird mittels Be- und Entlüftephasen unter Temperaturen zwischen 60 bis 70 °C erreicht, so dass nach erfolgreicher Sterilisation ein trocknes Produkt entnommen werden kann. Im Durchschnitt werden somit FO-Werte von 19 min bis 23 min erreicht.

### Sterilisationsprogramm B

Bei Sterilisationsprogramm B handelt es sich um eine Erweiterung von Sterilisationsprogramm A in der Form, dass eine Sterilisationszeit von mind. 20 min bei etwa 121 °C sichergestellt wird. Der Rest des Programms ist in seinen Phasen äquivalent zum Sterilisationsprogramm A. Durch die verlängerte Plateauphase in der Sterilisation ergeben sich FO-Werte im Bereich von 28 min bis 31 min, was einer Erhöhung der Sterilisationsbelastung um ca. 50 % bis 60 % entspricht.

### Stabilität

Die Versuchschargen werden nach der Sterilisation in Stabilitätskammern mit den Bedingungen 25 °C / 60 % RH und 40 °C / 75 % RH bis zu 3 Monaten gelagert.

### Extraktionsversuche

Die 0,1%ige Oxybutyninlösung analog Tabelle 2 wird im Labormaßstab wie in Kapitel "Herstellung der Laborchargen" hergestellt und die befüllten Spritzen dampfsterilisiert. Die Spritzen werden danach für 10 Tage sowohl bei 25 °C / 60 % RH als auch bei 40 °C / 75 % RH gelagert.

Vier der erhaltenen, ausgelagerten Proben werden gemeinsam mit unbehandelten Referenzpackmittel (Blanko) den Extraktionsversuchen unterzogen. Der Inhalt an Oxybutyninlösung von vier gefüllten Spritzen wird verworfen und die Zylinder sowie Gummiteile mit H₂O dest. gespült. Die Kolbenstange aller vier Spritzen wird herausgedreht und verworfen, die Gummidichtkappen und Gummistopfen werden entfernt um vom PP-Zylindermaterial separiert. Die leeren Spritzenkörper und Gummiteile werden in gleichmäßige Stücke zerkleinert (durchschnittliche Größe Zylinder PP: 1 cm², durchschnittliche Größe GT: 9 mm²).

Die vier zerkleinerten Spritzen, sowie die zerkleinerten Gummiteile werden separat mit 150 ml THF 1,5 h auf Rückfluss erhitzt. Nach 1,5 h werden die Gemische auf Raumtemperatur abgekühlt und das Packmittel abfiltriert und mit ca. 30 ml THF nachgewaschen. Das Filtrat wird bis zur Trockene eingeengt.

Das Filtrat der Gummiteile ist ein gelbliches Öl (1,993 g). Das Filtrat der Zylinder ist ein violettes Öl (0,1951 g).

### Qualitativer Nachweis mit DC

Die erhaltenen Extrakte von Gummiteilen und Zylinder werden, wie auch die Referenzsubstanzen von Oxybutyninhydrochlorid und Verunreinigung D in Aceton gelöst. Als Laufmittel wird Cyclohexan/Ethylacetat 1:6 verwendet.

### Sauer-wässrige Mikroaufarbeitung der Extrakte

Die Extrakte der Gummiteile und des Zylinders werden einer nicht volumetrisch genauen Mikroaufarbeitung unterzogen. Dabei wird der in der Trockene vorliegende Extrakt in ca. 3 ml Pufferlösung (pH 4, Zitronensäure, Natriumhydroxid, Salzsäure), sowie 3 ml Diethylether aufgenommen und ausgeschüttelt. Die organische Phase wird abgehoben. Beide Phasen werden über einen PTFE Spritzenfilter filtriert.

### Quantitativer Nachweis mit HPLC

### Probenvorbereitung zur HPLC

Die wässrigen und organischen Proben von Zylinder und Gummiteilen werden im Argonstrom bis zur Trockene eingedampft. Der Rückstand wird in 1 ml THF (Tetrahydrofuran) aufgenommen und mit einem weiteren ml quantitativ in einen 10 ml Messkolben überführt. Anschließend wird mit Wasser 0,1% TFA aufgefüllt. Die entstandene Eintrübung wird über PTFE Spritzenfilter entfernt.

### Bezugszeichenliste:

- 1: Kolbenspritze
- 2: Spritzenkörper
- 3: Spritzenzylinder
- 4: Adapter
- 4a: Auslassöffnung
- 4b: Einlassöffnung
- 5: Spritzenkolben
- 5a: Aufnahme- und/oder Befüllöffnung
- 6: Kolbenstopfen
- 7: Verschlusselement
- 8: Abnehm- und/oder Entfernungseinrichtung

- Z: Oxybutyninhaltige Zusammensetzung

- AR: Auslassrichtung

## Patentansprüche

1. Kolbenspritze, insbesondere Einweg-Kolbenspritze, wobei die Kolbenspritze einen Spritzenkörper einerseits und einen Spritzenkolben mit einem Kolbenstopfen andererseits aufweist, wobei der Spritzenkörper aus einem Polypropylen gebildet ist,
wobei die Kolbenspritze mit einer sterilen oxybutyninhaltigen Zusammensetzung befüllt ist,
wobei die Kolbenspritze erhältlich ist und/oder erhalten ist nach einem Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze,
wobei die nach Abschluss des Verfahrens, insbesondere nach Verfahrensschritt b), erhaltene oxybutyninhaltige Endzusammensetzung als sterile wässrige Zusammensetzung, insbesondere terminal sterilisierte Zusammensetzung, ausgebildet ist und die nachfolgend angeführten Spezifikationsvorgaben (i), (ii) und (iii) aufweist:
(i) vorgegebene Spezifikations-Konzentration von Oxybutynin, bevorzugt in Form des Hydrochlorids, mit vorgegebener Abweichung von höchstens ± 5 %, bezogen auf die Konzentration von Oxybutynin,
(ii) vorgegebener Spezifikations-pH-Wert mit vorgegebener Abweichung von höchstens ± 0,5 pH-Werteinheiten,
(iii) vorgegebene Spezifikations-Maximalmenge an Oxybutynin-Abbauprodukt(en), insbesondere reaktiv hervorgerufenen Oxybutynin-Abbauprodukt(en), vorzugsweise hydrolytisch und/oder oxidativ hervorgerufenen Oxybutynin-Abbauprodukt(en), vorzugsweise Phenylcyclohexylhydroxyessigsäure;
wobei das Verfahren die nachfolgenden Schritte umfasst:
a) Bereitstellen einer oxybutyninhaltigen Ausgangszusammensetzung in der Kolbenspritze und/oder Befüllen der Kolbenspritze mit einer oxybutyninhaltigen Ausgangszusammensetzung
und nachfolgend
b) Hitzesterilisation, insbesondere Dampfsterilisation, vorzugsweise Wasserdampfsterilisation, bevorzugt terminale Sterilisation, der oxybutyninhaltigen Ausgangszusammensetzung in der Kolbenspritze und/oder der mit der oxybutyninhaltigen Ausgangszusammensetzung befüllten Kolbenspritze zum Erhalt der sterilen oxybutyninhaltigen Endzusammensetzung in der vorzugsweise anwendungsfertigen Kolbenspritze und/oder zum Erhalt der vorzugsweise anwendungsfertigen, sterilen, mit der oxybutyninhaltigen Zusammensetzung befüllten Kolbenspritze,
wobei die im Verfahren, insbesondere in Verfahrensschritt b), auftretende Migration und/oder Einlagerung des Oxybutynins in den Spritzenkörper und/oder in den Kolbenstopfen, und der im Verfahren, insbesondere in Verfahrensschritt b), auftretende Abbau des Oxybutynins, insbesondere der reaktive Abbau des Oxybutynins, vorzugsweise der hydrolytisch und/oder oxidativ hervorgerufene Abbau, bevorzugt der Abbau zu Phenylcyclohexylhydroxyessigsäure, gesteuert und/oder kompensiert wird derart und mit der Maßgabe,
dass die nach Durchführung von Verfahrensschritt b) erhaltene sterile oxybutyninhaltige Endzusammensetzung die zuvor angeführten Spezifikationsvorgaben (i), (ii) und (iii) erfüllt und die sterile oxybutyninhaltige Endzusammensetzung in der vorzugsweise anwendungsfertigen Kolbenspritze bzw. die vorzugsweise anwendungsfertige, sterile, mit der oxybutyninhaltigen Endzusammensetzung befüllte Kolbenspritze erhalten wird,
wobei die im Verfahren, insbesondere in Verfahrensschritt b), auftretende Migration und/oder Einlagerung des Oxybutynins in den Spritzenkörper und/oder in den Kolbenstopfen, und der im Verfahren, insbesondere in Verfahrensschritt b), auftretende Abbau, insbesondere der reaktive Abbau des Oxybutynins, vorzugsweise der hydrolytisch und/oder oxidativ hervorgerufene Abbau des Oxybutynins, bevorzugt der Abbau zu Phenylcyclohexylhydroxyessigsäure, durch die nachfolgenden Maßnahmen und/oder Schritte (1), (2), (3) und (4) gesteuert und/oder kontrolliert wird, insbesondere verhindert und/oder minimiert und/oder kompensiert wird:
(1) Einstellung und/oder Verwendung einer im Vergleich zur Spezifikations-Konzentration und/oder Spezifikations-Menge des Oxybutynins, bevorzugt in Form des Hydrochlorids, erhöhte Konzentration und/oder Menge an Oxybutynin, bevorzugt in Form des Hydrochlorids, in der Ausgangszusammensetzung;
(2) Einstellung eines im Vergleich zu dem Spezifikations-pH-Wert abweichenden, insbesondere erhöhten pH-Werts in der Ausgangszusammensetzung;
(3) Einstellung und/oder Auswahl der Sterilisationsbedingungen, insbesondere ausgewählt aus der Gruppe von Sterilisationsart, Sterilisationsdauer, Sterilisationstemperatur, Sterilisationsdruck, Sterilisationsatmosphäre sowie deren Kombinationen, vorzugsweise Einstellung und/oder Auswahl des F0-Werts der Sterilisation;
(4) Ausrüstung und/oder Behandlung, insbesondere Beschichtung, der Innenwandung des Spritzenkörpers mit mindestens einem migrations- und/oder abbauverringernden Mittel, wobei das migrations- und/oder abbauverringernde Mittel ein siliciumhaltiges, insbesondere silikon- oder silikatbasiertes Mittel ist.

2. Kolbenspritze nach Anspruch 1,
wobei, für den Fall von Maßnahme und/oder Schritt (1), in der Ausgangszusammensetzung die Konzentration und/oder Menge an Oxybutynin, bevorzugt in Form des Hydrochlorids, um 1 % bis 20 % oberhalb der Spezifikations-Konzentration und/oder Spezifikations-Menge an Oxybutynin, bevorzugt in Form des Hydrochlorids, der Endzusammensetzung gewählt und/oder eingestellt wird; und/oder
wobei, für den Fall von Maßnahme und/oder Schritt (1), in der Ausgangszusammensetzung die Konzentration und/oder Menge an Oxybutynin, bevorzugt in Form des Hydrochlorids, auf einen Wert im Bereich von 101 % bis 120 % der Spezifikations-Konzentration und/oder der Spezifikations-Menge an Oxybutynin, bevorzugt in Form des Hydrochlorids, der Endzusammensetzung eingestellt wird; und/oder
wobei, für den Fall von Maßnahme und/oder Schritt (1), in der Ausgangszusammensetzung die Konzentration und/oder Menge an Oxybutynin, bevorzugt in Form des Hydrochlorids, auf einen Wert im Bereich von 0,51 mg/ml bis 3,5 mg/ml eingestellt wird.

3. Kolbenspritze nach Anspruch 1 oder 2,
wobei, für den Fall von Maßnahme und/oder Schritt (2), der pH-Wert der Ausgangszusammensetzung um 0,02 pH-Werteinheiten bis 2,4 pH-Werteinheiten oberhalb des Spezifikations-pH-Werts der Endzusammensetzung eingestellt wird; und/oder
wobei, für den Fall von Maßnahme und/oder Schritt (2), der pH-Wert der Ausgangszusammensetzung um 5 % bis 90 % oberhalb des Spezifikations-pH-Werts der Endzusammensetzung eingestellt wird; und/oder
wobei, für den Fall von Maßnahme und/oder Schritt (2), der pH-Wert der Ausgangszusammensetzung auf einen Wert im Bereich von 2,8 bis 7,5 eingestellt wird; und/oder
wobei, für den Fall von Maßnahme und/oder Schritt (2), der pH-Wert der Ausgangszusammensetzung mittels Salzsäure eingestellt wird.

4. Kolbenspritze nach einem der vorangehenden Ansprüche,
wobei, für den Fall von Maßnahme und/oder Schritt (3), die Hitzesterilisation, insbesondere Dampfsterilisation, vorzugsweise Wasserdampfsterilisation, bevorzugt terminale Sterilisation, mit einem F0-Wert im Bereich von 6 min bis 45 min durchgeführt wird; und/oder
wobei, für den Fall von Maßnahme und/oder Schritt (3), die Sterilisationsbedingungen auf einen F0-Wert im Bereich von 6 min bis 45 min eingestellt oder hieraus ausgewählt werden; und/oder
wobei, für den Fall von Maßnahme und/oder Schritt (4), die Ausrüstung und/oder Behandlung, insbesondere Beschichtung, der Innenwandung des Spritzenkörpers mit dem siliciumhaltigen Mittel durchgeführt wird, wobei das migrations- und/oder abbauverringernde Mittel Silikonöl oder Silikat ist.

5. Kolbenspritze nach einem der vorangehenden Ansprüche,
wobei die im Verfahren, insbesondere in Verfahrensschritt b), auftretende Migration und/oder Einlagerung des Oxybutynins in den Spritzenkörper und/oder in den Kolbenstopfen, und der im Verfahren, insbesondere in Verfahrensschritt b), auftretende Abbau, insbesondere der reaktive Abbau des Oxybutynins, bevorzugt der Abbau zu Phenylcyclohexylhydroxyessigsäure, durch Einstellung des Verhältnisses von (i) Oxybutynin in Form der freien Base einerseits und (ii) Oxybutynin in Form der komplexierten und/oder koordinierten und/oder versalzenen und/oder protonierten Base, vorzugsweise Oxybutynin in Form des Hydrochlorids, gesteuert und/oder kontrolliert wird, insbesondere verhindert und/oder minimiert wird, wobei das Verhältnis in Richtung von (ii) Oxybutynin in Form der komplexierten und/oder koordinierten und/oder versalzenen und/oder protonierten Base, vorzugsweise Oxybutynin in Form des Hydrochlorids, verschoben wird, vorzugsweise durch Einstellung, insbesondere Absenkung, des pH-Werts und/oder durch Einstellung eines insbesondere niedrigen pH-Werts auf einen pH-Wert im Bereich von 2,8 bis 5, vorzugsweise im Bereich von 3,1 bis 4,5, bevorzugt im Bereich von 3,2 bis 4,2, in der Ausgangzusammensetzung.

6. Kolbenspritze nach einem der vorangehenden Ansprüche,
wobei die mit der oxybutyninhaltigen Zusammensetzung befüllte Kolbenspritze vor Durchführung der Hitzesterilisation, vorzugsweise Wasserdampfsterilisation, bevorzugt terminalen Sterilisation, in eine wasser- und/oder keimdicht verschlossene Verpackung eingebracht worden ist, wobei zumindest ein Teil der Verpackung wenigstens wasserdampfdurchlässig ausgebildet ist, wobei die Verpackung bei Durchführung der Dampfsterilisation gleichermaßen sterilisiert wird.

7. Kolbenspritze nach einem der vorangehenden Ansprüche,
wobei die nach Abschluss des Verfahrens, insbesondere nach Verfahrensschritt b), erhaltene oxybutyninhaltige Endzusammensetzung als sterile wässrige Zusammensetzung, insbesondere terminal sterilisierte Zusammensetzung, ausgebildet ist und die nachfolgend angeführten Spezifikationsvorgaben (i), (ii) und (iii) aufweist:
(i) vorgegebene Spezifikations-Konzentration von Oxybutynin, bevorzugt in Form des Hydrochlorids:
0,5 mg/ml bis 2 mg/ml mit maximaler Abweichung von ± 5 %, bezogen auf die Konzentration von Oxybutynin, vorzugsweise 1 mg/ml mit maximaler Abweichung von ± 0,05 mg/ml,
(ii) vorgegebener Spezifikations-pH-Wert:
2,8 bis 5,2, insbesondere 3 bis 5, vorzugsweise 3,2 bis 4,8, bevorzugt 3,5 bis 4,5, besonders bevorzugt 3,5 bis 4,2, ganz besonders bevorzugt 3,8 bis 4,2,
(iii) vorgegebene Spezifikations-Menge an Oxybutynin-Abbau-produkt(en), insbesondere reaktiv hervorgerufenen Oxybutynin-Abbauprodukt(en), vorzugsweise hydrolytisch und/oder oxidativ hervorgerufenen Oxybutynin-Abbauprodukt(en), vorzugsweise Phenylcyclohexylhydroxyessigsäure:
höchstens 0,5 Gew.-%, insbesondere höchstens 0,2 Gew.-%, bezogen auf die Gesamtmenge an eingesetztem Oxybutynin, bevorzugt Oxybutyninhydrochlorid.

8. Verpackungseinheit, enthaltend mindestens eine Verpackung und mindestens eine Kolbenspritze, insbesondere Einweg-Kolbenspritze, wie in einem der Ansprüche 1 bis 7 definiert, wobei die Kolbenspritze in der Verpackung vorliegt.

9. Verpackungseinheit nach Anspruch 8, wobei die mit der oxybutyninhaltigen Zusammensetzung befüllte Kolbenspritze vor Durchführung der Hitzesterilisation, vorzugsweise Wasserdampfsterilisation, bevorzugt terminalen Sterilisation, in eine wasser- und/oder keimdicht verschlossene Verpackung eingebracht worden ist, wobei zumindest ein Teil der Verpackung wasserdampf- und gasdurchlässig ausgebildet ist, wobei die Verpackung bei Durchführung der Dampfsterilisation gleichermaßen sterilisiert wird.

10. Kit, insbesondere Applikations- und/oder Instillationssystem, umfassend: (i) mindestens eine in einer Kolbenspritze, insbesondere Einweg-Kolbenspritze, wie in einem der Ansprüche 1 bis 7 definiert, vorliegende oxybutyninhaltige Zusammensetzung; (ii) (a) mindestens eine an die Kolbenspritze anschließbare Applikations- und/oder Instillationsvorrichtung, vorzugsweise in Form eines Harnblasenkatheters, und/oder (b) mindestens eine an die Kolbenspritze anschließbare Anschlussvorrichtung zum Anschließen an eine Applikations- und/oder Instillationsvorrichtung, vorzugsweise in Form eines Harnblasenkatheters; und gegebenenfalls (iii) mindestens eine Applikations- und/oder Instillationsanleitung.

11. Kit, insbesondere Applikations- und/oder Instillationssystem, umfassend (i) mindestens eine in einer Kolbenspritze, insbesondere Einweg-Kolbenspritze, wie in einem der Ansprüche 1 bis 7 definiert, vorliegende oxybutyninhaltige Zusammensetzung; (ii) (a) mindestens eine an die Kolbenspritze, insbesondere an den Adapter (4) der Kolbenspritze, anschließbare Applikations- und/oder Instillationsvorrichtung, vorzugsweise in Form eines Harnblasenkatheters; gegebenenfalls (iii) ein Adapter-Gegenstück zum Anschließen der Applikations- und/oder Instillationsvorrichtung an die Kolbenspritze, insbesondere an den Adapter der Kolbenspritze; und gegebenenfalls (iv) mindestens eine Applikations- und/oder Instillationsanleitung.

## Claims

1. Syringe, in particular disposable syringe, wherein the syringe comprises a syringe body on the one hand and a syringe plunger having a plunger stopper on the other hand, wherein the syringe body is formed from polypropylene,
wherein the syringe is filled with a sterile composition comprising oxybutynin,
wherein the syringe is obtainable and/or is obtained by a method for producing a sterile composition comprising oxybutynin in a preferably ready-to-use syringe, in particular disposable syringe, and/or for producing a preferably ready-to-use sterile syringe, in particular disposable syringe, filled with a composition comprising oxybutynin,
wherein the final composition comprising oxybutynin obtained upon completion of the method, in particular after method step b), is in the form of a sterile aqueous composition, in particular terminally sterilized composition, and meets the following specification requirements (i), (ii), and (iii):
(i) prescribed specification-defined concentration of oxybutynin, preferably in the form of the hydrochloride, with a prescribed deviation of no more than ± 5%, based on the concentration of oxybutynin,
(ii) prescribed specification-defined pH value with a prescribed deviation of no more than ± 0.5 pH units,
(iii) prescribed specification-defined maximum amount of oxybutynin degradation product(s), in particular reaction-derived oxybutynin degradation product(s), preferably hydrolytically-derived and/or oxidatively-derived oxybutynin degradation product(s), preferably phenylcyclohexylhydroxyacetic acid;
wherein the method comprises the following steps:
a) providing a starting composition comprising oxybutynin in the syringe and/or filling the syringe with a starting composition comprising oxybutynin
and subsequently
b) heat sterilization, in particular steam sterilization, preferably water vapor sterilization, more preferably terminal sterilization, of the starting composition comprising oxybutynin in the syringe and/or of the syringe filled with the starting composition comprising oxybutynin to obtain the sterile final composition comprising oxybutynin in the preferably ready-to-use syringe and/or to obtain the preferably ready-for-use sterile syringe filled with the composition comprising oxybutynin,
wherein the migration and/or incorporation of the oxybutynin into the syringe body and/or into the plunger stopper occurring during the method, particularly during method step b), and the degradation of the oxybutynin, in particular the reactive degradation of the oxybutynin, preferably the hydrolytically-derived and/or oxidatively-derived degradation, more preferably the degradation to phenylcyclohexylhydroxyacetic acid, occurring during the method, particularly during method step b), is controlled and/or compensated for in such a way and with the proviso,
that the sterile final composition comprising oxybutynin obtained after carrying out process step b) meets the aforementioned specification requirements (i), (ii), and (iii) and the sterile final composition comprising oxybutynin is obtained in the preferably ready-to-use syringe and/or the preferably ready-to-use sterile syringe filled with the final composition comprising oxybutynin is obtained,
wherein the migration and/or incorporation of the oxybutynin into the syringe body and/or the plunger stopper occurring during the method, particularly during method step b), and the degradation of the oxybutynin, in particular the reactive degradation of the oxybutynin, preferably the hydrolytically-derived and/or oxidatively-derived degradation, more preferably the degradation to phenylcyclohexylhydroxyacetic acid, occurring during the method, particularly during method step b), is controlled and/or regulated, in particular prevented and/or minimized and/or compensated for, by the following measures and/or steps (1), (2), (3) and (4):
(1) Setting and/or using an increased concentration and/or amount of oxybutynin, preferably in the form of the hydrochloride, in the starting composition compared to the prescribed specification-defined concentration of oxybutynin, preferably in the form of the hydrochloride;
(2) Setting a pH value in the starting composition deviating from, in particular being higher than, the prescribed specification-defined pH value;
(3) Setting and/or selection of the sterilization conditions, in particular selected from the group consisting of sterilization method, sterilization duration, sterilization temperature, sterilization pressure, sterilization atmosphere, and combinations thereof, preferably setting and/or selection of the F0 value of the sterilization;
(4) Equipping and/or treating, in particular coating, the inner surface of the syringe body with at least one migration- and/or degradation-reducing agent, wherein the migration- and/or degradation-reducing agent is a silicon-containing agent, in particular a silicone- or silicate-based agent.

2. Syringe according to claim 1,
wherein, in the case of measure and/or step (1), the concentration and/or amount of oxybutynin, preferably in the form of the hydrochloride, in the initial composition is selected and/or set to be 1% to 20% above the specification-defined concentration and/or specification-defined amount of oxybutynin, preferably in the form of the hydrochloride, in the final composition; and/or
wherein, in the case of measure and/or step (1), the concentration and/or amount of oxybutynin, preferably in the form of the hydrochloride, in the starting composition is set to a value in the range of 101% to 120% of the specification-defined concentration and/or specification-defined amount of oxybutynin, preferably in the form of the hydrochloride, in the final composition; and/or
wherein, in the case of measure and/or step (1), the concentration and/or amount of oxybutynin, preferably in the form of the hydrochloride, in the starting composition is set to a value in the range of 0.51 mg/mL to 3.5 mg/mL.

3. Syringe according to claim 1 or 2,
wherein, in the case of measure and/or step (2), the pH value of the starting composition is set to be 0.02 pH units to 2.4 pH units higher than the specification-defined pH value of the final composition; and/or
wherein, in the case of measure and/or step (2), the pH value of the starting composition is set to be 5% to 90% higher than the specification-defined pH value of the final composition; and/or
wherein, in the case of measure and/or step (2), the pH value of the starting composition is set to a value in the range of 2.8 to 7.5; and/or
wherein, in the case of measure and/or step (2), the pH value of the starting composition is set using hydrochloric acid.

4. Syringe according to any of the preceding claims,
wherein, in the case of measure and/or step (3), the heat sterilization, in particular steam sterilization, preferably water vapor sterilization, more preferably terminal sterilization, is carried out with an F0 value in the range of 6 min to 45 min; and/or
wherein, in the case of measure and/or step (3), the sterilization conditions are set to or selected from an F0 value in the range of 6 min to 45 min; and/or
wherein, in the case of measure and/or step (4), the equipment and/or treatment, in particular coating, of the inner surface of the syringe body with the silicon-containing agent is carried out, wherein the migration- and/or degradation-reducing agent is silicone oil or silicate.

5. Syringe according to any of the preceding claims,
wherein the migration and/or incorporation of the oxybutynin into the syringe body and/or the plunger stopper occurring during the method, particularly during method step b), and the degradation of the oxybutynin, in particular the reactive degradation of the oxybutynin, preferably the degradation to phenylcyclohexylhydroxyacetic acid, occurring during the method, particularly during method step b), is controlled and/or regulated, in particular prevented and/or minimized, by adjusting the ratio of (i) oxybutynin in the form of the free base, on the one hand, and (ii) oxybutynin in the form of the complexed and/or coordinated and/or salted and/or protonated base, preferably oxybutynin in the form of the hydrochloride, on the other hand, wherein the ratio is adjusted toward (ii) oxybutynin in the form of the complexed and/or coordinated and/or salted and/or protonated base, preferably oxybutynin in the form of the hydrochloride, preferably by adjusting, in particular lowering, the pH value and/or by setting an, in particular low, pH value in the range of 2.8 to 5, preferably in the range of 3.1 to 4.5, most preferably in the range of 3.2 to 4.2, in the starting composition.

6. Syringe according to any of the preceding claims,
wherein the syringe filled with the composition comprising oxybutynin has been placed, prior to carrying out the heat sterilization, preferably water vapor sterilization, more preferably terminal sterilization, into a water- and/or germ-tight sealed packaging, wherein at least a portion of the packaging is configured to be at least water-vapor-permeable, wherein the packaging is likewise sterilized when carrying out the steam sterilization.

7. Syringe according to any of the preceding claims,
wherein the final composition comprising oxybutynin obtained upon completion of the method, in particular after method step b), is in the form of a sterile aqueous composition, in particular a terminally sterilized composition, and meets the following specification requirements (i), (ii), and (iii):
(i) prescribed specification-defined concentration of oxybutynin, preferably in the form of the hydrochloride:
0.5 mg/mL to 2 mg/mL with a maximum deviation of ± 5%, based on the concentration of oxybutynin, preferably 1 mg/mL with a maximum deviation of ± 0.05 mg/mL,
(ii) prescribed specification-defined pH value:
2.8 to 5.2, in particular 3 to 5, preferably 3.2 to 4.8, more preferably 3.5 to 4.5, particularly preferably 3.5 to 4.2, most preferably 3.8 to 4.2,
(iii) prescribed specification-defined maximum amount of oxybutynin degradation product(s), in particular reaction-derived oxybutynin degradation product(s), preferably hydrolytically-derived and/or oxidatively-derived oxybutynin degradation product(s), preferably phenylcyclohexylhydroxyacetic acid:
no more than 0.5% by weight, in particular no more than 0.2% by weight, based on the total amount of oxybutynin used, preferably oxybutynin hydrochloride.

8. Packaging unit comprising at least one packaging and at least one syringe, in particular a disposable syringe, as defined in any one of claims 1 to 7, wherein the syringe is present in the packaging.

9. Packaging unit according to claim 8,
wherein the syringe filled with the composition comprising oxybutynin has been placed, prior to carrying out the heat sterilization, preferably water vapor sterilization, more preferably terminal sterilization, into a water- and/or germ-tight sealed packaging, wherein at least a portion of the packaging is configured to be water-vapor-permeable, wherein the packaging is likewise sterilized when carrying out the steam sterilization.

10. Kit, in particular application and/or instillation system, comprising: (i) at least one composition comprising oxybutynin present in a syringe, in particular disposable syringe, as defined in any one of claims 1 to 7; (ii)
(a) at least one application and/or instillation device connectable to the syringe, preferably in the form of a urinary catheter, and/or
(b) at least one connection device connectable to the syringe for connecting to an application and/or instillation device, preferably in the form of a urinary catheter; and, optionally, (iii) at least one application and/or instillation instruction.

11. Kit, in particular application and/or instillation system, comprising: (i) at least one composition comprising oxybutynin present in a syringe, in particular disposable syringe, as defined in any one of claims 1 to 7; (ii)
(a) at least one application and/or instillation device connectable to the syringe, in particular to the adapter (4) of the syringe, preferably in the form of a urinary catheter; optionally, (iii) an adapter counterpart for connecting the application and/or instillation device to the syringe, in particular to the adapter of the syringe; and, optionally, (iv) at least one application and/or instillation instruction.

## Revendications

1. Seringue à piston, en particulier seringue à piston à usage unique, où la seringue à piston présente d'une part un corps de seringue et d'autre part un piston de seringue avec un bouchon de piston, où le corps de seringue est formé d'un polypropylène,
où la seringue à piston est remplie d'une composition stérile contenant de l'oxybutynine,
où la seringue à piston peut être obtenue et/ou est obtenue selon un procédé de fabrication d'une composition stérile contenant de l'oxybutynine dans une seringue à piston de préférence prête à l'emploi, en particulier seringue à piston à usage unique, et/ou pour la fabrication d'une seringue à piston de préférence prête à l'emploi, stérile, remplie d'une composition contenant de l'oxybutynine, en particulier seringue à piston à usage unique,
où la composition finale contenant de l'oxybutynine obtenue à l'issue du procédé, en particulier après l'étape b) du procédé, se présente sous la forme d'une composition aqueuse stérile, en particulier d'une composition stérilisée en phase terminale, et présente les spécifications (i), (ii) et (iii) indiquées ci-après:
(i) concentration de spécification prédéfinie d'oxybutynine, de préférence sous la forme du chlorhydrate, avec un écart prédéfini d'au plus ± 5 %, par rapport à la concentration d'oxybutynine,
(ii) valeur de pH de spécification prédéfinie avec un écart prédéfini d'au plus ± 0,5 unité de pH,
(iii) quantité maximale de spécification prédéfinie de produit(s) de dégradation d'oxybutynine, en particulier de produit(s) de dégradation d'oxybutynine provoqué(s) par réaction, de préférence de produit(s) de dégradation d'oxybutynine provoqué(s) par hydrolyse et/ou oxydation, de préférence d'acide phénylcyclohexylhydroxyacétique;
où le procédé comprend les étapes suivantes:
a) mise à disposition d'une composition de départ contenant de l'oxybutynine dans la seringue à piston et/ou remplissage de la seringue à piston avec une composition de départ contenant de l'oxybutynine
et ensuite
b) stérilisation par la chaleur, en particulier stérilisation à la vapeur, de préférence stérilisation à la vapeur d'eau, de préférence stérilisation terminale, de la composition de départ contenant de l'oxybutynine dans la seringue à piston et/ou de la seringue à piston remplie de la composition de départ contenant de l'oxybutynine pour obtenir la composition finale stérile contenant de l'oxybutynine dans la seringue à piston de préférence prête à l'emploi et/ou pour obtenir la seringue à piston de préférence prête à l'emploi, stérile, remplie de la composition contenant de l'oxybutynine,
où la migration et/ou l'incorporation de l'oxybutynine dans le corps de seringue et/ou dans le bouchon de piston se produisant dans le procédé, en particulier dans l'étape b) du procédé, et la dégradation de l'oxybutynine se produisant dans le procédé, en particulier dans l'étape b) du procédé, en particulier la dégradation réactive de l'oxybutynine, de préférence la dégradation provoquée par hydrolyse et/ou oxydation, de préférence la dégradation en acide phénylcyclohexylhydroxyacétique, sont commandées et/ou compensées de telle manière et avec la condition
que la composition finale stérile contenant de l'oxybutynine obtenue après la mise en oeuvre de l'étape b) du procédé satisfait aux spécifications (i), (ii) et (iii) indiquées ci-dessus et que la composition finale stérile contenant de l'oxybutynine est obtenue dans la seringue à piston de préférence prête à l'emploi ou la seringue à piston de préférence prête à l'emploi, stérile, remplie de la composition finale contenant de l'oxybutynine,
où la migration et/ou l'incorporation de l'oxybutynine dans le corps de seringue et/ou dans le bouchon de piston se produisant dans le procédé, en particulier dans l'étape b) du procédé, et la dégradation se produisant dans le procédé, en particulier dans l'étape b) du procédé, en particulier la dégradation réactive de l'oxybutynine, de préférence la dégradation provoquée par hydrolyse et/ou oxydation de l'oxybutynine, de préférence la dégradation en acide phénylcyclohexylhydroxyacétique, sont commandées et/ou contrôlées par les mesures et/ou étapes (1), (2), (3) et (4) suivantes, en particulier sont empêchées et/ou minimisées et/ou compensées:
(1) réglage et/ou utilisation d'une concentration et/ou d'une quantité d'oxybutynine, de préférence sous la forme du chlorhydrate, augmentée par rapport à la concentration de spécification et/ou à la quantité de spécification de l'oxybutynine, de préférence sous la forme du chlorhydrate, dans la composition de départ;
(2) réglage d'une valeur de pH différente, en particulier augmentée, par rapport à la valeur de pH de spécification dans la composition de départ;
(3) réglage et/ou sélection des conditions de stérilisation, en particulier choisies dans le groupe constitué par le type de stérilisation, la durée de stérilisation, la température de stérilisation, la pression de stérilisation, l'atmosphère de stérilisation ainsi que leurs combinaisons, de préférence réglage et/ou sélection de la valeur F0 de la stérilisation;
(4) équipement et/ou traitement, en particulier revêtement, de la paroi intérieure du corps de seringue avec au moins un agent réduisant la migration et/ou la dégradation, où l'agent réduisant la migration et/ou la dégradation est un agent contenant du silicium, en particulier à base de silicone ou de silicate.

2. Seringue à piston selon la revendication 1,
où, dans le cas de la mesure et/ou de l'étape (1), dans la composition de départ, la concentration et/ou la quantité d'oxybutynine, de préférence sous la forme du chlorhydrate, est choisie et/ou réglée de 1 % à 20 % au-dessus de la concentration de spécification et/ou de la quantité de spécification d'oxybutynine, de préférence sous la forme du chlorhydrate, de la composition finale; et/ou
où, dans le cas de la mesure et/ou de l'étape (1), dans la composition de départ, la concentration et/ou la quantité d'oxybutynine, de préférence sous la forme du chlorhydrate, est réglée à une valeur dans la plage de 101 % à 120 % de la concentration de spécification et/ou de la quantité de spécification d'oxybutynine, de préférence sous la forme du chlorhydrate, de la composition finale; et/ou
où, dans le cas de la mesure et/ou de l'étape (1), dans la composition de départ, la concentration et/ou la quantité d'oxybutynine, de préférence sous la forme du chlorhydrate, est réglée à une valeur dans la plage de 0,51 mg/ml à 3,5 mg/ml.

3. Seringue à piston selon la revendication 1 ou 2,
où, dans le cas de la mesure et/ou de l'étape (2), la valeur de pH de la composition de départ est réglée de 0,02 unité de pH à 2,4 unités de pH au-dessus de la valeur de pH de spécification de la composition finale; et/ou
où, dans le cas de la mesure et/ou de l'étape (2), la valeur de pH de la composition de départ est réglée de 5 % à 90 % au-dessus de la valeur de pH de spécification de la composition finale; et/ou
où, dans le cas de la mesure et/ou de l'étape (2), la valeur de pH de la composition de départ est réglée à une valeur dans la plage de 2,8 à 7,5; et/ou
où, dans le cas de la mesure et/ou de l'étape (2), la valeur de pH de la composition de départ est réglée au moyen d'acide chlorhydrique.

4. Seringue à piston selon l'une quelconque des revendications précédentes,
où, dans le cas de la mesure et/ou de l'étape (3), la stérilisation par la chaleur, en particulier la stérilisation à la vapeur, de préférence la stérilisation à la vapeur d'eau, de préférence la stérilisation terminale, est réalisée avec une valeur F0 dans la plage de 6 min à 45 min; et/ou
où, dans le cas de la mesure et/ou de l'étape (3), les conditions de stérilisation sont réglées à une valeur F0 dans la plage de 6 min à 45 min ou choisies à partir de celle-ci; et/ou
où, dans le cas de la mesure et/ou de l'étape (4), l'équipement et/ou le traitement, en particulier le revêtement, de la paroi intérieure du corps de seringue est réalisé avec l'agent contenant du silicium, où l'agent réduisant la migration et/ou la dégradation est une huile de silicone ou un silicate.

5. Seringue à piston selon l'une quelconque des revendications précédentes,
où la migration et/ou l'incorporation de l'oxybutynine dans le corps de seringue et/ou dans le bouchon de piston se produisant dans le procédé, en particulier dans l'étape b) du procédé, et la dégradation se produisant dans le procédé, en particulier dans l'étape b) du procédé, en particulier la dégradation réactive de l'oxybutynine, de préférence la dégradation en acide phénylcyclohexylhydroxyacétique, sont commandées et/ou contrôlées par ajustement du rapport entre (i) l'oxybutynine sous la forme de la base libre d'une part et (ii) l'oxybutynine sous la forme de la base complexée et/ou coordonnée et/ou salifiée et/ou protonée, de préférence l'oxybutynine sous la forme du chlorhydrate, d'autre part, en particulier sont empêchées et/ou minimisées, où le rapport est décalé en direction de (ii) l'oxybutynine sous la forme de la base complexée et/ou coordonnée et/ou salifiée et/ou protonée, de préférence l'oxybutynine sous la forme du chlorhydrate, de préférence par ajustement, en particulier abaissement, de la valeur de pH et/ou par ajustement d'une valeur de pH, en particulier une faible valeur de pH, dans la plage de 2,8 à 5, de préférence dans la plage de 3,1 à 4,5, de préférence dans la plage de 3,2 à 4,2, dans la composition de départ.

6. Seringue à piston selon l'une quelconque des revendications précédentes,
où la seringue à piston remplie de la composition contenant de l'oxybutynine a été introduite dans un emballage fermé de manière étanche à l'eau et/ou aux germes avant la mise en oeuvre de la stérilisation par la chaleur, de préférence la stérilisation à la vapeur d'eau, de préférence la stérilisation terminale, où au moins une partie de l'emballage est réalisée de manière perméable au moins à la vapeur d'eau, où l'emballage est stérilisé de la même manière lors de la mise en oeuvre de la stérilisation à la vapeur.

7. Seringue à piston selon l'une quelconque des revendications précédentes,
où la composition finale contenant de l'oxybutynine obtenue à l'issue du procédé, en particulier après l'étape b) du procédé, se présente sous la forme d'une composition aqueuse stérile, en particulier d'une composition stérilisée en phase terminale, et présente les spécifications (i), (ii) et (iii) indiquées ci-après:
(i) concentration de spécification prédéfinie d'oxybutynine, de préférence sous la forme du chlorhydrate:
0,5 mg/ml à 2 mg/ml avec un écart maximal de ± 5 %, par rapport à la concentration d'oxybutynine, de préférence 1 mg/ml avec un écart maximal de ± 0,05 mg/ml,
(ii) valeur de pH de spécification prédéfinie:
2,8 à 5,2, en particulier 3 à 5, de préférence 3,2 à 4,8, de préférence 3,5 à 4,5, de manière particulièrement préférée 3,5 à 4,2, de manière tout particulièrement préférée 3,8 à 4,2,
(iii) quantité de spécification prédéfinie de produit(s) de dégradation d'oxybutynine, en particulier de produit(s) de dégradation d'oxybutynine provoqué(s) par réaction, de préférence de produit(s) de dégradation d'oxybutynine provoqué(s) par hydrolyse et/ou oxydation, de préférence d'acide phénylcyclohexylhydroxyacétique:
au plus 0,5 % en poids, en particulier au plus 0,2 % en poids, par rapport à la quantité totale d'oxybutynine utilisée, de préférence de chlorhydrate d'oxybutynine.

8. Unité d'emballage, contenant au moins un emballage et au moins une seringue à piston, en particulier une seringue à piston à usage unique, telle que définie dans l'une quelconque des revendications 1 à 7, où la seringue à piston se trouve dans l'emballage.

9. Unité d'emballage selon la revendication 8,
où la seringue à piston remplie de la composition contenant de l'oxybutynine a été introduite dans un emballage fermé de manière étanche à l'eau et/ou aux germes avant la mise en oeuvre de la stérilisation par la chaleur, de préférence la stérilisation à la vapeur d'eau, de préférence la stérilisation terminale, où au moins une partie de l'emballage est réalisée de manière perméable au moins à la vapeur d'eau et aux gaz, où l'emballage est stérilisé de la même manière lors de la mise en oeuvre de la stérilisation à la vapeur.

10. Kit, en particulier système d'application et/ou d'instillation, comprenant: (i) au moins une composition contenant de l'oxybutynine se trouvant dans une seringue à piston, en particulier une seringue à piston à usage unique, telle que définie dans l'une quelconque des revendications 1 à 7; (ii) (a) au moins un dispositif d'application et/ou d'instillation pouvant être raccordé à la seringue à piston, de préférence sous la forme d'un cathéter à vessie urinaire, et/ou (b) au moins un dispositif de raccordement pouvant être raccordé à la seringue à piston pour le raccordement à un dispositif d'application et/ou d'instillation, de préférence sous la forme d'un cathéter à vessie urinaire; et éventuellement (iii) au moins un manuel d'application et/ou d'instillation.

11. Kit, en particulier système d'application et/ou d'instillation, comprenant (i) au moins une composition contenant de l'oxybutynine se trouvant dans une seringue à piston, en particulier une seringue à piston à usage unique, telle que définie dans l'une quelconque des revendications 1 à 7; (ii) (a) au moins un dispositif d'application et/ou d'instillation pouvant être raccordé à la seringue à piston, en particulier à l'adaptateur (4) de la seringue à piston, de préférence sous la forme d'un cathéter à vessie urinaire; éventuellement (iii) une contre-pièce d'adaptateur pour le raccordement du dispositif d'application et/ou d'instillation à la seringue à piston, en particulier à l'adaptateur de la seringue à piston; et éventuellement (iv) au moins un manuel d'application et/ou d'instillation.
